# EUROPEAN PATENT SPECIFICATION

(11) **EP 4 430 398 B1**
(45) Date of publication and mention of the grant of the patent: **31.12.2025**
(21) Application number: 22821819.4
(22) Date of filing: 09.11.2022
(51) Int. Cl.: G01N 33/537, G01N 33/577, G01N 33/58, G01N 33/68

(54) **METHODS FOR IDENTIFICATION OF ANTIGEN-BINDING MOLECULES**
VERFAHREN ZUR IDENTIFIZIERUNG VON ANTIGENBINDENDEN MOLEKÜLEN
PROCÉDÉS D'IDENTIFICATION DE MOLÉCULES DE LIAISON À L'ANTIGÈNE

(30) Priority: 10.11.2021 US 202163278012 P
(43) Date of publication of application: 18.09.2024
(73) Proprietor: 10X Genomics, Inc., Pleasanton, CA 94588-3260 (US)
(72) Inventor: MCDONNELL, Wyatt James, Pleasanton, California 94588-3260 (US); STUBBINGTON, Michael John Terry, Bristol, Bridgewater House Counterslip Bristol BS1 6BX (GB); MCDERMOTT, Geoffrey P., Pleasanton, California 94588-3260 (US); PATTERSON, David Michael, Pleasanton, California 94588-3260 (US); ENGBLOM, Camilla, Pleasanton, California 94588-3260 (US); THRANE, Kim, 113 63 Stockholm (SE); MOLD, Jeffrey, 113 63 Stockholm (SE); FRISEN, Jonas, 113 63 Stockholm (SE); LUNDEBERG, Joakim, 113 63 Stockholm (SE); LIN, Qirong, Pleasanton, California 94588-3260 (US); GALLANT, Caroline Julie, 113 63 Stockholm (SE); STOECKIUS, Marlon, 113 63 Stockholm (SE); PFEIFFER, Katherine, Pleasanton, California 94588-3260 (US); BENT, Zachary, Pleasanton, California 94588-3260 (US); GALONSKA, Christina, 113 63 Stockholm (SE); ROYALL, Ariel, Pleasanton, California 94588-3260 (US)
(74) Representative: J A Kemp LLP
(86) International application number: PCT/US2022/079548
(87) International publication number: WO 2023/086824

(56) References cited:
- WO-A1-2021/168287
- US-A1- 2015 148 239
- CHRISTOF M NIEMEYER ET AL: "Combination of DNA-directed immobilization and immuno-PCR: Very Sensitive Antigen Detection by Means of Self-Assembled DNA-Protein Conjugates", NUCLEIC ACIDS RESEARCH, OXFORD UNIVERSITY PRESS, GB, vol. 31, no. 16, 1 January 2003 (2003-01-01), XP008130337, ISSN: 0305-1048, DOI: 10.1093/NAR/GNG090

## Description

### FIELD

The present disclosure relates to methods for identification of antigen-binding molecules.

### BACKGROUND

Drug-reactive antibodies (DRAs), also referred to interchangeably herein as anti-drug antibodies (ADAs), can be antibodies that develop from adaptive immune recognition of antibody-based drugs or therapies. The development of drug-reactive antibodies (DRAs) or anti-drug antibodies (ADAs) can be hazardous for patients on antibody-based therapies and severely inhibit or prevent drug efficacy. Anti-drug antibodies can be found in individuals who have never been exposed to a drug. Anti-drug antibodies can be characterized into three classes - (1) a paratope-specific, inhibitory, neutralizing, anti-idiotypic antibody; (2) an anti-idiotypic, non-paratope-specific, non-inhibitory; and (3) a drug-target complex specific, non-inhibitory antibody. Presence of any of these anti-drug antibody can contribute to poor outcomes. Thus, identifying these drug-reactive antibodies (DRAs) or anti-drug antibodies (ADAs) is an essential part of all clinical trials of antibodies and antibody-based therapies. Furthermore, a better understanding the drug-reactive antibody repertoire can guide drug development and even drug discovery. There is a clear need for efficiently identifying drug-reactive antibodies (DRAs) or anti-drug antibodies (ADAs). WO2021168287 discloses an integrated assay of a biological sample comprising an in situ assay module and a spatial assay module.

### SUMMARY OF THE INVENTION

The present invention, as defined by the appended claims, provides herein a method, said method comprising: (a) contacting a tissue sample comprising one or more cells expressing an antigen-binding molecule (ABM) with a reporter oligonucleotide conjugated antigen, wherein the reporter oligonucleotide comprises a reporter barcode sequence and the antigen is an antibody therapeutic and/or antibody drug conjugate; (b) attaching a first analyte of an ABM-expressing cell of the tissue sample to a first capture domain of a first capture probe of an array of capture probes attached to a substrate, the first capture probe comprising (i) a spatial barcode sequence and (ii) the first capture domain comprising a first capture sequence, wherein the first analyte of the ABM-expressing cell comprises a sequence encoding the ABM expressed by the ABM-expressing cell or a reverse complement thereof, wherein the ABM expressed by the ABM-expressing cell binds to the antigen; (c) attaching the reporter oligonucleotide to a second capture probe of the array, the second capture probe of the array comprising (i) the spatial barcode sequence and (ii) a second capture domain comprising a second capture sequence; (d) using the first analyte of the ABM-expressing cell and the first capture probe attached thereto to generate a first spatially barcoded polynucleotide comprising (i) all or a portion of the sequence of the first analyte of the ABM-expressing cell or reverse complement thereof; and (ii) the spatial barcode sequence or reverse complement thereof; and (e) using the reporter oligonucleotide and the second capture probe attached thereto to generate a second spatially barcoded polynucleotide comprising (i) the reporter barcode sequence or reverse complement thereof and (ii) the spatial barcode sequence or reverse complement thereof, wherein the method further comprises: (i) using the first spatially barcoded polynucleotide to determine all or a portion of the sequence of the first analyte attached to the first capture probe, wherein the determining comprises sequencing the first spatially barcoded polynucleotide or an amplicon thereof; (ii) using the second spatially barcoded polynucleotide to determine the reporter barcode sequence of the reporter oligonucleotide attached to the second capture probe, wherein the determining of the reporter barcode sequence comprises sequencing the second spatially barcoded polynucleotide or an amplicon thereof; and (iii) using the determined sequences to determine that the ABM expressed by the ABM-expressing cell binds to the antigen.

In some embodiments, the ABM expressed by the ABM-expressing cell is an immune receptor and the contacting of (a) labels the ABM-expressing cell with the reporter oligonucleotide conjugated antigen. In some embodiments, the immune receptor is a BCR or a TCR. In some embodiments, the immune receptor is an Fc receptor. In some embodiments, the antigen is an antibody drug conjugate.

In some embodiments, the ABM expressed by the ABM-expressing cell is a secreted antibody and wherein the contacting of (a) labels the secreted antibody with the reporter oligonucleotide conjugated antigen. In some embodiments, prior to or during (b), the labeled secreted antibody is in proximity to the ABM-expressing cell.

In some embodiments, the reporter oligonucleotide further comprises a capture handle sequence that is complementary to the second capture sequence of the second capture domain.

In some embodiments, the first capture sequence of the first capture domain and the second capture sequence of the second capture domain are identical. In some embodiments, the first capture sequence of the first capture domain and the second capture sequence of the second capture domain are different.

In some embodiments, the first capture sequence of the first capture domain is a homopolymeric sequence. In some embodiments, the homopolymeric sequence is a polyT sequence. In some embodiments, the first capture sequence of the first capture domain is a defined non-homopolymeric sequence. In some embodiments, the defined non-homopolymeric sequence specifically binds to a nucleic acid sequence in the first analyte encoding a region of the ABM expressed by the ABM-expressing cell. In some embodiments, the ABM is selected from: a TCR alpha chain, a TCR beta chain, a TCR gamma chain, a TCR delta chain an immunoglobulin kappa light chain, an immunoglobulin lambda light chain, and an immunoglobulin heavy chain. In some embodiments, the region of the ABM is a constant region of the ABM or a variable region of the ABM.

In some embodiments, the second capture sequence of the second capture domain is a homopolymeric sequence.

In some embodiments, the second capture sequence of the second capture domain is a defined non-homopolymeric sequence.

In some embodiments, the homopolymeric sequence is a polyT sequence.

In some embodiments, the capture handle sequence is blocked prior to (a), optionally wherein the capture handle sequence is blocked by a blocking probe, optionally wherein the blocking probe is removed prior to (c).

In some embodiments, the method further comprises amplifying the first spatially barcoded polynucleotide or amplicon thereof with a first primer that specifically binds to a functional sequence of the first capture probe or reverse complement thereof and a second primer that binds to a nucleic acid sequence encoding a variable region of the ABM expressed by the ABM-expressing cell or reverse complement thereof. In some embodiments, the first primer and the second primer flank the spatial barcode of the first spatially barcoded polynucleotide.

In some embodiments, the tissue sample is in contact with the substrate comprising the array of capture probes attached thereto during (a), (b), or (a) and (b).

In some embodiments, the method comprises, following (a), releasing the first analyte from the ABM-expressing cell of the tissue sample; and migrating the first analyte to the substrate comprising the array of capture probes attached thereto.

In some embodiments, the substrate comprising the array of capture probes attached thereto is a second substrate, wherein the tissue sample is mounted on a first substrate during (a), and wherein the method comprises, following (a), mounting the first substrate on a first member of a support device, the first member configured to retain the first substrate; mounting the second substrate on a second member of the support device, the second member configured to retain the second substrate, applying a reagent medium to the first substrate and/or the second substrate, the reagent medium comprising a permeabilization agent, operating an alignment mechanism of the support device to move the first member and/or the second member such that a portion of the tissue sample comprising the ABM-expressing cell is aligned with a portion of the array of capture probes and within a threshold distance of the array of capture probes, and such that the portion of the tissue sample and the capture probe contact the reagent medium, wherein the permeabilization agent releases the first analyte from the ABM-expressing cell.

In some embodiments, the first analyte comprises a nucleic acid sequence encoding a variable sequence and a constant sequence of the ABM. In some embodiments, the variable sequence is a VDJ sequence. In some embodiments, the ABM-expressing cell is a B cell, a natural killer (NK) cell, a T-Reg cell, a CAR-T cell, or a T cell. In some embodiments, the tissue sample is a tissue section, optionally a fresh frozen tissue section, a fixed tissue section, or an FFPE tissue section. In some embodiments, the first spatially barcoded polynucleotide and the second spatially barcoded polynucleotide comprise the same spatial barcode sequence or reverse complement thereof. In some embodiments, the method comprises amplifying the first spatially barcoded polynucleotide to generate a spatially barcoded nucleic acid library member comprising the variable sequence and the constant sequence. In some embodiments, the method further comprises removing all or a portion of the nucleic acid sequence encoding the constant sequence from the spatially barcoded nucleic acid library member or amplicon thereof.

In some embodiments, the antibody is a monoclonal antibody. In some embodiments, the antibody is a polyclonal antibody. In some embodiments, the antibody is a bispecific antibody. In some embodiments, the antibody is a secreted antibody. In some embodiments, the antibody is a surface-bound antibody. In some embodiments, the antigen binding fragment is selected from the group consisting of a Fab, an Fab', an F(ab')2, an Fv, and an Fc. In some embodiments, the antigen is a component of a vaccine. In some embodiments, the antigen is a chimeric antigen receptor. In some embodiments, the chimeric antigen receptor is selected from the group consisting of axicabtagene ciloleucel (YESCARTA^{®}), tisagenlecleucel (KYMRIAH^{®}), and brexucabtagene autoleucel (TECARTUS).

In some embodiments, the reporter oligonucleotide conjugated antigen further comprises an enzyme tag, a fluorophore tag, a quantum dot, a covalently or non-covalently attached protein tag, a covalently or non-covalently attached peptide tag, a fused tag, a carbohydrate tag, or a small molecule tag. In some embodiments, the covalently or non-covalently attached protein tag is selected from BCCP (biotin carboxyl carrier protein) tag, glutathione-S-transferase tag, green fluorescent protein tag, halo-tag, SNAP tag, CLIP tag, HUH tag, maltose binding protein tag, Nus tag, thioredoxin tag, Fc tag, and CRDSAT tag. In some embodiments, the covalently or non-covalently attached peptide tag is selected from ALFA tag, AviTag, C-tag, calmodulin tag, polyglutamate tag, polyarginine tag, E tag, FLAG tag, HA tag, His tag, Myc tag, NE tag, Rho1D4 tag, S tag, SBP tag, Softag 1, Softag 3, Spot tag, Strep tag, T7 tag, TC tag, Ty tag, V5 tag, VSV tag, Xpress tag, Isopeptag, Spy tag, Snoop tag, DogTag, and SdyTag.

In some embodiments, the conjugating the antibody therapeutic to the oligonucleotide comprises one or more of the following: ReACT chemistry, direct/non-specific (lysine) click chemistry, site-specific sortase motif-dependent conjugation, site-specific photo-crosslinking-dependent conjugation, site-specific conformation-dependent conjugation, and nitrilotriacetate conjugation.

In some embodiments, the reporter oligonucleotide is coupled to a constant region of the antibody.

In some embodiments, the isolating step comprises capturing the reporter oligonucleotide.

In some embodiments, the antibody therapeutic is selected from the group consisting of abciximab, adalimumab, adalimumab-atto, ado-trastuzumab emtansine, alemtuzumab, alirocumab, atezolizumab, avelumab, basiliximab, belimumab, bevacizumab, bezlotoxumab, blinatumomab, brentuximab vedotin, brodalumab, canakinumab, capromab pendetide, certolizumab pegol, cetuximab, daclizumab, daratumumab, denosumab, dinutuximab, dupilumab, durvalumab, eculizumab, elotuzumab, evolocumab, golimumab, ibritumomab tiuxetan, idarucizumab, infliximab, infliximab-abda, infliximab dyyb, ipilimumab, ixekizumab, mepolizumab, natalizumab, necitumumab, nivolumab, oblitoxaximab, obinutuzumab, ocrelizumab, ofatumumab, olaratumab, omalizumab, palivizumab, panitumumab, pembrolizumab, pertuzumab, ramucirumab, ranibizumab, raxibacumab, reslizumab, rituximab, secukinumab, siltuximab, tocilizumab, trastuzumab, ustekinumab, vedolizumab, sarilumab, guselkumab, inotuzumab ozogamicin, adalimumab-adbm, gemtuzumab ozogamicin, bevacizumab-awwb, benralizumab, emicizumab-kxwh, trastuzumab-dkst, infliximab-qbtx, ibalizumab-uiyk, tildrakizumab-asmn, burosumab-twsa, erenumab-aooe, tositumomab, mogamulizumab, moxetumomab pasudotox, cempilimab, and polatuzumab vedotin.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** shows a flow chart depicting a scheme for characterizing an immune response to a therapeutic antibody using methods provided herein.
**FIG. 2** shows a schematic diagram of an exemplary capture probe.
**FIG. 3** is a schematic illustrating an exemplary cleavable capture probe.
**FIG. 4** shows is a schematic diagram of an exemplary multiplexed spatially-barcoded feature.
**FIG. 5** is a schematic diagram of an exemplary analyte capture agent.
**FIG. 6** is a schematic diagram depicting an exemplary interaction between a feature-immobilized capture probe and an analyte capture agent.
**FIG. 7** is a schematic diagram depicting an exemplary sandwiching process.
**FIG. 8A** is a perspective view of an example sample handling apparatus in a closed position in accordance with some example implementations.
**FIG. 8B** is a perspective view of the example sample handling apparatus in an open position in accordance with some example implementations.
**FIG. 9A** shows an exemplary sandwiching process where a first substrate including a biological sample and a second substrate (e.g., including spatially barcoded capture probes) are brought into proximity with one another.
**FIG. 9B** shows a fully formed sandwich configuration creating a chamber formed from one or more spacers, the first substrate, and the second substrate, in accordance with some example implementations.
**FIGS. 10A-10C** depict a side view and a top view of an angled closure workflow for sandwiching a first substrate and a second substrate in accordance with some example implementations.
**FIG. 11A** depicts an exemplary capture probe with a capture sequence that specifically binds to a nucleic acid sequence encoding a constant region of an antigen binding molecule.
**FIG. 11B** depicts an exemplary poly(A) capture of an analyte encoding an antigen binding molecule with a poly(T) capture domain.
**FIG. 12** shows an exemplary analyte enrichment strategy following analyte capture on the array.
**FIG. 13** shows a sequencing strategy with a primer specific complementary to the sequencing flow cell attachment sequence (e.g., P5) and a custom sequencing primer complementary to a portion of a constant region of an analyte.
**FIG. 14** shows an exemplary nucleic acid library preparation method to remove a portion of an analyte sequence via double circularization of a member of a nucleic acid library.
**FIG. 15** depicts another exemplary workflow for processing a double-stranded circularized nucleic acid product.
**FIG. 16** shows an exemplary nucleic acid library preparation method to remove all or a portion of a constant sequence of an analyte from a member of a nucleic acid library via circularization.
**FIG. 17** shows an exemplary nucleic acid library method to reverse the orientation of an analyte sequence in a member of a nucleic acid library.
**FIG. 18** is a schematic diagram showing an exemplary feature comprising an attached first comprising a polyT capture domain and second probe comprising a poly(GI) capture domain.
**FIG. 19A** is a workflow schematic illustrating exemplary steps for generating a spatially-barcoded sample for analysis and for use in further steps of the methods described herein.
**FIG. 19B** is a workflow schematic illustrating exemplary steps for specific binding of the extended first probe with the second probe.
**FIG. 19C** is a workflow schematic illustrating exemplary steps for generating a spatially-barcoded sample for analysis that allows for the sequencing of the target nucleic acid from both the 3' end and the 5' end.
**FIG. 19D** is a schematic diagram showing an exemplary spatially-barcoded sample for analysis generated using the methods described herein.
**FIGS. 20A** - **20J** depict an exemplary workflow for detecting and/or determining spatial location of a target polynucleotide of interest.
**FIG. 21** shows an example workflow for associating a reporter molecule on an antibody (e.g., an ADA, and ADC, or an AbTx) using an antibody-binding protein.
**FIG. 22A** shows an example of a reporter oligonucleotide conjugated to an antigen (*e.g.,* an antibody or an MHC multimer).
**FIG. 22B** depicts example schematics of labeling agents disclosed herein.
**FIG. 22C** depicts an example schematic of a labeled cell bound to a labelling agent disclosed herein.
**FIG. 23A** schematically shows an example of an oligonucleotide (e.g., reporter oligonucleotide) hybridized to a capture probe. **FIG. 23B** schematically shows an example workflow for processing nucleic acid molecules. **FIG. 23C** schematically shows another example workflow for processing nucleic acid molecules. **FIG. 23D** schematically shows another example workflow for processing nucleic acid molecules.
**FIG. 24** depicts exemplary capture probes attached to a substrate (*e.g*., a substrate feature).
**FIG. 25** illustrates an exemplary count matrix that can be obtained from experiments described in **Examples 5** and **7.**
**FIG. 26** illustrates exemplary two distinct populations of single cells in the resulting clustering and visualization analyses, indicating that these populations of cells can recognize distinct (*e.g*., disjoint) sets of the five antigens used in **FIG. 25****.**
**FIG. 27A** depicts an exemplary workflow for identifying anti-drug antibodies (ADAs) or drug-reactive antibodies (DRAs).
**FIG. 27B** depicts an exemplary workflow for identifying a patient specific engineered antibody therapeutic (AbTx).
**FIG. 27C** depicts an exemplary workflow for characterizing anti-drug antibody (ADA) binding to anti-therapeutic (AbTx).
**FIG. 27D** depicts an exemplary workflow for characterizing anti-drug antibody (ADA) binding to anti-therapeutic (AbTx).
**FIG. 28** shows a labeling scheme for conjugating variants of an antigen with reporter oligonucleotides.
**FIG. 29** depicts a system diagram illustrating an example of an analysis system.
**FIG. 30** illustrates an exemplary count matrix that can be obtained from experiments described in **Example 8.**
**FIG. 31** illustrates an exemplary count matrix that can be obtained from experiments described in **Example 9.**
**FIG. 32** depicts a flowchart illustrating an example of a process for analyzing and visualizing data associated with cells expressing one or more antigen binding molecules.
**FIG. 33** shows a computer system that is programmed or otherwise configured to implement methods provided herein.
**FIG. 34** is a schematic diagram showing reverse transcription of the target nucleic acid with a first primer and the addition of a capture sequence capable of hybridizing to a capture domain of a capture probe.
**FIG. 35** is a schematic diagram showing capture and extension on an array of the extension product (e.g., cDNA product) shown in **FIG. 34** and extension of the capture probe and the captured extension product (e.g., cDNA product) followed by release of the extended capture product.

### DETAILED DESCRIPTION OF THE DISCLOSURE

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes.

A better understanding of drug-reactive antibody repertoire can guide drug development and discovery. Successfully identified and validated drug-reactive antibodies have several important uses, each of which can be addressed by the methods described herein. Described herein is a method enabling the capture of drug-specific antibodies. A method described herein utilizes a reporter oligonucleotide conjugated antigen to profile samples from a subject treated with an antibody-drug conjugate (ADC) and/or an antibody therapeutic (AbTX). A method described herein enables quantitation of many features of the subject's response to the ADC or AbTX. For example, the number of cells that express drug-reactive antibody can be quantified. In another example, a diversity of anti-drug antibody response in the subject can be quantified. In yet another example, the subject's response to the ADC or AbTX can be monitored. A method described herein can assess whether or not an engineered or modified version of an existing ADC or AbTX is recognized by a pre-existing immune response in the subject.

In the following detailed description, reference is made to the accompanying drawings, which form a part hereof. In the drawings, similar symbols generally identify similar components, unless context dictates otherwise. The illustrative alternatives described in the detailed description, drawings, and claims are not meant to be limiting. It will be readily understood that the aspects, as generally described herein, and illustrated in the drawings, can be arranged, substituted, combined, and designed in a wide variety of different configurations, all of which are explicitly contemplated and make part of this application.

The section headings used herein are for organizational purposes only and are not to be construed as limiting the subject matter described.

### Definitions

Unless otherwise defined, all terms of art, notations and other scientific terms or terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The terms "a," "an," and "the," as used herein, generally refers to singular and plural references unless the context clearly dictates otherwise.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. If the degree of approximation is not otherwise clear from the context, "about" means either within plus or minus 10% of the provided value, or rounded to the nearest significant figure, in all cases inclusive of the provided value. The term "about" may indicate the designated value ± up to 10%, up to ± 5%, or up to ± 1%.

Headings, e.g., (a), (b), (i) etc., are presented merely for ease of reading the specification and claims. The use of headings in the specification or claims does not require the steps or elements be performed in alphabetical or numerical order or the order in which they are presented.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, the use of these terms in the specification does not by itself connote any required priority, precedence, or order.

The term "real time," as used herein, can refer to a response time of less than about 1 second, a tenth of a second, a hundredth of a second, a millisecond, or less. The response time can be greater than 1 second. Real time can refer to simultaneous or substantially simultaneous processing, detection or identification.

The term "subject," as used herein, generally refers to an animal, such as a mammal (*e.g.,* human) or avian (*e.g.,* bird), or other organism, such as a plant. For example, the subject can be a vertebrate, a mammal, a rodent (*e.g.,* a mouse), a primate, a simian or a human. Animals can include, but are not limited to, farm animals, sport animals, and pets. A subject can be a healthy or asymptomatic individual, an individual that has or is suspected of having a disease (*e.g*., cancer) or a pre-disposition to the disease, and/or an individual that is in need of therapy or suspected of needing therapy. A subject can be a patient. A subject can be a microorganism or microbe (*e.g*., bacteria, fungi, archaea, viruses).

The term "genome," as used herein, generally refers to genomic information from a subject, which can be, for example, at least a portion or an entirety of a subject's hereditary information. A genome can be encoded either in DNA or in RNA. A genome can comprise coding regions (*e.g*., that code for proteins) as well as non-coding regions. A genome can include the sequence of all chromosomes together in an organism. For example, the human genome ordinarily has a total of 46 chromosomes. The sequence of all of these together can constitute a human genome.

The terms "adaptor(s)", "adapter(s)" and "tag(s)" can be used synonymously. An adaptor or tag can be coupled to a polynucleotide sequence to be "tagged" by any approach, including ligation, hybridization, or other approaches.

The term "sequencing," as used herein, generally refers to methods and technologies for determining the sequence of nucleotide bases in one or more polynucleotides (*e.g*., nucleic acid barcode molecules). The polynucleotides can be, for example, nucleic acid molecules such as deoxyribonucleic acid (DNA) or ribonucleic acid (RNA), including variants or derivatives thereof (*e.g.*, single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing can be performed using nucleic acid amplification, polymerase chain reaction (PCR) (*e.g.,* digital PCR, quantitative PCR, or real time PCR), or isothermal amplification. Such systems can provide a plurality of raw genetic data corresponding to the genetic information of a subject (*e.g*., human), as generated by the systems from a sample provided by the subject. In some examples, such systems provide sequencing reads (also "reads" herein). A read can include a string of nucleic acid bases corresponding to a sequence of *e.g.,* a nucleic acid barcode molecule that has been sequenced. In some situations, systems and methods described herein can be used with proteomic information.

As used herein, the term "barcoded nucleic acid molecule" generally refers to a nucleic acid molecule that results from, for example, the processing of a nucleic acid barcode molecule (e.g., a capture probe comprising a spatial barcode sequence) with a nucleic acid sequence (e.g., nucleic acid sequence complementary to a nucleic acid primer sequence encompassed by the nucleic acid barcode molecule). The nucleic acid sequence may be a targeted sequence or a non-targeted sequence. For example, in the methods and systems described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). A barcoded nucleic acid molecule may serve as a template, such as a template polynucleotide, that can be further processed (e.g., amplified) and sequenced to obtain the target nucleic acid sequence. For example, in the methods and systems described herein, a barcoded nucleic acid molecule may be further processed (e.g., amplified) and sequenced to obtain the nucleic acid sequence of the mRNA.

The term "sample," as used herein, generally refers to a biological sample of a subject. The biological sample can include any number of macromolecules, for example, cellular macromolecules. The sample can include one or more cells. The sample can include one or more microbes. The biological sample can be a nucleic acid sample or protein sample. The biological sample can also be a carbohydrate sample or a lipid sample. The biological sample can be derived from another sample. The sample can be a tissue sample. Exemplary tissue samples are disclosed herein.

The term "barcode" is used herein to refer to a label, or identifier, that conveys or is capable of conveying information (*e.g*., information about an analyte in a sample, a bead, a feature, a capture probe, and/or a nucleic acid barcode molecule). A barcode can be part of an analyte, a capture probe, a reporter oligonucleotide, an analyte capture agent, or nucleic acid barcode molecule, or independent of an analyte, a capture probe, a reporter oligonucleotide, an analyte capture agent, or nucleic acid barcode molecule. A barcode can be attached to an analyte, a capture probe, a reporter oligonucleotide, an analyte capture agent, or nucleic acid barcode molecule in a reversible or irreversible manner. A particular barcode can be unique relative to other barcodes. Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for or facilitates identification and/or quantification of individual sequencing-reads. A barcode can be configured for use as a fluorescent barcode. For example, a barcode can be configured for hybridization to fluorescently labeled oligonucleotide probes. Barcodes can be configured to spatially resolve molecular components found in biological samples, for example, at single-cell resolution (*e.g*., a barcode can be or can include a "spatial barcode"). A barcode may include two or more sub-barcodes that together function as a single barcode. For example, a polynucleotide barcode can include two or more polynucleotide sequences (*e.g*., sub-barcodes). The two or more sub-barcodes may be separated by one or more non-barcode sequences. The two or more sub-barcodes may not be separated by non-barcode sequences.

A barcode can include one or more unique molecular identifiers (UMIs). Generally, a unique molecular identifier is a contiguous nucleic acid segment or two or more non-contiguous nucleic acid segments that function as a label or identifier for a particular analyte, or for a nucleic acid barcode molecule that binds a particular analyte (*e.g*., mRNA) via the capture sequence.

A UMI can include one or more specific polynucleotides sequences, one or more random nucleic acid and/or amino acid sequences, and/or one or more synthetic nucleic acid and/or amino acid sequences. The UMI may be a nucleic acid sequence that does not substantially hybridize to analyte nucleic acid molecules in a biological sample. The UMI may have less than 80% sequence identity (*e.g*., less than 70%, 60%, 50%, or less than 40% sequence identity) to the nucleic acid sequences across a substantial part (*e.g.,* 80% or more) of the nucleic acid molecules in the biological sample. These nucleotides can be completely contiguous, i.e., in a single stretch of adjacent nucleotides, or they can be separated into two or more separate subsequences that are separated by 1 or more nucleotides.

The terms "biological particle" is used herein to generally refer to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or may include a matrix (*e.g*., a gel or polymer matrix) comprising a cell or one or more constituents from a cell (*e.g.,* cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix.

The term "macromolecular constituent," as used herein, generally refers to a macromolecule contained within or from an biological particle. The macromolecular constituent may comprise a nucleic acid. In some cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may be or include a primer. The molecular tag may be, or include, a protein. The molecular tag may include a polypeptide. The molecular tag may be a barcode.

### OVERVIEW

A drug, such as an antibody or antibody-based drug, can elicit an immune response when administered to a subject. In some such cases, the subject can produce antibodies against the antibody or antibody-based drug (*i.e*., an antigen binding molecule; anti-drug antibody or drug-reactive antibody) that can bind to the antibody or antibody-based drug (*i.e*., the antigen), thus inhibiting and/or preventing efficacy of the antibody or antibody-based drug. Such drug-reactive antibodies (DRAs) or anti-drug antibodies (ADAs) can result in decreased effectiveness of the antibody or antibody-based drug and/or side effects occurring as a result of the elicited immune response. Interaction between an antigen and an antigen binding molecule can be beneficial. For example, interaction between an antibody of a subject (*i.e*., the antigen binding molecule) and an antigen (*i.e*., a vaccine composition) can indicate that immunity can be conferred to the subject upon administering the antigen to the subject.

Described herein are methods for characterizing anti-drug antibodies (ADAs) or drug-reactive antibodies (DRAs) via barcoding. The terms "anti-drug antibody (ADA)" and "drug-reactive antibody (DRA)" are used interchangeably herein. Further described herein is a method for identifying an antigen binding molecule that can interact with an antigen conjugated to a reporter oligonucleotide (also referred to herein as a reporter oligonucleotide conjugated antigen), wherein the reporter oligonucleotide includes a reporter barcode. The reporter oligonucleotide conjugated antigen may be an antibody therapeutic (AbTx) or antibody drug conjugate (ADC). An antigen binding molecule can be an immune receptor, an antibody, or antigen binding fragment thereof produced by a subject. Methods described herein can be useful for identifying an antigen binding molecule (e.g., immune receptor, antibody, or antigen binding fragment thereof) that can interact with an antigen. For example, an antibody from a subject can be identified that can interact with an antigen that subject has been exposed to.

Methods for identifying an antigen binding molecule that can interact with an antigen can include (a) contacting a biological sample comprising one or more cells expressing an antigen-binding molecule (ABM) with a reporter oligonucleotide conjugated antigen, wherein the reporter oligonucleotide comprises a reporter barcode sequence and the antigen is an antibody therapeutic and/or antibody drug conjugate; (b) attaching a first analyte of an ABM-expressing cell of the biological sample to a first capture domain of a first capture probe of an array of capture probes attached to a substrate, the first capture probe comprising (i) a spatial barcode sequence and (ii) the first capture domain, the first capture domain comprising a first capture sequence, wherein the first analyte of the ABM-expressing cell comprises a sequence or portion of a sequence encoding the ABM expressed by the ABM-expressing cell or a reverse complement thereof, wherein the ABM expressed by the ABM-expressing cell binds to the antigen; and (c) using the first analyte of the ABM-expressing cell and the first capture probe attached thereto to generate a first spatially barcoded polynucleotide or amplicon thereof comprising (i) all or a portion of the sequence of the first analyte of the ABM-expressing cell or reverse complement thereof and (ii) the spatial barcode sequence or reverse complement thereof.

The one or more cells expressing an ABM, e.g., the ABM-expressing cell, can include, for example, a B cell, natural killer (NK) cell, a T-Reg cell, a CAR-T cell, a lymphocyte, T cell or a combination thereof.

The biological sample may be a tissue sample.

The ABM expressed by the ABM-expressing cell may be an immune receptor and the contacting of (a) labels the ABM-expressing cell with the reporter oligonucleotide conjugated antigen. The immune receptor can be a BCR or a TCR. The immune receptor can be an Fc receptor. In some cases wherein the antigen is an ADC, it may be advantageous to analyze recruitment of effector cells (e.g., effector cells expressing an Fc receptor) to the reporter oligonucleotide conjugated antigen.

The ABM expressed by the ABM-expressing cell may be a secreted antibody and the contacting of (a) labels the secreted antibody with the reporter oligonucleotide conjugated antigen. Prior to or during (b), the labeled secreted antibody may be in proximity to the ABM-expressing cell, such that analytes from the ABM-expressing cell and the reporter oligonucleotide of the labeled secreted antibody are captured by capture probes within the same feature of a capture probe array, e.g., capture probes comprising the same spatial barcode. For example, the labeled secreted antibody may be within 100 microns of the ABM-expressing cell. By way of other example only, the labeled secreted antibody may be within 55 microns of the ABM-expressing cell.

(d) may further comprise attaching the reporter oligonucleotide to a second capture probe of the array, the second capture probe of the array comprising (i) the spatial barcode sequence and (ii) a second capture domain comprising a second capture sequence; and (e) further comprises using the reporter oligonucleotide and the second capture probe attached thereto to generate a second spatially barcoded polynucleotide or amplicon thereof comprising (i) the reporter barcode sequence or reverse complement thereof and (ii) the spatial barcode sequence or reverse complement thereof. Reporter oligonucleotides (also referred to herein as analyte-binding moiety barcode domains of an analyte capture agents), are further disclosed herein, e.g., the section entitled "Systems and Methods for Spatial Analysis".

The method may further comprise (d) using the first spatially barcoded polynucleotide or amplicon thereof to determine the sequence of the first analyte attached to the first capture probe. The determining of the sequence of the first analyte may comprise sequencing the first spatially barcoded polynucleotide or amplicon thereof. (d) may further comprise using the second spatially barcoded polynucleotide or amplicon thereof to determine the reporter barcode sequence of the reporter oligonucleotide attached to the second capture probe. Determining the sequence of the reporter barcode sequence may comprise sequencing the second spatially barcoded polynucleotide or amplicon thereof. The method may further comprise (e) using the determined sequences to determine that the ABM expressed by the ABM-expressing cell binds to the antigen. By way of example only, by associating the first analyte and the reporter barcode sequence that identifies the antigen to the same spatial barcode, e.g., to the same location on the array of spatially barcoded capture probes, it may be determined that the ABM expressed by the ABM-expressing cell binds to the antigen.

The tissue sample can include one or more ABMs. The tissue sample may be obtained from a subject (e.g., a human). The subject may be a subject that was previously exposed to or may be exposed to the antigen. For example, the subject may be currently undergoing or is a candidate for a therapy, such as a subject that has received, is receiving, or may receive in the future an antibody therapeutic or antibody drug conjugate. Methods described herein can identify whether an antigen binding molecule in the subject can bind an antigen. Other exemplary subjects are described herein. The tissue sample obtained from the subject may be a sample comprising an immune cell. Exemplary samples that comprise immune cells include, e.g., spleen, lymph node, tonsil, bone marrow sample, tumor samples, and the like.

The tissue sample obtained from the subject may be a diseased tissue sample. For example, the tissue sample can comprise tumor cells. The tissue sample obtained from the subject may be a healthy tissue sample. The tissue sample obtained from the subject may be a tissue sample comprising or suspected of comprising the antigenic target to which the antibody therapeutic or antibody drug conjugate is expected to bind.

The tissue sample may be in contact with the substrate comprising the array of capture probes during (a), (b), or (a) and (b). The tissue sample may be in contact with the array of capture probes during (a), (b), or (a) and (b). A portion of the tissue sample comprising the ABM-expressing cell may be in contact with the first capture probe of the array of capture probes and optionally the second capture probe of the array of capture probes. The method may comprise, following (a), releasing the first analyte from the ABM-expressing cell of the tissue sample; and migrating the first analyte to the substrate comprising the array of capture probes attached thereto.

In some cases, it may be advantageous to contact the tissue sample with the reporter oligonucleotide conjugated antigens while the tissue sample is mounted on a regular slide, and migrate analytes from the slide-mounted tissue to the array of capture probes for capture in a manner that preserves spatial context. Accordingly, the tissue sample may be processed according to a "sandwiching process" for the release and migration of the analytes to the array of capture probes in a manner that preserves their spatial context. Sandwiching processes are disclosed in further detail herein.

Methods described herein can include isolating the antigen binding molecule bound to the antigen at least by using a reporter oligonucleotide. An antigen binding molecule isolated by such a method can have affinity to the antigen. Methods can include identifying the antigen binding molecule. For example, an identified antigen binding molecule can be an anti-drug ABM, such as an anti-drug antibody (ADA) or a drug-reactive antibody (DRA). A method described herein utilizes an oligonucleotide conjugated antigen that is an antibody therapeutic (AbTx) or antibody drug conjugate (ADC) to profile samples from a subject treated with the ADC and/or AbTX. A method described herein enables quantitation of features of the subject's immune response to the ADC or AbTX. For example, the number of cells that express drug-reactive antibody can be quantified. In another example, a diversity of anti-drug antibody response in the subject can be quantified. In yet another example, the subject's response to the ADC or AbTX can be monitored. A method described herein can assess whether or not an engineered or modified version of an existing ADC or AbTX is recognized by a pre-existing immune response in the subject.

A candidate AbTX or ADC can be converted into a reporter oligonucleotide conjugated antigen by conjugated a reporter oligonucleotide to the constant region or to a specific site of the AbTX or ADC via a number of methods described herein. This can then be used to stain a tissue sample containing B cells from a subject or a naive donor or a tissue sample comprising engineered cells expressing a known drug-reactive antibody. Using barcoding as described herein, the binding of an antigen conjugated to a reporter oligonucleotide to a memory B cell or a plasma cell or a plasmablast can be detected.

The ADAs isolated using the methods described herein can be used to validate the efficacy of AbTX and ADC re-engineering. By expressing and labeling these ADAs with reporter oligonucleotides and staining engineered cells or tissue samples comprising such engineered cells expressing mutants of the AbTX or ADC of choice, the same workflow described herein, or workflows described in PCT/US2021/030976, can be used to confirm that the new AbTX/ADC variant is not bound by the same antibodies. Furthermore, use of these antibodies in functional assays such as antibody-dependent cellular cytotoxicity (ADCC), antibody-dependent cellular phagocytosis (ADCP), antibody-dependent complement deposition (ADCD), and other assays can be used to identify ADAs with therapeutic value - such ADAs can be developed as drugs to be administered to subjects experiencing side effects induced by an AbTX or ADC. Such ADAs can also be deployed as reference standards in traditional serum-based ADA assays. This is especially important for AbTX and ADCs that have been engineered to be recycled efficiently by addition of Fc domains that can be recognized by the neonatal Fc receptor (FcRn, product of the FCGRT gene). The methods described herein can also be more broadly used to characterize the response in subjects with diverse immunoglobulin haplotypes, and in combination with traditional statistical models such as contingency tests, GWAS, PheWAS, and cellular assays discover genomic and other elements associated with the development of ADAs.

A method described herein can quantify a subject's response to an antigen. For example, the method can include (a) contacting (i) a tissue sample comprising one or more cells from the subject wherein the one or more cells express at least one antigen binding molecule with (ii) a reporter oligonucleotide conjugated antigen, wherein the reporter oligonucleotide comprises a reporter barcode and wherein the antigen is an antibody therapeutic; (b) measuring the number of cells that express the at least one antigen binding molecule that bind to the antibody therapeutic.

A method described herein can determine diversity of a subject's immune response to an antigen. For example, the method can include (a) contacting (i) a tissue sample comprising one or more cells from the subject wherein the one or more cells express one or more antigen binding molecules with (ii) a reporter oligonucleotide conjugated antigen, wherein the reporter oligonucleotide comprises a reporter barcode and wherein the antigen is an antibody therapeutic; (b) sequencing the one or more antigen binding molecules; and (c) identifying the one or more antigen binding molecules that bind to the antibody therapeutic to determine the diversity of a subject's immune response to the antibody therapeutic. The method can further include (d) isolating the one or more antigen binding molecules. The diversity of a subject's immune response may be production of different antibodies and/or antibody lineages.

A method described herein can monitor a subject's response to an antibody therapeutic or antibody drug conjugate (ADC). For example, the method can include (a) contacting a tissue sample comprising one or more cells from the subject wherein the one or more cells express one or more antigen binding molecules with (ii) a reporter oligonucleotide conjugated antigen, wherein the reporter oligonucleotide comprises a reporter barcode; (b) identifying the one or more antigen binding molecules; and (c) measuring the number of cells that express at least one antigen binding molecule that bind to the antibody therapeutic or ADC over a course of time. The method can further include (d) isolating the one or more antigen binding molecule. The course of time may be about bi-weekly, about monthly, or about yearly.

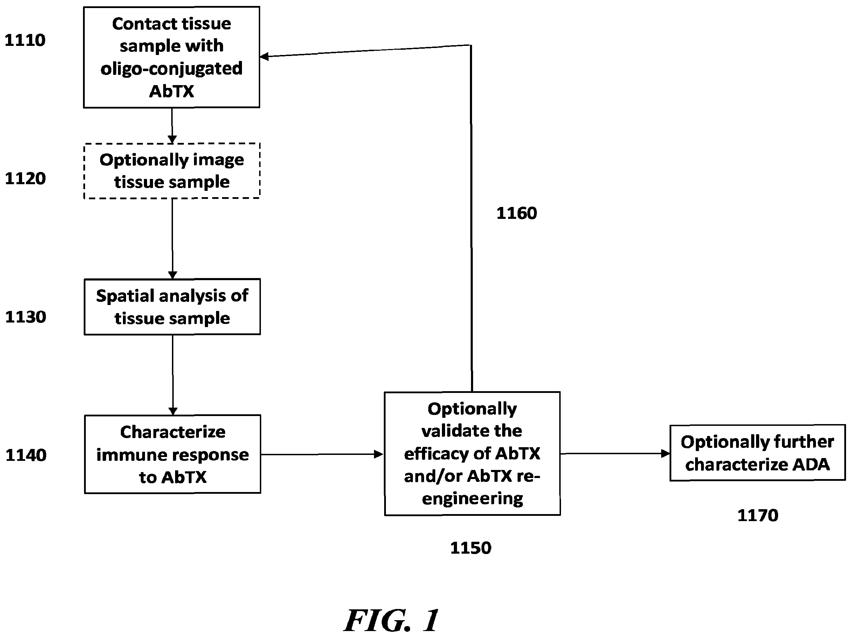

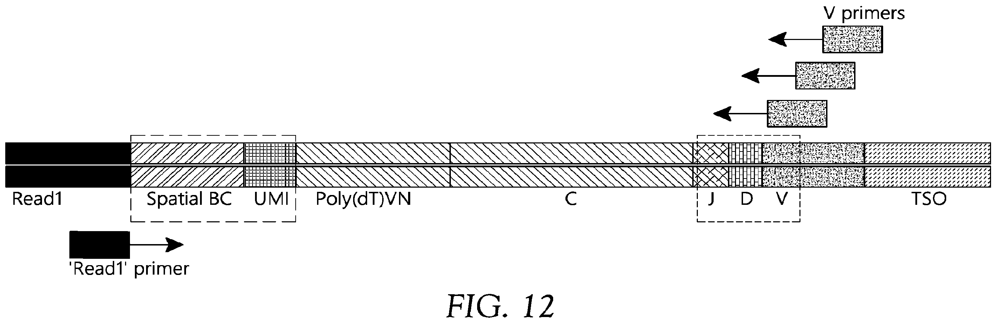

An exemplary workflow is described in FIG. 1. In operation 1110, a tissue sample (such as a tissue sample comprising one or more cells expressing an ABM) may be contacted with an antibody therapeutic (AbTx) complex, e.g., a reporter oligonucleotide conjugated to an antibody therapy (AbTx), such as a therapeutic antibody or an antibody drug conjugate. The AbTx complex may include a detectable tag (such as a fluorophore) as described elsewhere herein. Optionally, in operation 1120, the tissue sample may be imaged, e.g., to image the detectable tag of the AbTx complex in the tissue sample. In operation 1130, the tissue sample is subjected to a spatial analysis methodology as disclosed herein. For example, analytes from cells expressing an ABM that binds the AbTx complex, and the reporter oligonucleotide of the AbTx complex can be spatially barcoded with the same spatial barcode, thereby generating spatially barcoded polynucleotides for subsequent processing and analysis (such as by nucleic acid sequencing) in operation 1140. Further details on spatial analysis methodologies are disclosed herein. Operation 1130 may include generating spatially barcoded polynucleotides, wherein the same spatial barcode sequence is associated with the reporter barcode sequence or reverse complement thereof of the reporter oligonucleotide of the AbTx complex, and with analytes from a cell expressing an ABM that binds the AbTx complex, such as antibody or BCR sequences from a B cell. See, e.g., FIGS. 12A-12D for a representative barcoding scheme). Thus, in operation 1140, a common barcode (e.g., a spatial barcode) sequence can be utilized to associate a specific AbTx with an immune response, e.g., a specific BCR of a B cell. As such, this allows the identification of ABMs that bind, e.g., specifically bind to the AbTx, such as putative anti-drug antibodies (ADAs), which can optionally be further analyzed in operations 1150, 1160, and/or 1170. For example, in operation 1150, the putative ADAs identified in operation 1140 can themselves be used to validate the efficacy of an AbTx (e.g., therapeutic antibody or antibody drug conjugate) and/or be utilized to validate the efficacy of an AbTx upon re-engineering (e.g., operations 1110-1140 are repeated in operation 1160 with one or more re-engineered AbTx with the goal of observing an altered or reduced ADA response). For example, operation 1150 can include expressing one or more of the putative ADAs identified in operation 1140 and labeling these ADAs with oligonucleotides, e.g., reporter oligonucleotides, as described elsewhere herein. Mutants or alterations of the AbTx of interest can be provided, and operations 1110-1140 described above can be used to confirm that the new AbTx variant is not bound by the ADAs. The ADAs identified in operation 1140 can be further characterized in operation 1170. For instance, use of these antibodies in functional assays such as ADCC, ADCP, ADCD, and other assays can be used to identify ADAs with therapeutic value - such ADAs can be developed as drugs to be administered to subjects experiencing side effects induced by an AbTx or ADC by neutralizing the drug or promoting its destruction. Such ADAs can also be deployed as reference standards in traditional serum-based ADA assays. This can be especially important for AbTx that have been engineered to be recycled efficiently by addition of Fc domains that can be recognized by the neonatal Fc receptor (FcRn, product of the FCGRT gene). Molecules identified in operation 1140 more broadly can also be used to characterize the response in patients with diverse immunoglobulin haplotypes, and in combination with traditional statistical models such as contingency tests, GWAS, PheWAS, and cellular assays discover genomic and other elements associated with the development of ADAs.

### Systems and Methods for Spatial Analysis

Exemplary methods disclosed herein comprising contacting a biological sample, e.g., a tissue sample comprising one or more cells expressing an ABM with an antigen conjugated to a reporter oligonucleotide, wherein the reporter oligonucleotide comprises a reporter barcode sequence and the antigen is AbTx or ADC. The tissue sample may be a tissue section. The tissue section can be a fresh frozen tissue section, a fixed tissue section, or an FFPE tissue section. The tissue sample may be fixed and/or stained (e.g., a fixed and/or stained tissue section). Non-limiting examples of stains include histological stains (e.g., hematoxylin and/or eosin) and immunological stains (e.g., fluorescent stains). a biological sample (e.g., a fixed and/or stained biological sample) can be imaged. Tissue samples are also described in Section (I)(d) of WO 2020/176788 and/or U.S. Patent Application Publication No. 2020/0277663. the tissue sample may be subjected to spatial analysis. Systems and methods for spatial analysis are disclosed herein.

Non-limiting examples of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLOS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev D, dated October 2020), the Visium Spatial Gene Expression for FFPE User Guide (e.g., Rev A, dated June 2021), and/or the Visium Spatial Tissue Optimization Reagent Kits User Guide (e.g., Rev D, dated October 2020), both of which are available at the 10x Genomics Support Documentation website, and can be used herein in any combination. Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed..." of WO 2020/176788 and/or U.S. Patent Application Publication No. 2020/0277663. Further non-limiting examples of spatial analysis methodologies and compositions are described herein.

A biological sample may be permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods can involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. The capture probe may be a nucleic acid or a polypeptide. The capture probe may include a barcode (e.g., a spatial barcode and/or a unique molecular identifier (UMI)) and a capture domain). A capture probe can include a cleavage domain and/or a functional domain (e.g., a primerbinding site, such as for next-generation sequencing (NGS)).

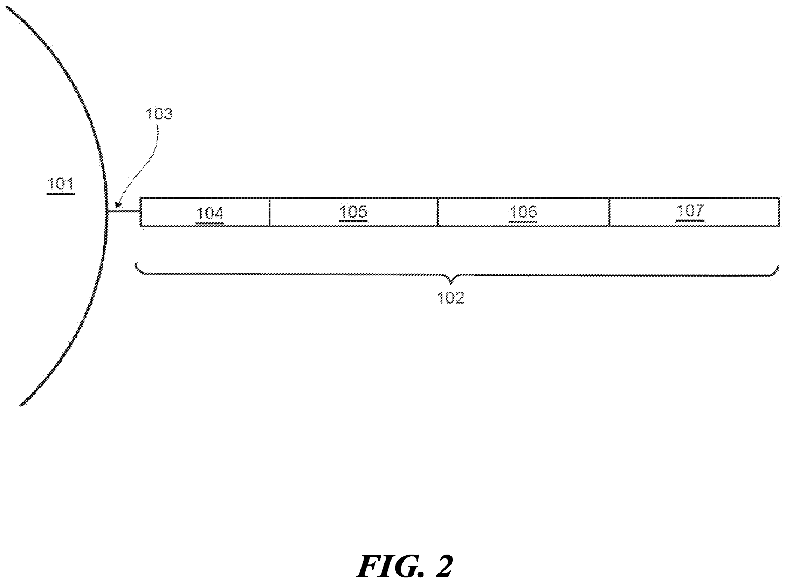

FIG. 2 is a schematic diagram showing an exemplary capture probe, as described herein. As shown, the capture probe 102 is optionally coupled to a feature 101 by a cleavage domain 103, such as a disulfide linker. The capture probe can include a functional sequence 104 (also referred to herein as "adapter" or "adaptor") that is useful for subsequent processing. The functional sequence 104 can include all or a part of sequencer specific flow cell attachment sequence (e.g., a P5 or P7 sequence), all or a part of a sequencing primer sequence, (e.g., a R1 primer binding site, a R2 primer binding site), or combinations thereof. The capture probe can also include a spatial barcode 105. The capture probe can also include a unique molecular identifier (UMI) sequence 106. While FIG. 2 shows the spatial barcode 105 as being located upstream (5') of UMI sequence 106, it is to be understood that capture probes wherein UMI sequence 106 is located upstream (5') of the spatial barcode 105 is also suitable for use in any of the methods described herein. The capture probe can also include a capture domain 107 to facilitate capture of a target analyte. The capture domain can have a sequence complementary to a sequence of a nucleic acid analyte. The capture domain can have a sequence complementary to a connected probe described herein. The capture domain can have a sequence complementary to a capture handle sequence present in an analyte capture agent. The capture domain can have a sequence complementary to a splint oligonucleotide. Such splint oligonucleotide, in addition to having a sequence complementary to a capture domain of a capture probe, can have a sequence of a nucleic acid analyte, a sequence complementary to a portion of a connected probe described herein, and/or a capture handle sequence described herein.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. Functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, functional sequences can be selected for compatibility with other sequencing systems, including non-commercialized sequencing systems.

The spatial barcode 105 and functional sequences 104 may be common to all of the probes attached to a given feature. The UMI sequence 106 of a capture probe attached to a given feature may be different from the UMI sequence of a different capture probe attached to the given feature.

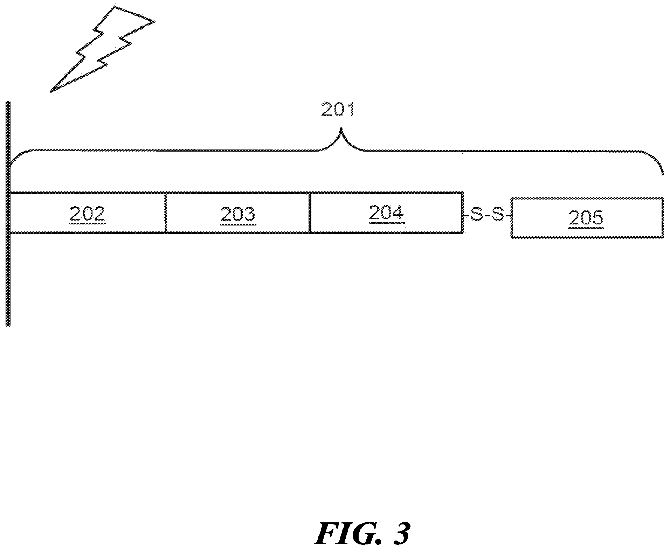

FIG. 3 is a schematic illustrating a cleavable capture probe, wherein the cleaved capture probe can enter into a non-permeabilized cell and bind to analytes within the sample. The capture probe 201 contains a cleavage domain 202, a cell penetrating peptide 203, a reporter molecule 204, and a disulfide bond (-S-S-). 205 represents all other parts of a capture probe, for example a spatial barcode and a capture domain.

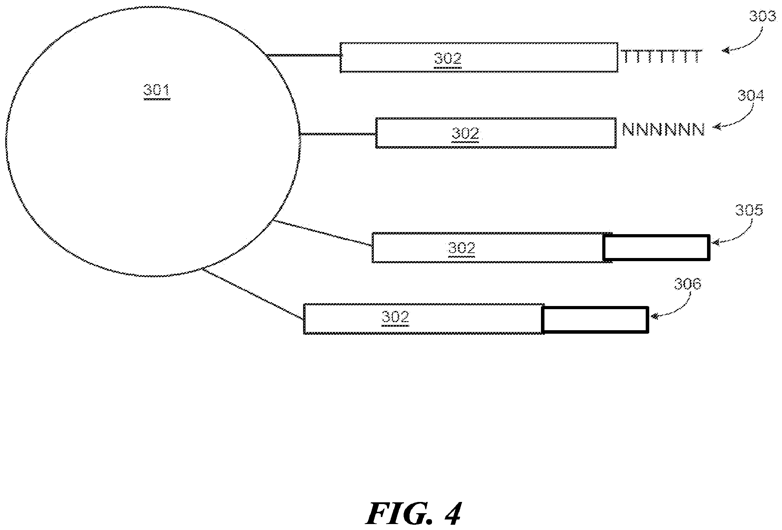

FIG. 4 is a schematic diagram of an exemplary multiplexed spatially-barcoded feature. In FIG. 4, the feature 301 can be coupled to spatially-barcoded capture probes, wherein the spatially-barcoded probes of a particular feature can possess the same spatial barcode, but have different capture domains designed to associate the spatial barcode of the feature with more than one target analyte. For example, a feature may be coupled to four different types of spatially-barcoded capture probes, each type of spatially-barcoded capture probe possessing the spatial barcode 302. One type of capture probe associated with the feature includes the spatial barcode 302 in combination with a poly(T) capture domain 303, designed to capture mRNA target analytes. A second type of capture probe associated with the feature includes the spatial barcode 302 in combination with a random N-mer capture domain 304 for gDNA analysis. A third type of capture probe associated with the feature includes the spatial barcode 302 in combination with a capture domain complementary to a capture handle sequence of an analyte capture agent of interest 305. A fourth type of capture probe associated with the feature includes the spatial barcode 302 in combination with a capture domain that can specifically bind a nucleic acid molecule 306 that can function in a CRISPR assay (e.g., CRISPR/Cas9).

A feature can be coupled to (i) a first capture probe comprising spatial barcode sequence 302 and a first capture domain comprising a first capture sequence. The feature can be coupled to (ii) a second capture probe comprising the same spatial barcode sequence 302 and a second capture domain comprising a second capture sequence. The first capture sequence of the first capture domain and the second capture sequence of the second capture domain may be identical. The first capture sequence of the first capture domain and the second capture sequence of the second capture domain may be different. The first capture sequence of the first capture domain may be a homopolymeric sequence. The first capture sequence of the first capture domain may be a defined non-homopolymeric sequence. The defined non-homopolymeric sequence may be a sequence that binds to the first analyte. The defined non-homopolymeric sequence may specifically binds to a nucleic acid sequence encoding a region of an ABM. Wherein the ABM is selected from: a TCR alpha chain, a TCR beta chain, a TCR gamma chain, a TCR delta chain an immunoglobulin kappa light chain, an immunoglobulin lambda light chain, an immunoglobulin heavy chain. the region of the ABM may be a constant region of the ABM or a variable region of the ABM. The second capture sequence of the second capture domain may be a homopolymeric sequence. The second capture sequence of the second capture domain may be a defined non-homopolymeric sequence. The homopolymeric sequence may be a polyT sequence.

While four different capture probe-barcoded constructs are shown in FIG. 4, captureprobe barcoded constructs can be tailored for analyses of any given analyte associated with a nucleic acid and capable of binding with such a construct. For example, the schemes shown in FIG. 4 can also be used for concurrent analysis of other analytes disclosed herein, including, but not limited to: (a) mRNA, a lineage tracing construct, cell surface or intracellular proteins and metabolites, and gDNA; (b) mRNA, accessible chromatin (e.g., ATAC-seq, DNase-seq, and/or MNase-seq) cell surface or intracellular proteins and metabolites, and a perturbation agent (e.g., a CRISPR crRNA/sgRNA, TALEN, zinc finger nuclease, and/or antisense oligonucleotide as described herein); (c) mRNA, cell surface or intracellular proteins and/or metabolites, a barcoded labelling agent (e.g., the MHC multimers described herein), and a V(D)J sequence of an immune cell receptor (e.g., T-cell receptor). A perturbation agent can be a small molecule, an antibody, a drug, an aptamer, a miRNA, a physical environmental (e.g., temperature change), or any other known perturbation agents. See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

More than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

Detection of one or more analytes (e.g., protein analytes) can be performed using one or more analyte capture agents. As used herein, an "analyte capture agent" (also referred to herein as "labeling agent") refers to an agent that interacts with an analyte (e.g., an analyte in a biological sample) and with a capture probe (e.g., a capture probe attached to a substrate or a feature) to identify the analyte. The analyte capture agent may include: (i) an analyte binding moiety (e.g., that binds to an analyte), for example, an antibody or antigen-binding fragment thereof; (ii) analyte binding moiety barcode; and (iii) a capture handle sequence. As used herein, the term "analyte binding moiety barcode" refers to a barcode that is associated with or otherwise identifies the analyte binding moiety. As used herein, the term "analyte capture sequence" or "capture handle sequence" refers to a region or moiety configured to hybridize to, bind to, couple to, or otherwise interact with a capture domain of a capture probe. A capture handle sequence may be complementary to a capture domain of a capture probe. In some cases, an analyte binding moiety barcode (or portion thereof) may be able to be removed (e.g., cleaved) from the analyte capture agent.

It should be noted that an "analyte capture agent" can refer to a reporter oligonucleotide conjugated antigen (e.g., wherein the antigen is an AbTx or ADC) and vice versa. Accordingly, the "analyte binding moiety" can also refer to an antigen, wherein the antigen is the AbTx or ADC.

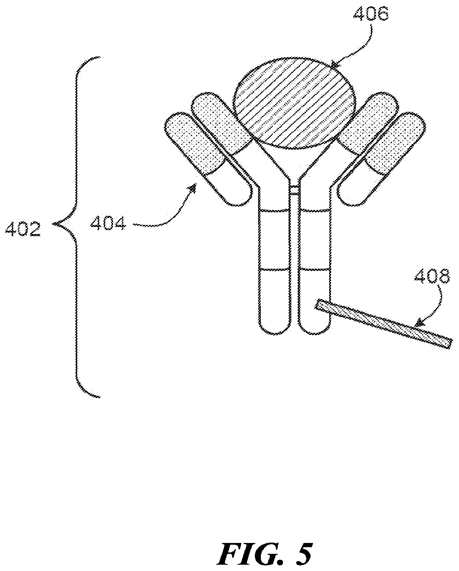

FIG. 5 is a schematic diagram of an exemplary analyte capture agent 402 comprised of an analyte-binding moiety 404 and an analyte-binding moiety barcode domain 408. The exemplary analyte -binding moiety 404 is a molecule capable of binding to an analyte 406 and the analyte capture agent is capable of interacting with a spatially-barcoded capture probe. The analyte -binding moiety can bind to the analyte 406 with high affinity and/or with high specificity. The analyte capture agent can include an analyte -binding moiety barcode domain 408, a nucleotide sequence (e.g., an oligonucleotide), which can hybridize to at least a portion or an entirety of a capture domain of a capture probe. It should be noted that a reporter oligonucleotide, e.g., of a reporter oligonucleotide conjugated antigen, can be considered an analyte-binding moiety barcode domain 408. The analyte-binding moiety barcode domain 408 (also referred to herein as a reporter oligonucleotide) can comprise an analyte binding moiety barcode (also referred to herein as a "reporter barcode sequence") and a capture handle sequence described herein. The analyte-binding moiety 404 can include a polypeptide and/or an aptamer. The analyte-binding moiety 404 can include an antibody or antibody fragment (e.g., an antigen-binding fragment). The analyte-binding moiety 404 can include an antibody therapeutic (or antigen binding fragment thereof) and/or antibody drug conjugate.

The analyte capture agent may include a capture agent barcode domain that is conjugated or otherwise attached to the analyte binding moiety. The capture agent barcode domain may be covalently-linked to the analyte binding moiety. A capture agent barcode domain may be a nucleic acid sequence. A capture agent barcode domain may include an analyte binding moiety barcode and an analyte capture sequence.

As used herein, the term "analyte binding moiety barcode" refers to a barcode that is associated with or otherwise identifies the analyte binding moiety. By identifying an analyte binding moiety and its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of examples of barcodes described herein. For example, an analyte capture agent that is specific to one type of analyte can have coupled thereto a first capture agent barcode domain (e.g., that includes a first analyte binding moiety barcode), while an analyte capture agent that is specific to a different analyte can have a different capture agent barcode domain (e.g., that includes a second barcode analyte binding moiety barcode) coupled thereto. Such a capture agent barcode domain can include an analyte binding moiety barcode that permits identification of the analyte binding moiety to which the capture agent barcode domain is coupled. The selection of the capture agent barcode domain can allow significant diversity in terms of sequence, while also being readily attachable to most analyte binding moieties (e.g., antibodies or aptamers) as well as being readily detected, (e.g., using sequencing or array technologies).

The capture agent barcode domain of an analyte capture agent may include an analyte capture sequence. As used herein, the term "analyte capture sequence" and "capture handle sequence" may be used interchangeably herein to refer to a region or moiety configured to hybridize to, bind to, couple to, or otherwise interact with a capture domain of a capture probe. A capture handle sequence may include a nucleic acid sequence that is complementary to or substantially complementary to a capture sequence of the capture domain of a capture probe such that the capture handle sequence hybridizes to the capture domain of the capture probe. A capture handle sequence may comprise a poly(A) nucleic acid sequence that hybridizes to a capture domain that comprises a poly(T) nucleic acid sequence. A capture handle sequence may comprise a poly(T) nucleic acid sequence that hybridizes to a capture domain that comprises a poly(A) nucleic acid sequence. A capture handle sequence may comprise a non-homopolymeric nucleic acid sequence that hybridizes to a capture domain that comprises a non-homopolymeric nucleic acid sequence that is complementary (or substantially complementary) to the non-homopolymeric nucleic acid sequence of the analyte capture region.

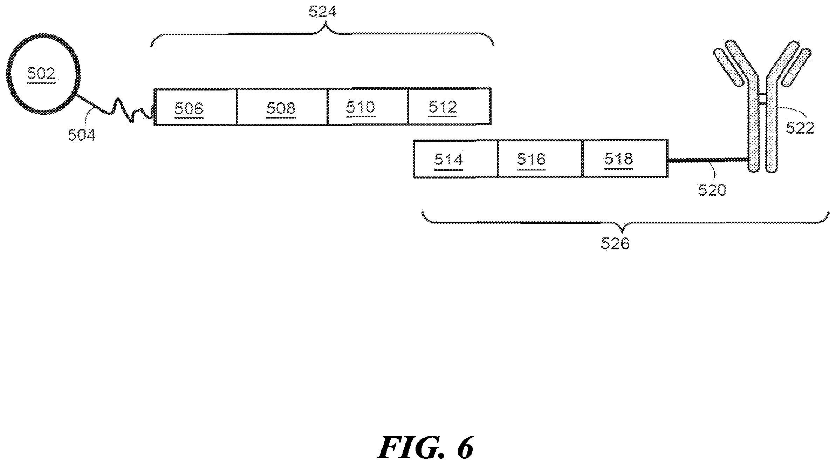

FIG. 6 is a schematic diagram depicting an exemplary interaction between a feature-immobilized capture probe 524 and an analyte capture agent 526 (also referred to herein as a labeling agent, e.g., a reporter oligonucleotide conjugated antigen). The feature-immobilized capture probe 524 can include a spatial barcode 508 as well as functional sequences 506 and UMI 510, as described elsewhere herein. The capture probe can also include a capture domain 512 that is capable of binding to an analyte capture agent 526. The analyte capture agent 526 can include a functional sequence 518, analyte binding moiety barcode 516 (also referred to herein as a reporter barcode sequence), and a capture handle sequence 514 that is capable of binding to the capture domain 512 of the capture probe 524. For example, the capture handle sequence 514 may be complementary to a capture sequence of the capture domain 512. The analyte capture agent can also include a linker 520 that allows the capture agent barcode domain 516 to couple to the analyte binding moiety 522.

Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) from a cell towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a connected probe (e.g., a ligation product) or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form a connected probe (e.g., a ligation product) with a template (e.g., a DNA or RNA template, such as an analyte or an intermediate agent, or portion thereof), thereby creating ligations products that serve as proxies for a template. As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). Extending the capture probe may include adding to a 3' end of a capture probe a nucleic acid sequence that is complementary to a nucleic acid sequence of an analyte or intermediate agent specifically bound to the capture domain of the capture probe. The capture probe may be extended using reverse transcription. The capture probe may be extended using one or more DNA polymerases. The extended capture probes include the sequence of the capture probe and the sequence of the spatial barcode of the capture probe. Extended capture probes may be amplified (e.g., in bulk solution or on the array) to yield quantities that are sufficient for downstream analysis, e.g., for determining sequences of one or more analytes, e.g., via sequencing. Extended capture probes (e.g., DNA molecules) may act as templates for an amplification reaction (e.g., a polymerase chain reaction). Additional variants of spatial analysis methods, including an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

For spatial array-based methods, a substrate may function as a support for direct or indirect attachment of capture probes to features of the array. A "feature" is an entity that acts as a support or repository for various molecular entities used in spatial analysis. Some or all of the features in an array may be functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample (e.g., a tissue sample) with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., a tissue sample). A plurality of molecules (e.g., a plurality of nucleic acid molecules) having a plurality of barcodes (e.g., a plurality of spatial barcodes) may be introduced to a biological sample (e.g., to a plurality of cells in a biological sample) for use in spatial analysis. After attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. For example, spatial analysis can be performed using RNA-templated ligation (RTL). Methods of RTL have been described previously. See, e.g., Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). The oligonucleotides may be DNA molecules. One of the oligonucleotides may include at least two ribonucleic acid bases at the 3' end and/or the other oligonucleotide includes a phosphorylated nucleotide at the 5' end. One of the two oligonucleotides may include a capture domain (e.g., a poly(A) sequence, a non-homopolymeric sequence). After hybridization to the analyte, a ligase (e.g., SplintR ligase) ligates the two oligonucleotides together, creating a connected probe (e.g., a ligation product). The two oligonucleotides hybridize to sequences that may not be adjacent to one another. For example, hybridization of the two oligonucleotides creates a gap between the hybridized oligonucleotides. A polymerase (e.g., a DNA polymerase) can extend one of the oligonucleotides prior to ligation. After ligation, the connected probe (e.g., a ligation product) is released from the analyte. The connected probe (e.g., a ligation product) may be released using an endonuclease. The endonuclease may be an RNAse. The endonuclease may be one of RNase A, RNase C, RNase H, and RNase I. The endonuclease may be RNAse H. The RNase H may be RNase H1 or RNase H2. The released connected probe (e.g., a ligation product) can then be captured by capture probes (e.g., instead of direct capture of an analyte) on an array, optionally amplified, and sequenced, thus determining the location and optionally the abundance of the analyte in the biological sample. Accordingly, the released connected probe can be considered an "analyte".

During analysis of spatial information, sequence information for a spatial barcode associated with an analyte is obtained, and the sequence information can be used to provide information about the spatial distribution of the analyte in the biological sample. Various methods can be used to obtain the spatial information. Specific capture probes and the analytes they capture may be associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in PCT Application No. 2020/061064 and/or U.S. Patent Application Serial No. 16/951,854.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2020/061108 and/or U.S. Patent Application Serial No. 16/951,864.In some cases, a map of analyte presence and/or level can be aligned to an image of a biological sample using one or more fiducial markers, e.g., objects placed in the field of view of an imaging system which appear in the image produced, as described in the Substrate Attributes Section, Control Slide for Imaging Section of WO 2020/123320, PCT Application No. 2020/061066, and/or U.S. Patent Application Serial No. 16/951,843. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### Sandwiching processes

As described herein, following the contacting of the tissue sample with a reporter oligonucleotide conjugated antigen (e.g., wherein the antigen is an AbTx or ADC), one or more analytes from the tissue sample may be released from the tissue sample and migrate to a substrate comprising an array of capture probes for attachment to the capture probes of the array.

The tissue sample may be mounted on a first substrate during the contacting step, and the substrate comprising the array of capture probes is a second substrate. Following the contacting step, the method can include a "sandwiching process" using a device, sample holder, sample handling apparatus, or system described in, e.g., PCT/US2019/065100, PCT/US2021/036788, or PCT/US2021/050931 for the release and migration of the analytes to the array of capture probes in a manner that preserves their spatial context.

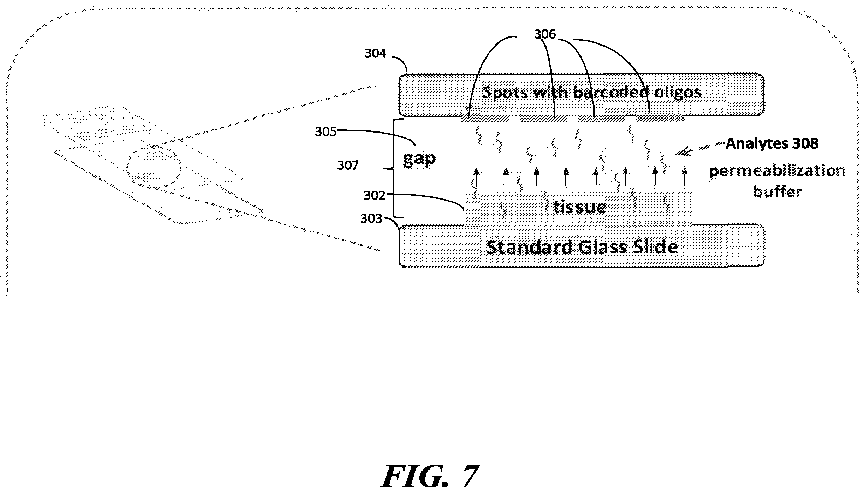

FIG. 7 is a schematic diagram depicting an exemplary sandwiching process 104 between a first substrate comprising a biological sample (e.g., a tissue section 302 on a slide 303) and a second substrate comprising a spatially barcoded array, e.g., a slide 304 that is populated with spatially-barcoded capture probes 306. During the exemplary sandwiching process, the first substrate is aligned with the second substrate, such that at least a portion of the biological sample is aligned with at least a portion of the array (e.g., aligned in a sandwich configuration). As shown, the slide 304 is in a superior position to the pathology slide 303. The pathology slide 303 may be positioned superior to the slide 304. A permeabilization solution 305 within a gap 307 between the pathology slide 303 and the slide 304 creates a permeabilization buffer which permeabilizes or digests the sample 302 and the analytes (e.g., mRNA transcripts) 308 of the tissue sample 302 may release, actively or passively migrate (e.g., diffuse) across the gap 307 toward the capture probes 306, and bind on the capture probes 306.

After the analytes (e.g., transcripts) 308 bind on the capture probes 306, an extension reaction may occur, thereby generating a spatially barcoded library. For example, in the case of mRNA transcripts, reverse transcription may be used to generate a cDNA library associated with a particular spatial barcode. Barcoded cDNA libraries may be mapped back to a specific spot on a capture area of the capture probes 306. This gene expression data may be subsequently layered over a high-resolution microscope image of the tissue section, making it possible to visualize the expression of any mRNA, or combination of mRNAs, within the morphology of the tissue in a spatially-resolved manner. The extension reaction can be performed separately from the sample handling apparatus described herein that is configured to perform the exemplary sandwiching process 104.The sandwich configuration of the sample 302, the pathology slide 303 and the slide 304 may provide advantages over other methods of spatial analysis and/or analyte capture. For example, the sandwich configuration may reduce a burden of users to develop in house tissue sectioning and/or tissue mounting expertise. Further, the sandwich configuration may decouple sample preparation/tissue imaging from the barcoded array (e.g., spatially-barcoded capture probes 306) and enable selection of a particular region of interest of analysis (e.g., for a tissue section larger than the barcoded array). The sandwich configuration also beneficially enables spatial analysis without having to place a tissue section 302 directly on the array slide (e.g., slide 304).

The sandwiching process may comprise: mounting the first substrate on a first member of a support device, the first member configured to retain the first substrate; mounting the second substrate on a second member of the support device, the second member configured to retain the second substrate, applying a reagent medium to the first substrate and/or the second substrate, the reagent medium comprising a permeabilization agent, operating an alignment mechanism of the support device to move the first member and/or the second member such that a portion of the tissue sample comprising the ABM-expressing cell is aligned (e.g., vertically aligned) with a portion of the array of capture probes and within a threshold distance of the array of capture probes, and such that the portion of the tissue sample and the capture probe contact the reagent medium, wherein the permeabilization agent releases the first analyte from the ABM-expressing cell.

The sandwiching process methods described above can be implemented using a variety of hardware components.

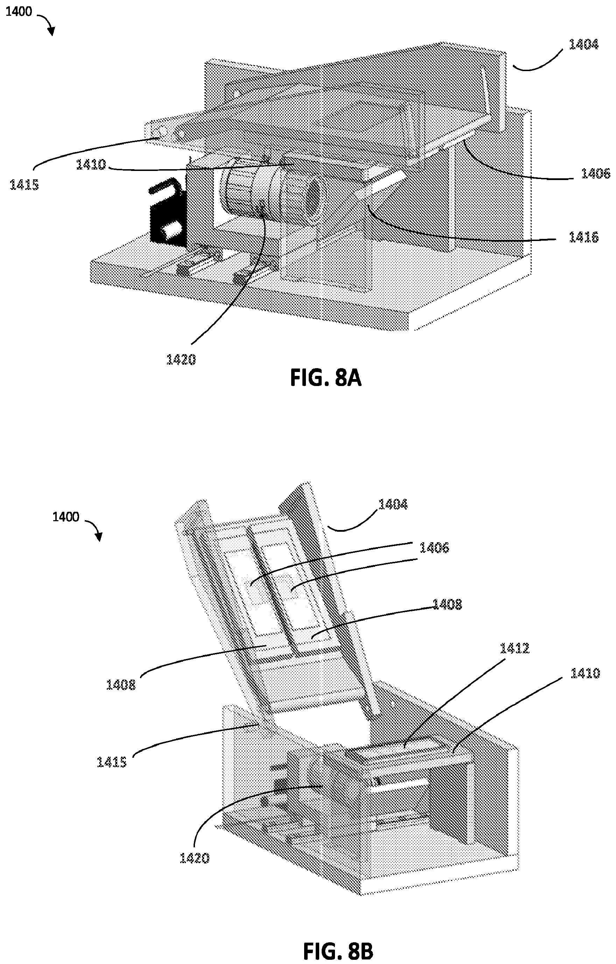

FIG. 8A is a perspective view of an example sample handling apparatus 1400 in a closed position in accordance with some example implementations. As shown, the sample handling apparatus 1400 includes a first member 1404, a second member 1410, an image capture device 1420, a first substrate 1406, a hinge 1415, and a mirror 1416. The hinge 1415 may be configured to allow the first member 1404 to be positioned in an open or closed configuration by opening and/or closing the first member 1404 in a clamshell manner along the hinge 1415.

FIG. 8B is a perspective view of the example sample handling apparatus 1400 in an open position in accordance with some example implementations. As shown, the sample handling apparatus 1400 includes one or more first retaining mechanisms 1408 configured to retain one or more first substrates 1406. In the example of FIG. 8B, the first member 1404 is configured to retain two first substrates 1406, however the first member 1404 may be configured to retain more or fewer first substrates 1406.

When the sample handling apparatus 1400 is in an open position (as in FIG. 8B), the first substrate 1406 and/or the second substrate 1412 may be loaded and positioned within the sample handling apparatus 1400 such as within the first member 1404 and the second member 1410, respectively. As noted, the hinge 1415 may allow the first member 1404 to close over the second member 1410 and form a sandwich configuration (e.g., the sandwich configuration shown in FIG. 7).

After the first member 1404 closes over the second member 1410, an adjustment mechanism (not shown) of the sample handling apparatus 1400 may actuate the first member 1404 and/or the second member 1410 to form the sandwich configuration for the permeabilization step (e.g., bringing the first substrate 1406 and the second substrate 1412 closer to each other and within a threshold distance for the sandwich configuration). The adjustment mechanism may be configured to control a speed, an angle, or the like of the sandwich configuration.

The tissue sample (e.g., sample 302) may be aligned within the first member 1404 (e.g., via the first retaining mechanism 1408) prior to closing the first member 1404 such that a desired region of interest of the sample 302 is aligned with the bar-coded array of the spatially barcoded array slide (e.g., the slide 304) , e.g., when the first and second substrates are aligned in the sandwich configuration. Such alignment may be accomplished manually (e.g., by a user) or automatically (e.g., via an automated alignment mechanism). After or before alignment, spacers may be applied to the first substrate 1406 and/or the second substrate 1412 to maintain a minimum spacing between the first substrate 1406 and the second substrate 1412 during sandwiching. The permeabilization solution (e.g., permeabilization solution 305) may be applied to the first substrate 1406 and/or the second substrate 1412. The first member 1404 may then close over the second member 1410 and form the sandwich configuration. Analytes (e.g., mRNA transcripts) 308 may be captured by the capture probes 306 and may be processed for spatial analysis.

During the permeabilization step, the image capture device 1420 may capture images of the overlap area (e.g., overlap area 710) between the tissue 302 and the capture probes 306. If more than one first substrates 1406 and/or second substrates 1412 are present within the sample handling apparatus 1400, the image capture device 1420 may be configured to capture one or more images of one or more overlap areas 710. Further details on support devices, sample holders, sample handling apparatuses, or systems for implementing a sandwiching process are described in, e.g., PCT/US2019/065100 and PCT/US2021/050931.

Analytes within a biological sample are generally released through disruption (e.g., permeabilization, digestion, etc.) of the biological sample or may be released without disruption. Various methods of permeabilizing (e.g., any of the permeabilization reagents and/or conditions described herein) a biological sample are described herein, including for example including the use of various detergents, buffers, proteases, and/or nucleases for different periods of time and at various temperatures. Additionally, various methods of delivering fluids (e.g., a buffer, a permeabilization solution) to a biological sample are described herein including the use of a substrate holder (e.g., sandwich assembly, sandwich configuration, as described herein)

Described herein are methods for delivering a fluid to a biological sample disposed on an area of a first substrate and an array disposed on a second substrate.

With reference to FIG. 7, the sandwich configuration described herein between a tissue sample slide (e.g., pathology slide 303) and a spatially barcoded array slide (e.g., slide 304 with barcoded capture probes 306) may require the addition of a liquid reagent (e.g., permeabilization solution 305 or other target molecule release and capture solution) to fill a gap (e.g., gap 307). It may be desirable that the liquid reagent be free from air bubbles between the slides to facilitate transfer of target molecules with spatial information. Additionally, air bubbles present between the slides may obscure at least a portion of an image capture of a desired region of interest. Accordingly, it may be desirable to ensure or encourage suppression and/or elimination of air bubbles between the two slides during a permeabilization step (e.g., step 104).

It may be possible to reduce or eliminate bubble formation between the slides using a variety of filling methods and/or closing methods.

Workflows described herein may include contacting a drop of the liquid reagent disposed on a first substrate or a second substrate with at least a portion of a first substrate or second substrate, respectively. The contacting may comprise bringing the two substrates into proximity such that the sample on the first substrate is aligned with the barcode array of capture probes on the second substrate.

The drop may include permeabilization reagents (e.g., any of the permeabilization reagents described herein). The rate of permeabilization of the biological sample may be modulated by delivering the permeabilization reagents (e.g., a fluid containing permeabilization reagents) at various temperatures.

In the example sandwich maker workflows described herein, a liquid reagent (e.g., the permeabilization solution 305) may fill a gap (e.g., the gap 307) between a tissue slide (e.g., slide 303) and a capture slide (e.g., slide 304 with barcoded capture probes 306) to warrant or enable transfer of target molecules with spatial information. Described herein are examples of filling methods that may suppress bubble formation and suppress undesirable flow of transcripts and/or target molecules or analytes. Robust fluidics in the sandwich making described herein may preserve spatial information by reducing or preventing deflection of molecules as they move from the tissue slide to the capture slide.

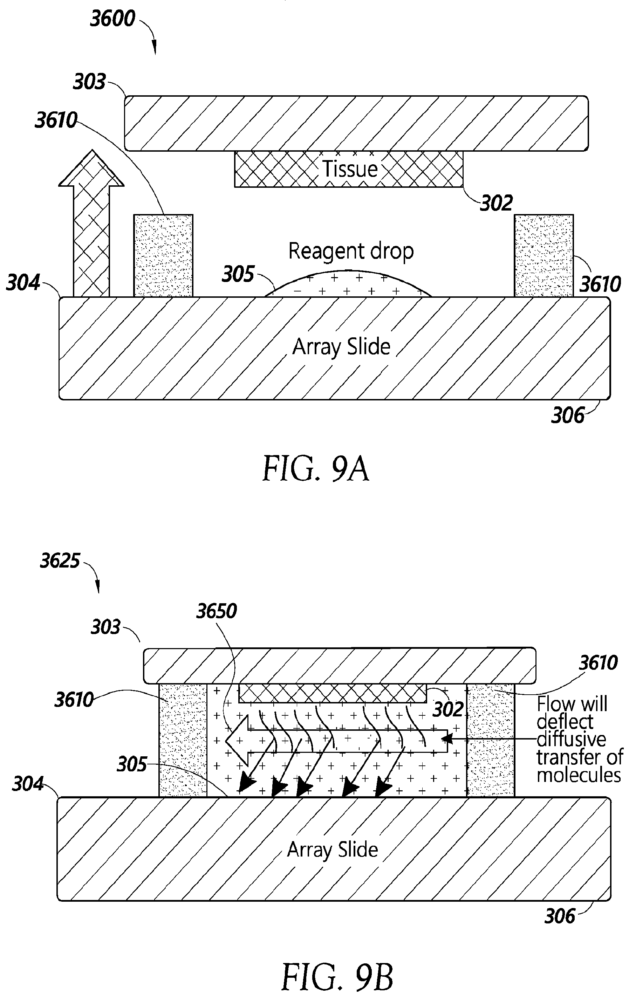

FIG. 9A shows an exemplary sandwiching process 3600 where a first substrate (e.g., pathology slide 303), including a biological sample 302 (e.g., a tissue section), and a second substrate (e.g., slide 304 including spatially barcoded capture probes 306) are brought into proximity with one another. As shown in FIG. 9A a liquid reagent drop (e.g., permeabilization solution 305) is introduced on the second substrate in proximity to the capture probes 306 and in between the biological sample 302 and the second substrate (e.g., slide 304). The permeabilization solution 305 may release analytes that can be captured by the capture probes 306 of the array. As further shown, one or more spacers 3610 may be positioned between the first substrate (e.g., pathology slide 303) and the second substrate (e.g., slide 304). The one or more spacers 3610 may be configured to maintain a separation distance between the first substrate and the second substrate. While the one or more spacers 3610 is shown as disposed on the second substrate, the spacer may additionally or alternatively be disposed on the first substrate.

The one or more spacers 3610 may be configured to maintain a separation distance between first and second substrates that is between about 2 microns and 1 mm (e.g., between about 2 microns and 800 microns, between about 2 microns and 700 microns, between about 2 microns and 600 microns, between about 2 microns and 500 microns, between about 2 microns and 400 microns, between about 2 microns and 300 microns, between about 2 microns and 200 microns, between about 2 microns and 100 microns, between about 2 microns and 25 microns, or between about 2 microns and 10 microns), measured in a direction orthogonal to the surface of first substrate that supports the sample. The separation distance may be about 2, 3, 4, 5, 6, 7, 8, 9, 10, 11, 12, 13, 14, 15, 16, 17, 18, 19, 20, 21, 22, 23, 24, or 25 microns. The separation distance may be less than 50 microns. The separation distance may be less than 25 microns. The separation distance may be less than 20 microns. The separation distance may include a distance of at least 2 µm.

FIG. 9B shows a fully formed sandwich configuration creating a chamber 3650 formed from the one or more spacers 3610, the first substrate (e.g., the pathology slide 303), and the second substrate (e.g., the slide 304) in accordance with some example implementations. In the example of FIG. 9B, the liquid reagent (e.g., the permeabilization solution 305) fills the volume of the chamber 3650 and may create a permeabilization buffer that allows analytes (e.g., mRNA transcripts and/or other molecules) to diffuse from the biological sample 302 toward the capture probes 306 of the slide 304. Any flow of the permeabilization buffer may deflect transcripts and/or molecules from the biological sample 302 and may affect diffusive transfer of analytes for spatial analysis. A partially or fully sealed chamber 3650 resulting from the one or more spacers 3610, the first substrate, and the second substrate may reduce or prevent flow from undesirable convective movement of transcripts and/or molecules over the diffusive transfer from the biological sample 302 to the capture probes 306.

The first substrate and the second substrate may be arranged in an angled sandwich assembly as described herein. For example, during the sandwiching of the two slides (e.g., the pathology slide 303 and the slide 304) it may be possible to provide an angled closure of the slides to suppress or eliminate bubble formation.

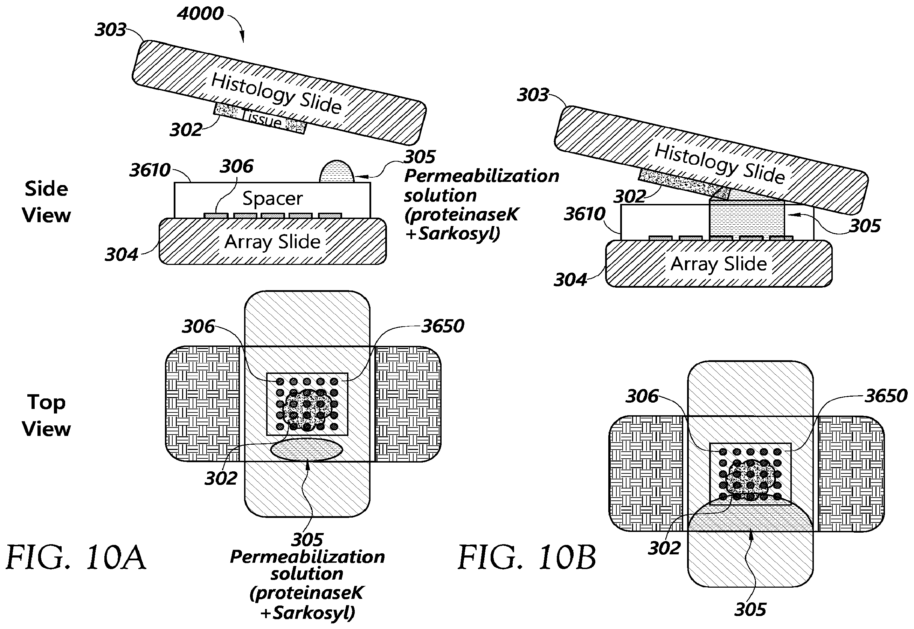

FIGS. 10A-10C depict a side view and a top view of an angled closure workflow 4000 for sandwiching a first substrate (e.g., pathology slide 303) having a tissue sample 302 and a second substrate (e.g., slide 304 having capture probes 306) in accordance with some example implementations.

FIG. 10A depicts the first substrate (e.g., the pathology slide 303 including sample 302) angled over (superior to) the second substrate (e.g., slide 304). As shown, a drop of the permeabilization solution 305 is located on top of the spacer 3610 toward the right-hand side of the side view in FIG. 10A.

FIG. 10B shows that as the first substrate lowers, or as the second substrate rises, the dropped side of the first substrate (e.g., a side of the slide 303 angled inferior to the opposite side) may contact the drop of the permeabilization solution 305. The dropped side of the first substrate may urge the permeabilization solution 305 toward the opposite direction. For example, in the side view of FIG. 10B the permeabilization solution 305 may be urged from right to left as the sandwich is formed.

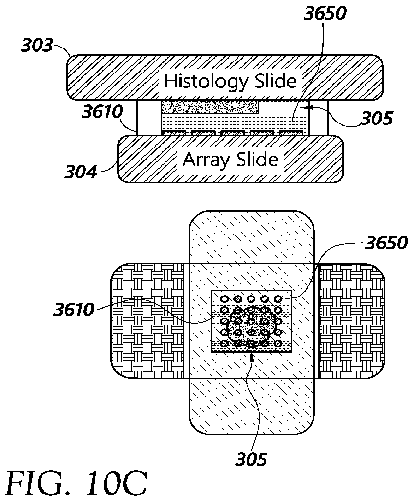

FIG. 10C depicts a full closure of the sandwich between the first substrate and the second substrate with the spacer 3610 contacting both the first substrate and the second substrate and maintaining a separation distance between the two. As shown in the top view of FIG. 10C, the spacer 3610 fully encloses and surrounds the tissue sample 302 and the capture probes 306, and the spacer 3610 forms the sides of chamber 2650 which holds a volume of the permeabilization solution 305.

The alignment of the tissue sample 302 with the capture probes 306 shown in FIGs. 10A-10C may be performed by an alignment mechanism of a sample handling apparatus (e.g., as described in PCT/US2021/050931).

Further details on angled closure workflows, and devices and systems for implementing an angled closure workflow, are described in PCT/US2021/036788 and PCT/US2021/050931.

Additional configurations for reducing or eliminating bubble formation, and/or for reducing unwanted fluid flow, are described in PCT/US2021/036788.

### Exemplary Spatial Methodologies for immune profiling

As described herein, the methods may be used to identify immune cell profiles. Immune cells express various adaptive immunological receptors relating to immune function, such as T cell receptors (TCRs) and B cell receptors (BCRs). T cell receptors and B cell receptors play a part in the immune response by specifically recognizing and binding to antigens and aiding in their destruction.

The T cell receptor, or TCR, is a molecule found on the surface of T cells that is generally responsible for recognizing fragments of antigen as peptides bound to major histocompatibility complex (MHC) molecules. The TCR is generally a heterodimer of two chains, each of which is a member of the immunoglobulin superfamily, possessing an N-terminal variable (V) domain, and a C terminal constant domain. In humans, in 95% of T cells, the TCR consists of an alpha (α) and beta (β) chain, whereas in 5% of T cells, the TCR consists of gamma and delta (γ/δ) chains. This ratio can change during ontogeny and in diseased states as well as in different species. When the TCR engages with antigenic peptide and MHC (peptide/MHC or pMHC), the T lymphocyte is activated through signal transduction.

Each of the two chains of a TCR contains multiple copies of gene segments - a variable 'V' gene segment, a diversity 'D' gene segment, and a joining 'J' gene segment. The TCR alpha chain (TCRa) is generated by recombination of V and J segments, while the beta chain (TCRb) is generated by recombination of V, D, and J segments. Similarly, generation of the TCR gamma chain involves recombination of V and J gene segments, while generation of the TCR delta chain occurs by recombination of V, D, and J gene segments. The intersection of these specific regions (V and J for the alpha or gamma chain, or V, D and J for the beta or delta chain) corresponds to the CDR3 region that is important for antigen-MHC recognition. Complementarity determining regions (e.g., CDR1, CDR2, and CDR3), or hypervariable regions, are sequences in the variable domains of antigen receptors (e.g., T cell receptor and immunoglobulin) that can complement an antigen. Most of the diversity of CDRs is found in CDR3, with the diversity being generated by somatic recombination events during the development of T lymphocytes. A unique nucleotide sequence that arises during the gene arrangement process can be referred to as a clonotype.

The B cell receptor, or BCR, is a molecule found on the surface of B cells. The antigen binding portion of a BCR is composed of a membrane-bound antibody that, like most antibodies (e.g., immunoglobulins), has a unique and randomly determined antigen-binding site. The antigen binding portion of a BCR includes membrane-bound immunoglobulin molecule of one isotype (e.g., IgD, IgM, IgA, IgG, or IgE). When a B cell is activated by its first encounter with a cognate antigen, the cell proliferates and differentiates to generate a population of antibody-secreting plasma B cells and memory B cells. The various immunoglobulin isotypes differ in their biological features, structure, target specificity, and distribution.

Where immune cells are to be analyzed, primer sequences useful in any of the various operations for attaching barcode sequences and/or amplification reactions can include gene specific sequences which target genes or regions of genes of immune cell proteins, for example antigen binding molecules, such as immune receptors. Such gene sequences include, but are not limited to, sequences of various T cell receptor alpha variable genes (TRAV genes), T cell receptor alpha joining genes (TRAJ genes), T cell receptor alpha constant genes (TRAC genes), T cell receptor beta variable genes (TRBV genes), T cell receptor beta diversity genes (TRBD genes), T cell receptor beta joining genes (TRBJ genes), T cell receptor beta constant genes (TRBC genes), T cell receptor gamma variable genes (TRGV genes), T cell receptor gamma joining genes (TRGJ genes), T cell receptor gamma constant genes (TRGC genes), T cell receptor delta variable genes (TRDV genes), T cell receptor delta diversity genes (TRDD genes), T cell receptor delta joining genes (TRDJ genes), and T cell receptor delta constant genes (TRDC genes).

A fundamental understanding of spatial heterogeneity with respect to T-cell receptor (TCR) and B-cell receptor (BCR) clonotypes within a biological sample is needed to understand multiple facets of their functionality, including, for example, which cells a particular TCR or BCR may be interacting with within the biological sample, the identity of TCR and/or BCR clonotypes in a given biological sample, and/or the identity of TCR and/or BCR clonotypes that are autoreactive in different autoimmune disorders. Numerous single-cell sequencing approaches can identify TCR and BCR clonotypes from a biological sample, however, at present methods are needed to link TCR and BCR sequences to spatial locations within a biological sample. Additionally, identifying the clonal regions, that is, regions defined by the places where variable (V), diverse (D), and joining (J) segments join to form the complementarity determining regions, including CDR1, CDR2, and CDR3, which provide specificity to the TCRs and/or BCRs, would greatly benefit the scientific arts. By coupling clonal information to spatial information it is possible to understand which T-cell and B-cell clonotypes may be specifically interacting with given cell types within a biological sample.

However, capturing analytes encoding immune cell receptors can provide unique challenges. For example, spatially capturing the TCR and BCR gene components with sufficient efficiency to profile the majority of clonotypes in a given tissue is difficult. Capturing analytes encoding immune cell receptors with conventional short-read sequencing methods can result in a loss of sequenced regions that are more than about 1 kb away from the point where sequencing starts. Linking separate TCR or BCR gene components that together form a complete receptor using sequencing data from spots containing multiple different cells are challenges addressed by the methods described herein.

The method may comprise: attaching a first analyte of an ABM-expressing cell of a tissue sample to a capture domain of a first capture probe of an array of capture probes attached to a substrate, the first capture probe comprising (i) a spatial barcode sequence and (ii) the capture domain, the capture domain comprising a first capture sequence, wherein the first analyte of the ABM-expressing cell comprises a sequence or portion of a sequence encoding the ABM expressed by the ABM-expressing cell or a reverse complement thereof; and using the first analyte of the ABM-expressing cell and the first capture probe attached thereto to generate a first spatially barcoded polynucleotide comprising (i) a sequence of the first analyte of the ABM-expressing cell or reverse complement thereof and (ii) the spatial barcode sequence or reverse complement thereof.

As described herein, analytes or reporter oligonucleotides can be attached to a capture domain of a capture probe via hybridization, or by ligation, e.g., splint-mediated ligation.

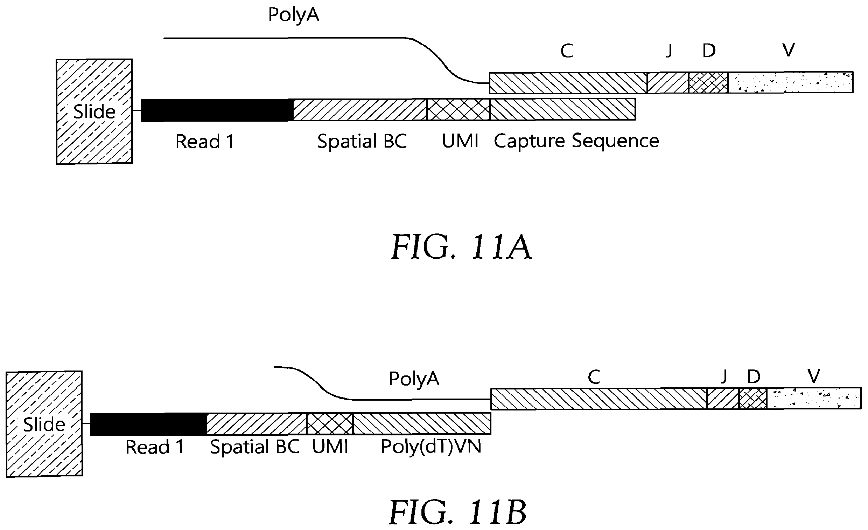

The first capture sequence binds may specifically to a nucleic acid sequence encoding a region of an ABM. An exemplary capture probe with a capture sequence that specifically binds to a nucleic acid sequence encoding a constant region of an ABM is depicted in FIG. 11A. The ABM may be selected from: a TCR alpha chain, a TCR beta chain, a TCR gamma chain, a TCR delta chain an immunoglobulin kappa light chain, an immunoglobulin lambda light chain, an immunoglobulin heavy chain. The first capture sequence may bind specifically to a nucleic acid sequence encoding a constant region of the T cell receptor alpha chain. The first capture sequence may bind specifically to a nucleic acid sequence encoding a constant region of the T cell receptor beta chain. The first capture sequence may bind specifically to a nucleic acid sequence encoding a constant region of the T cell receptor delta chain. The first capture sequence may bind specifically to a nucleic acid sequence encoding a constant region of the T cell receptor gamma chain. The first capture sequence may bind specifically to a nucleic acid sequence encoding a constant region of the immunoglobulin kappa light chain. The first capture sequence may bind specifically to a nucleic acid sequence encoding a constant region of the immunoglobulin lambda light chain. The first capture sequence may bind specifically to a nucleic acid sequence encoding a constant region of the immunoglobulin heavy chain.

The first capture sequence may be a homopolymeric sequence, e.g., a polyT sequence. FIG. 11B shows an exemplary poly(A) capture with a poly(T) capture domain. A poly(T) capture domain can capture other analytes, including analytes encoding ABMs within the tissue sample.

Following capture of analytes by capture probes, capture probes can be extended, e.g., via reverse transcription. Second strand synthesis can generate double stranded cDNA products that are spatially barcoded. The double stranded cDNA products, which may comprise ABM encoding sequences and non-ABM related analytes, can be enriched for ABM encoding sequences.

An exemplary enrichment workflow may comprise amplifying the cDNA products (or amplicons thereof) with a first primer that specifically binds to a functional sequence of the first capture probe or reverse complement thereof and a second primer that binds to a nucleic acid sequence encoding a variable region of the ABM expressed by the ABM-expressing cell or reverse complement thereof. The first primer and the second primer may flank the spatial barcode of the first spatially barcoded polynucleotide or amplicon thereof. The first primer and the second primer may flank a J junction, a D junction, and/or a V junction.

FIG. 12 shows an exemplary analyte enrichment strategy following analyte capture on the array. The portion of the immune cell analyte of interest includes the sequence of the V(D)J region, including CDR sequences. As described herein, a poly(T) capture probe captures an analyte encoding an ABM, an extended capture probe is generated by a reverse transcription reaction, and a second strand is generated. The resulting nucleic acid library can be enriched by the exemplary scheme shown in FIG. 12, where an amplification reaction including a Read 1 primer complementary to the Read 1 sequence of the capture probe and a primer complementary to a portion of the variable region of the immune cell analyte, can enrich the library via PCR. While FIG. 12 depicts a Read 1 primer, it is understood that a primer complementary to other functional sequences, such as other sequencing primer sequences, or sequencer specific flow cell attachment sequences, or portions of such functional sequences, may also be used. While FIG. 12 depicts a polyT capture sequence, it is understood that other capture sequences disclosed herein may be present in library members. The enriched library can be further enriched by nested primers complementary to a portion of the variable region internal (e.g., 5') to the initial variable region primer for practicing nested PCR.

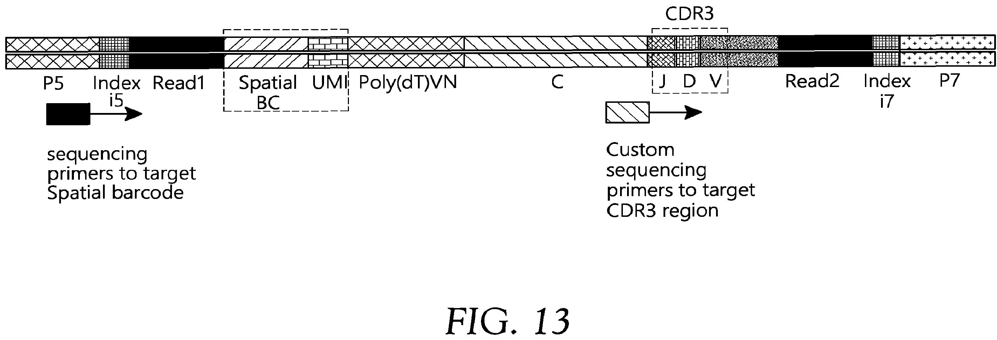

FIG. 13 shows a sequencing strategy with a primer specific complementary to the sequencing flow cell attachment sequence (e.g., P5) and a custom sequencing primer complementary to a portion of the constant region of the analyte. This sequencing strategy targets the constant region to obtain the sequence of the CDR regions, including CDR3, while concurrently or sequentially sequencing the spatial barcode (BC) and/or unique molecular identifier (UMI) of the capture probe. By capturing the sequence of a spatial barcode, UMI and a V(D)J region the receptor is not only determined, but its spatial location and abundance within a cell or tissue is also identified.

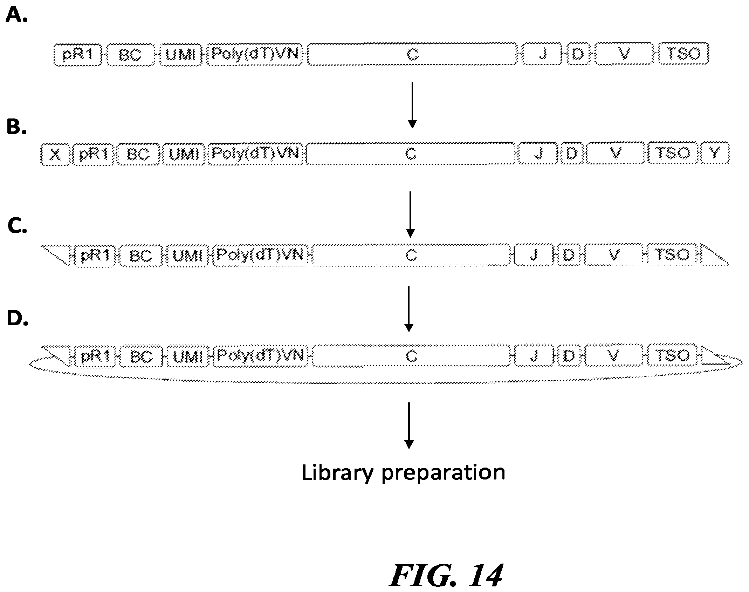

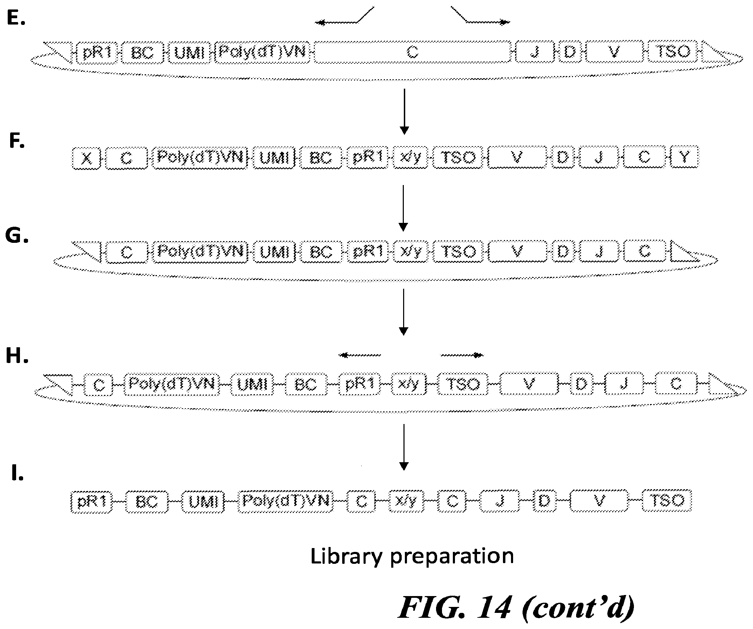

FIG. 14 shows an exemplary nucleic acid library preparation method to remove a portion of an analyte sequence via double circularization of a member of a nucleic acid library. Panel A shows an exemplary member of a nucleic acid library including, in a 5' to 3' direction, a first adaptor (e.g., primer sequence R1, pR1 (e.g., Read 1)), a barcode (e.g., a spatial barcode or a cell barcode), a unique molecular identifier (UMI), a capture domain (e.g., poly(T) VN sequence), a sequence complementary to an analyte (C, J, D and V), and a second adaptor (e.g., template switching oligonucleotide sequence (TSO)). For purposes of this example an analyte including a constant region (C) and V(D)J sequence are shown, however, the methods described herein can be equally applied to other analyte sequences in a nucleic acid library. Panel B shows the exemplary member of a nucleic acid library where additional sequences can be added to both the 3' and 5' ends of the nucleic acid member (shown as a X and Y) via a PCR reaction. The additional sequences added can include a recognition sequence for a restriction enzyme (e.g., restriction endonuclease). The restriction recognition sequence can be for a rare restriction enzyme. The exemplary member of the nucleic acid library shown in Panel B can be digested with a restriction enzyme to generate sticky ends shown in Panel C (shown as triangles) and can be intramolecularly circularized by ligation to generate the circularized member of the nucleic acid library shown in Panel D. The ligation can be performed with a DNA ligase. The ligase can be T4 ligase. A primer pair can be hybridized to a circularized nucleic acid member, where a first primer hybridizes to a 3' portion of a sequence encoding the constant region (C) and includes a second restriction enzyme (e.g., restriction endonuclease) sequence that is non-complementary to the analyte sequence, and where a second primer hybridized to a 5' portion of a sequence encoding the constant region (C), and where the second primer includes a second restriction enzyme sequence (Panel E). The first primer and the second primer can generate a linear amplification product (e.g., a first double-stranded nucleic acid product) as shown in Panel F, which includes the second restriction enzyme recognition sequences (shown as X and Y end sequences). The linear amplification product (Panel F) can be digested with a second restriction enzyme to generate sticky ends and can be intramolecularly ligated with a ligase (e.g., T4 DNA ligase) to generate a second double-stranded circularized nucleic acid product as shown in Panel G. The second double-stranded circularized nucleic product (Panel G) can be amplified with a third primer, pR1, substantially complementary to the first adaptor (e.g., Read 1) sequence and a fourth primer substantially complementary to the second adapter (e.g., TSO) as shown in Panel H to generate a version of the double-stranded member of the nucleic acid library lacking all, or a portion of, the sequence encoding the constant region (C) of the analyte (Panel I). The resulting double-stranded member of the nucleic acid library lacking all or a portion of the constant region can undergo library preparation methods, such as library preparation methods used in single-cell or spatial analyses. For example, the double-stranded member of the nucleic acid library lacking all, or a portion of, the sequence encoding the constant region of the analyte can be fragmented, followed by end repair, a-tailing, adaptor ligation, and/or additional amplification (e.g., PCR). The fragments can then be sequenced using, for example, paired-end sequencing using TruSeq Read 1 and TruSeq Read 2 as sequencing primer sites or any other sequencing method described herein.

As such, sequences can be determined from regions more than about 1 kb away from the end of an analyte (e.g., 3' end) and can link such a sequence to a barcode sequence (e.g., a spatial barcode, a cell barcode) in library preparation methods (e.g., sequencing preparation). For purposes of this example an analyte including a constant region (C) and V(D)J sequences are shown, however, the methods described herein can be equally applied to other analyte sequences in a nucleic acid library.

An exemplary member of a nucleic acid library can be prepared as shown in FIG. 14 to generate a first double-stranded circularized nucleic acid product shown in Panel D of FIG. 14 as previously described.

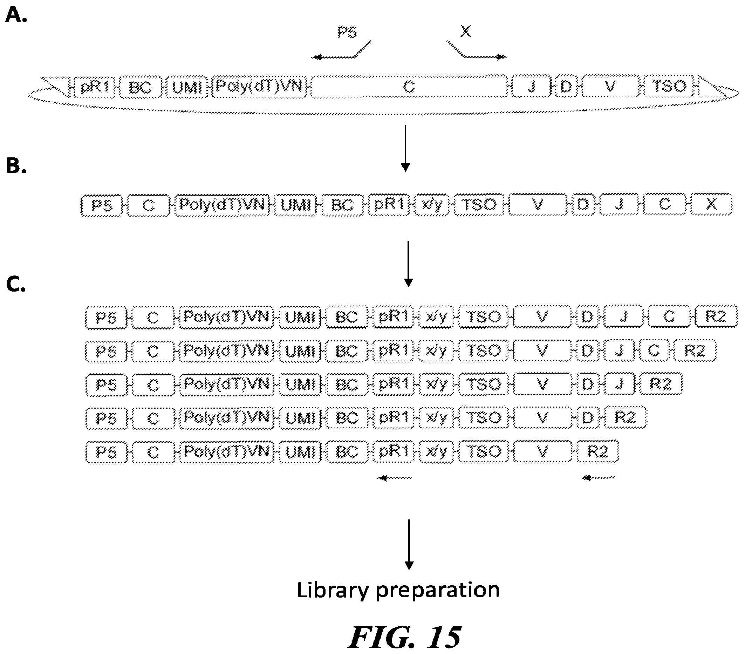

FIG. 15 depicts another exemplary workflow for processing such double-stranded circularized nucleic acid product. A primer pair can be contacted with the double-stranded circularized nucleic acid produce with a first primer that can hybridize to a sequence from a 3' region of the sequence encoding the constant region of the analyte and a sequence including a first functional domain (e.g., P5). The second primer can hybridize to a sequence from a 5' region of the sequence encoding the constant region of the analyte, and includes a sequence including a second functional domain (shown as "X") as shown in Panel A. Amplification of the double-stranded circularized nucleic acid product results in a linear product as shown in Panel B, where all, or a portion of, the constant region (C) is removed. The first functional domain can include a sequencer specific flow cell attachment sequence (e.g., P5). The second functional domain can include an amplification domain such as a primer sequence to amplify the nucleic acid library prior to further sequencing preparation. The resulting double-stranded member of the nucleic acid library lacking all or a portion of the constant region can undergo library preparation methods, such as library preparation methods used in single-cell or spatial analyses. For example, the double-stranded member of the nucleic acid library lacking all, or a portion of, the sequence encoding the constant region of the analyte can be fragmented, followed by end repair, A-tailing, adaptor ligation, and/or amplification (e.g., PCR) (Panel C). The fragments can then be sequenced using, for example, paired-end sequencing using TruSeq Read 1 and TruSeq Read 2 as sequencing primer sites (Panel C, arrows), or any other sequencing method described herein. After library preparation methods described herein, a different sequencing primer for the first adaptor (e.g., Read 1) is used since the orientation of the first adaptor (e.g., Read 1) sequence will be reversed. Accordingly, sequences can be determined from regions more than about 1 kb away from the end of an analyte (e.g., 3' end) and can link such a sequence to a barcode sequence (e.g., a spatial barcode, a cell barcode) in further library preparation methods (e.g., sequencing preparation). For purposes of this example an analyte including a constant region (C) and V(D)J sequence are shown, however, the methods described herein can be applied to other analyte sequences in a nucleic acid library as well.

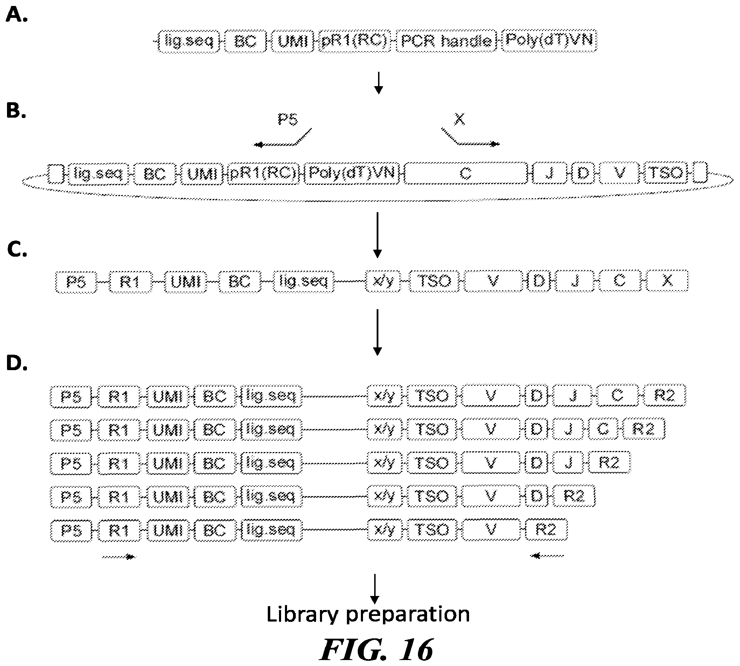

FIG. 16 shows an exemplary nucleic acid library preparation method to remove all or a portion of a constant sequence of an analyte from a member of a nucleic acid library via circularization. Panels A and B shows an exemplary member of a nucleic acid library including, in a 5' to 3' direction, a ligation sequence, a barcode sequence, a unique molecular identifier, a reverse complement of a first adaptor (e.g., primer sequence pR1 (e.g., Read 1)), a capture domain, a sequence complementary to the captured analyte sequence, and a second adapter (e.g., TSO sequence). The ends of the double-stranded nucleic acid can be ligated together via a ligation reaction where the ligation sequence splints the ligation to generate a circularized double-stranded nucleic acid as shown in Panel B. The circularized double-stranded nucleic acid can be amplified with a pair of primers to generate a linear nucleic acid product lacking all or a portion of the constant region of the analyte (Panels B and C). The first primer can include a sequence substantially complementary to the reverse complement of the first adaptor and a first functional domain. The first functional domain can be a sequencer specific flow cell attachment sequence (e.g., P5). The second primer can include a sequence substantially complementary to a sequence from a 5' region of the sequence encoding the constant region of the analyte, and a second functional domain. The second functional domain can include an amplification domain such as a primer sequence to amplify the nucleic acid library prior to further sequencing preparation. The resulting double-stranded member of the nucleic acid library lacking all or a portion of the constant region can undergo library preparation methods, such as library preparation methods used in single-cell or spatial analyses. For example, the double-stranded member of the nucleic acid library lacking all, or a portion of, the sequence encoding the constant region of the analyte can be fragmented, followed by end repair, A-tailing, adaptor ligation, and/or amplification (e.g., PCR) (Panel C). The fragments can then be sequenced using, for example, paired-end sequencing using TruSeq Read 1 and TruSeq Read 2 as sequencing primer sites, or any other sequencing method described herein (Panel D). After library preparation methods (e.g., described herein), sequencing primers can be used since the orientation of Read 1 will be in the proper orientation for sequencing primer pR1. Accordingly, sequences can be determined from regions more than about 1 kb away from the end of an analyte (e.g., 3' end) and can link such a sequence to a barcode sequence (e.g., a spatial barcode, a cell barcode) in further library preparation methods (e.g., sequencing preparation). For purposes of this example an analyte including a constant region (C) and V(D)J sequence are shown, however, the methods described herein can be applied to other analyte sequences in a nucleic acid library as well.

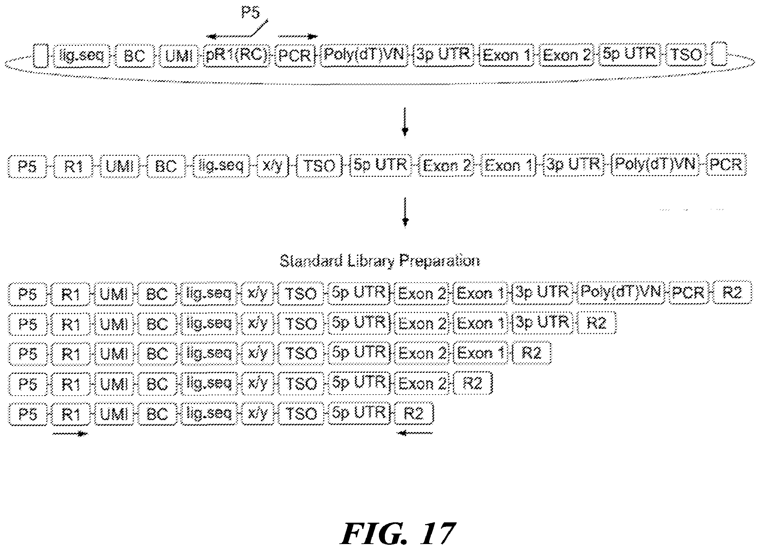

FIG. 17 shows an exemplary nucleic acid library method to reverse the orientation of an analyte sequence in a member of a nucleic acid library. Panel A shows an exemplary member of a nucleic acid library including, in a 5' to 3' direction, a ligation sequence, a barcode (e.g., a spatial barcode or a cell barcode), unique molecular identifier, a reverse complement of a first adaptor, an amplification domain, a capture domain, a sequence complementary to an analyte, and a second adapter. The ends of the double-stranded nucleic acid can be ligated together via a ligation reaction where the ligation sequence splints the ligation to generate a circularized double-stranded nucleic acid also shown in Panel A. The circularized double-stranded nucleic acid can be amplified to generate a linearized double-stranded nucleic acid product, where the orientation of the analyte is reversed such that the 5' sequence (e.g., 5' UTR) is brought in closer proximity to the barcode (e.g., a spatial barcode or a cell barcode) (Panel B). The first primer includes a sequence substantially complementary to the reverse complement of the first adaptor and a functional domain. The functional domain can be a sequencer specific flow cell attachment sequence (e.g., P5). The second primer includes a sequence substantially complementary to the amplification domain. The resulting double-stranded member of the nucleic acid library including a reversed analyte sequence (e.g., the 5' end of the analyte sequence is brought in closer proximity to the barcode) can undergo library preparation methods, such as library preparation methods used in single-cell or spatial analyses. For example, the double-stranded member of the nucleic acid library lacking all, or a portion of, the sequence encoding the constant region of the analyte can be fragmented, followed by end repair, A-tailing, adaptor ligation, and/or amplification (e.g., PCR) (Panel C). The fragments can then be sequenced using, for example, paired-end sequencing using TruSeq Read 1 and TruSeq Read 2 as sequencing primer sites, or any other sequencing method described herein. Accordingly, sequences from the 5' end of an analyte will be included in sequencing libraries (e.g., paired end sequencing libraries). Any type of analyte sequence in a nucleic acid library can be prepared by the methods described in this Example (e.g., reversed).

### Spatial analysis using array features comprising poly(T) and poly(GI) capture domains

Provided herein are methods of determining a location of a target nucleic acid in a biological sample that include: (a) contacting the biological sample with an array comprising a feature, where the feature comprises an attached first and second probe, wherein: a 5' end of the first probe is attached to the feature; the first probe comprises in a 5' to a 3' direction: a spatial barcode and a poly(T) capture domain, where the poly(T) capture domain binds specifically to the target nucleic acid; a 5' end of the second probe is attached to the feature; a 3' end of the second probe is reversibly blocked; and the second probe comprises a poly(GI) capture domain; (b) extending a 3' end of the first probe to add a sequence that is complementary to a portion of the target nucleic acid; (c) ligating an adapter to the 5' end of the target nucleic acid specifically bound to the first probe; (d) adding a sequence complementary to the adapter to the 3' end of the first probe; (e) adding non-templated cytosines to the 3' end of the first probe to generate a poly(C) sequence, where the poly(C) sequence specifically binds to the poly(GI) capture domain of the second probe; (f) unblocking the 3' end of the second probe and extending the 3' end of the second probe to add a sequence comprising a sequence in the target nucleic acid and a sequence that is complementary to the spatial barcode; (g) cleaving a region of the second probe at a cleavage site that is 5' to the poly(GI) capture domain, thereby releasing the second probe from the feature; and (h) determining (i) all or a part of the sequence of the spatial barcode, or a complement thereof, and (ii) all or a part of the sequence of the target nucleic acid, or a complement thereof, and using the sequences of (i) and (ii) to determine the location of the target nucleic acid in the biological sample.

A feature can include two or more pairs of a first and a second probe (e.g., any of the first and second probes described in this section). A first pair of a first and a second probe at a feature, as compared to a second pair of a first and a second probe at the feature, can have a different first and/or second probe as compared to first and/or second probe of the second pair (e.g., a different capture domain in the first probe and/or a different barcode in the first and/or second probes). The spatial barcode in the first probe of the first pair and the spatial barcode in the first probe of the second pair may be the same. The spatial barcode in the first probe of the first pair and the spatial barcode in the first probe of the second pair may be different. The capture domain of the first probe of the first pair may be the same as the capture domain of the first probe of the second pair. The capture domain of the first probe of the first pair may be different from the capture domain of the first probe of the second pair.

The capture domain on the first probe may have a poly(T) capture domain, where the poly(T) capture domain is configured to interact with the target nucleic acid (e.g., positioned at the 3' end of the first probe). For example, the poly(T) capture domain specifically binds to a messenger RNA (mRNA), via the poly(A) tail of the mRNA. For example, a poly(T) capture domain can include at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, or at least 30 contiguous thymidines.

The poly(GI) capture domain of the second probe may be configured to interact with a poly(C) tail of an oligonucleotide, e.g., a poly(C) tail added to the 3' end of the extended first probe. The poly(C) tail may be added to the 3' end of the first probe after the extension of the first probe to add a sequence that is complementary to a portion of the target nucleic acid. The poly(GI) capture domain may comprise a sequence of at least 5 contiguous guanosine(s) and/or inosine(s). For example, a poly(GI) capture domain may comprise a sequence of (GGI)n, wherein n is about 3 to about 20. The poly(GI) capture domain may comprise a sequence of (GGI)n, wherein n is 1, 2, 3, 4, 5, 6, 7, 8, 9, 10, 11, 12, 13, 14, 15, 16, 17, 18, 19, or 20. For example, a poly(GI) capture domain may comprise a sequence of (GI)n, wherein n is about 4 to about 30. For example, a poly(GI) capture domain may comprise a sequence of (GI)n, wherein n is 4, 5, 6, 7, 8, 9, 10, 11, 12, 13, 14, 15, 16, 17, 18, 19, 20, 21, 22, 23, 24, 25, 26, 27, 28, 29, or 30. For example, a poly(GI) capture domain may comprise a sequence of (IG)n, wherein n is about 4 to about 30. For example, a poly(GI) capture domain may comprise a sequence of (IG)n, wherein n is 4, 5, 6, 7, 8, 9, 10, 11, 12, 13, 14, 15, 16, 17, 18, 19, 20, 21, 22, 23, 24, 25, 26, 27, 28, 29, or 30.

The second probe can comprise a spatial barcode, which is positioned 5' to the poly(GI) capture domain. The spatial barcode in the first probe may be different from the spatial barcode sequence in the second probe. The spatial barcode in the first probe may be the same as the spatial barcode sequence in the second probe.

Both the first and the second probes may be cleavable. The first probe and the second probe may have different cleavage site and may be cleavable using different methods. The first probe and the second probe may have the same cleavable site and may be cleavable using the same method. The cleavage domain of the first probe may be 5' to the poly(T) capture domain and/or the cleavage domain of the second probe may be 5' to the poly(GI) capture domain.

The first probe may not be cleavable and the second probe may be cleavable. The cleavage site of the second probe may be 5' to the poly(GI) capture domain of the second probe. The cleavage site on the second probe may be a uracil. The uracil may be cleaved by USER (Uracil-Specific Excision Reagent).

The first probe may further comprise a unique molecular identifier (UMI). The second probe may further comprise a unique molecular identifier (UMI). The UMI in the first probe and the UMI in the second probe may comprise different sequences. The UMI in the first probe and the UMI in the second probe may comprise the same sequence.

Step (h) may include sequencing all or a part of the sequence of the spatial barcode, or a complement thereof, and sequencing all of a part of the sequence of the target nucleic acid, or a complement thereof. The sequencing can be performed using any of the aforementioned methods. Step (h) may include sequencing the full-length sequence of the spatial barcode, or a complement thereof. Step (h) may include sequencing a part of the sequence of the spatial barcode, or a complement thereof. Step (h) may include sequencing the full-length sequence of the target nucleic acid, or a complement thereof. Step (h) may include sequencing a part of the target nucleic acid, or a complement thereof. The sequencing may be performed using high throughput sequencing. The target nucleic acid may be sequenced from the 5' end of the target nucleic acid. The target nucleic acid may be sequenced from the 3' end of the target nucleic acid. The target nucleic acid may be sequenced from both the 3' end and the 5' end of the target nucleic acid.

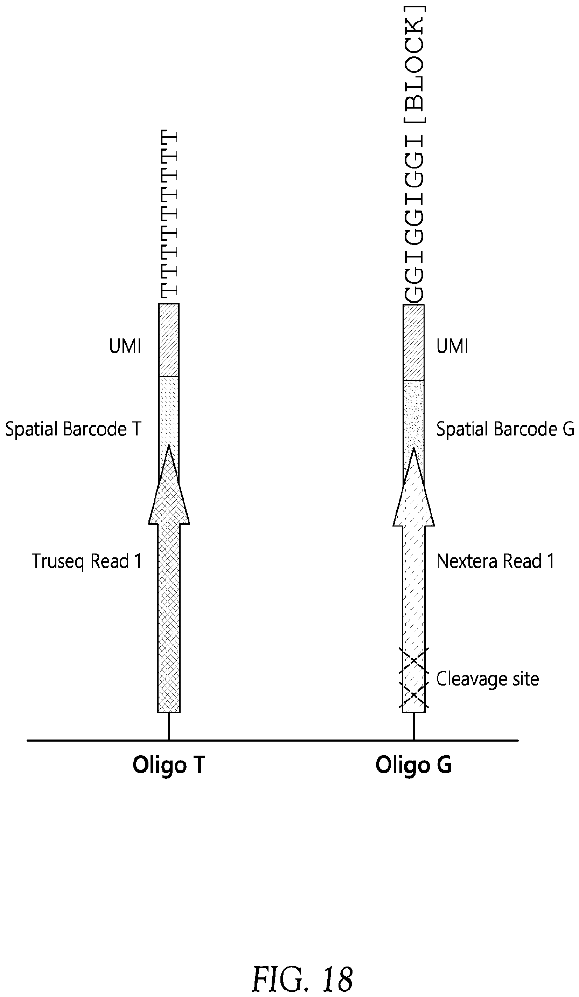

FIG. 18 is a schematic diagram showing an exemplary feature comprising an attached first and second probe. The first probe comprises in a 5' to 3' direction: a functional domain comprising a Truseq Read 1 primer, a spatial barcode, a UMI, and a poly(T) capture domain, where the poly(T) capture domain binds specifically to the target nucleic acid. The 5' end of the first probe is attached to the feature.

The second probe comprises in a 5' to 3' direction: a cleavage domain, a functional domain comprising a Nextera Read 1 primer, a spatial barcode, a UMI, and a poly(GI) capture domain. The 5' end of the second probe is attached to the feature. The poly(GI) capture domain may comprise a sequence of (GGI)n, wherein n is about 3 to about 20. The poly(GI) capture domain may comprise a sequence of (GGI)n, wherein n is 1, 2, 3, 4, 5, 6, 7, 8, 9, 10, 11, 12, 13, 14, 15, 16, 17, 18, 19, or 20. The 3' end of the second probe may be reversibly blocked.

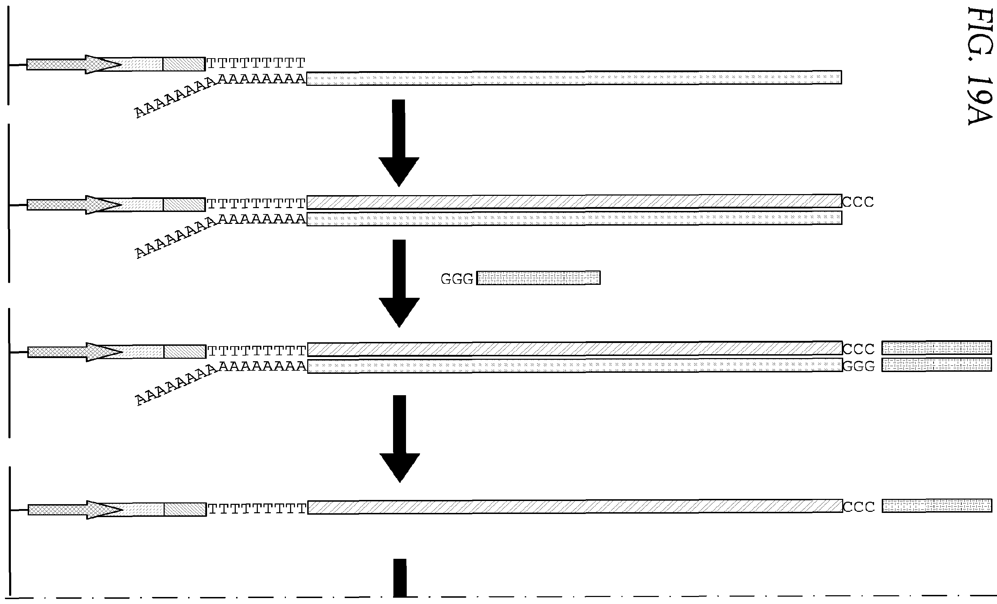

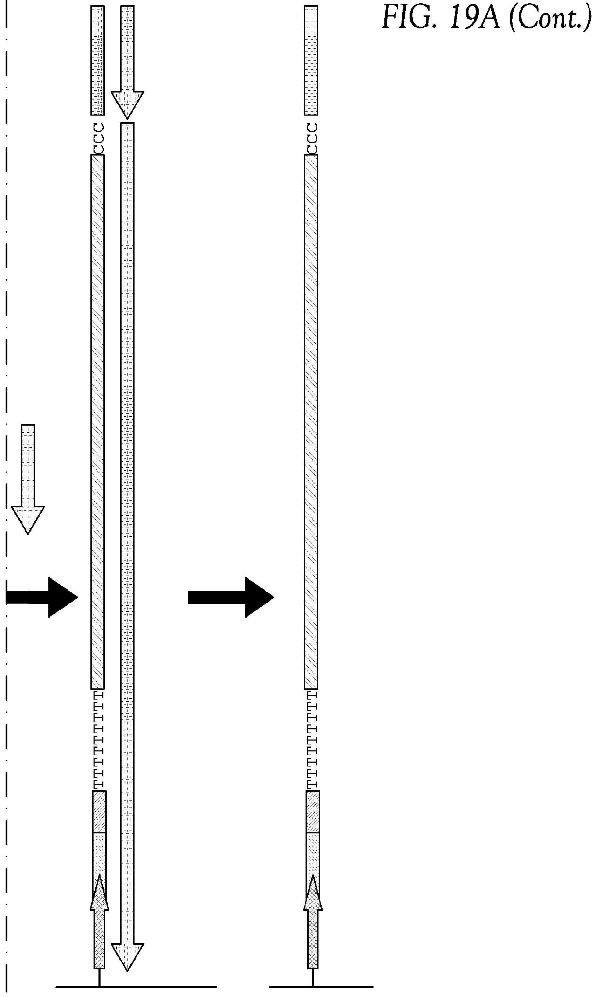

FIG. 19A is an exemplary diagram showing, from left to right, the annealing of the target analyte (e.g., target nucleic acid) to the poly(T) capture domain of the first probe; the extension of the first probe to add a sequence that is complementary to a portion of the target nucleic acid; the ligation of an adaptor to the 5' end of the target nucleic acid specifically bound to the first probe; the addition of a sequence complementary to the adaptor to the 3' end of the first probe; the releasing of the target nucleic acid from the first probe; the generation of a complement of the extended first probe; and the releasing of the complement of the extended first probe. The released target nucleic acid may be sequenced. The released complement of the extended first probe may be sequenced.

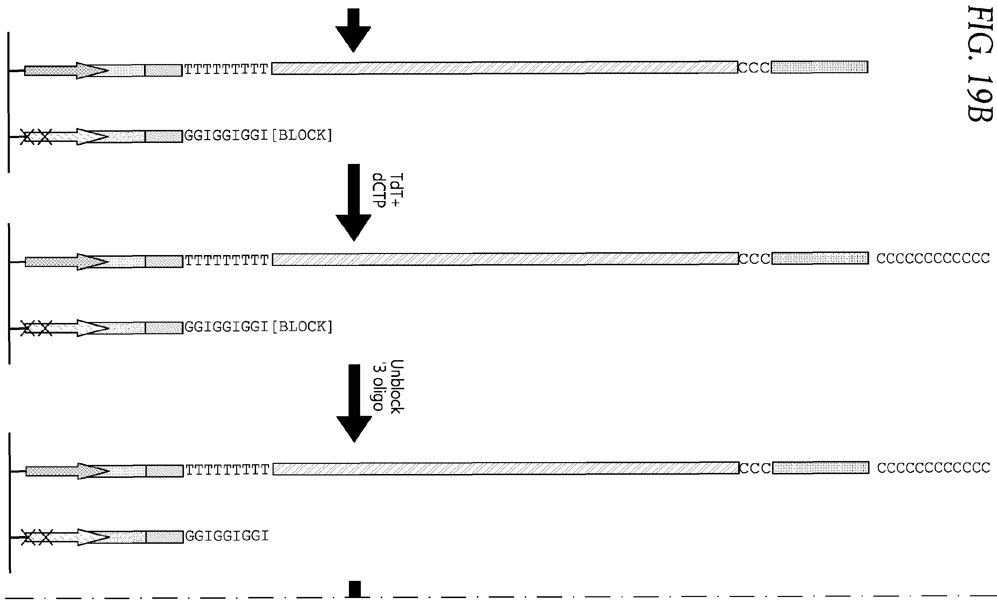

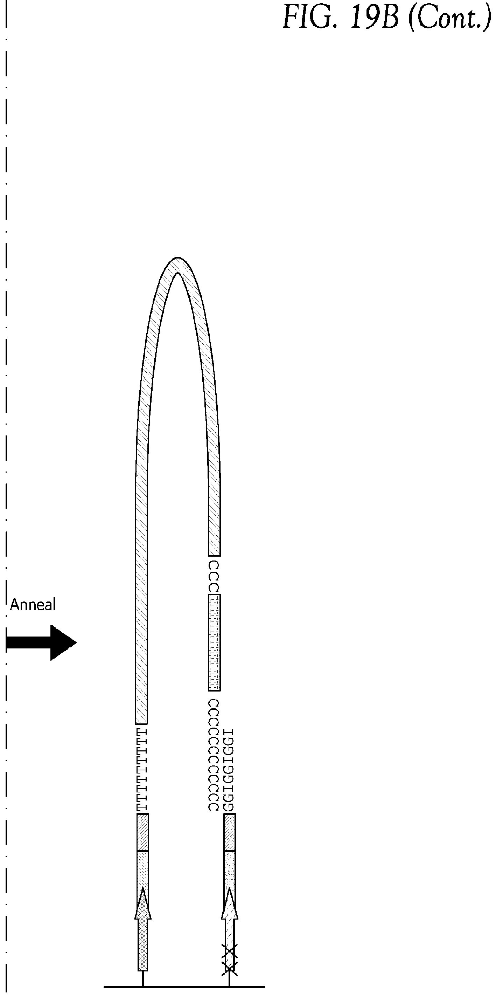

FIG. 19B is an exemplary diagram showing, from left to right, the addition of non-templated cytosines to the 3' end of the extended first probe (e.g., extended to include a sequence that is complementary to part of a sequence of a target nucleic acid) to generate a poly(C) sequence, where the poly(C) sequence specifically binds to the poly(GI) capture domain of the second probe; the unblocking of the 3' end of the second capture probe; and the hybridization of the poly(C) sequence on the 3' end of the first probe to the poly(GI) capture domain at the 3' end of the second probe.

The second probe may comprise a poly(T) capture domain and a poly(A) sequence is added to the 3' end of the extended first probe (e.g., extended to add a sequence that is complementary to a portion of the sequence of a target nucleic acid), and the poly(A) sequence hybridizes to the poly(T) capture domain of the second probe.

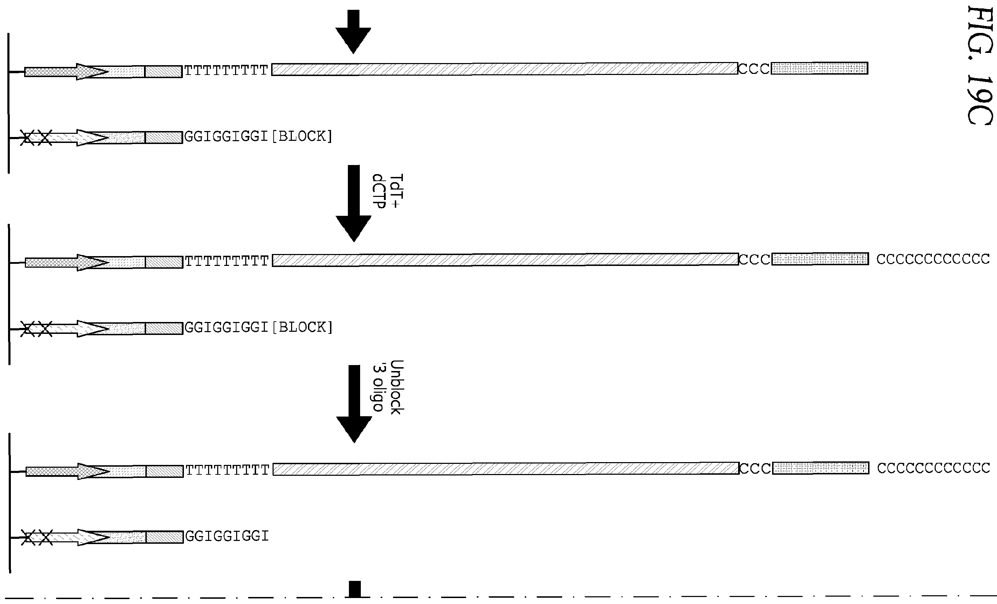

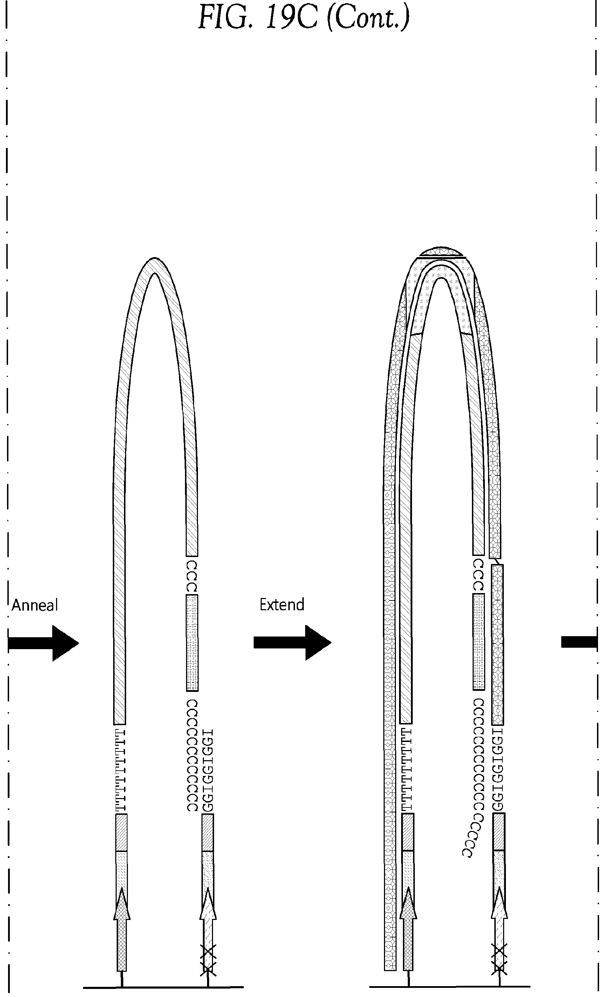

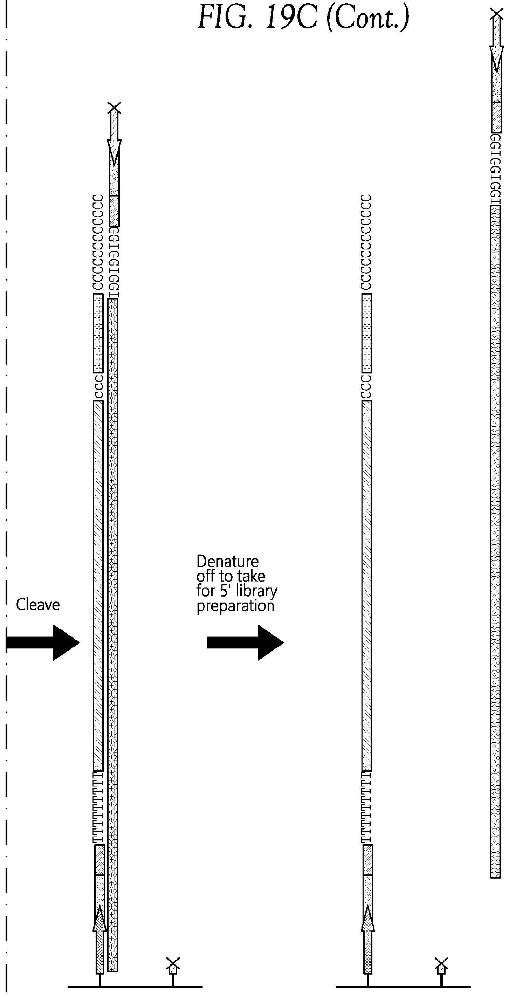

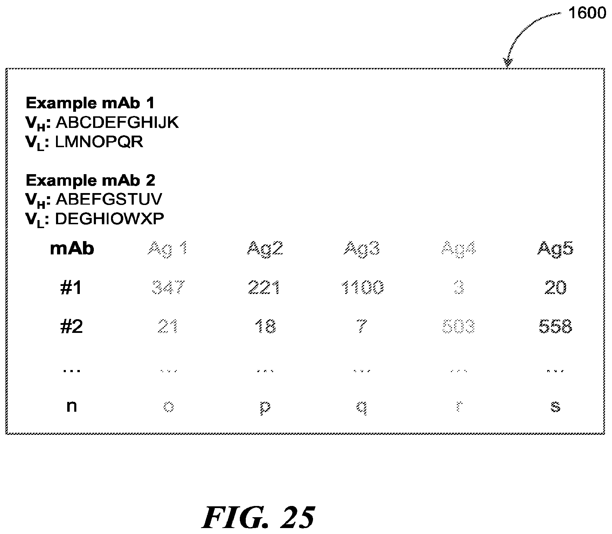

FIG. 19C is an exemplary diagram showing from left to right, the addition of non-templated cytosines to the 3' end of the extended first probe (e.g., extended to include a sequence that is complementary to a portion of the sequence of a target nucleic acid, and optionally, further comprising an adaptor sequence or a functional domain) to generate a poly(C) sequence, where the poly(C) sequence specifically binds to the poly(GI) capture domain of the second probe; the unblocking of the 3' end of the second probe; the hybridizing the poly(C) sequence on the first probe to the poly(GI) capture domain on the second capture probe; the extension of the 3' end of the second probe to add a sequence complementary to the extended first capture probe. The final step depicted in FIG. 25C is the releasing of the extended second probe sequence from the feature.

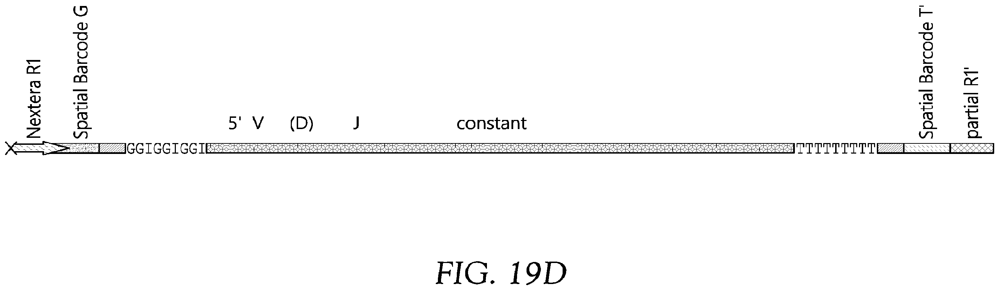

FIG. 19D is a schematic diagram showing an example of a sequence generated by the methods described in this section. The exemplary sequence shown comprises, from 5' end to 3' end, the functional domain of the second probe, which comprises a sequencing primer; the spatial barcode of the second probe; the UMI sequence of the second probe; the poly(GI) sequence of the second probe; the target nucleic acid sequence (from 5' end to 3' end); a sequence complementary to the UMI sequence of the first probe; a sequence complementary to the spatial barcode of the first probe; and a sequence complementary to part or the full sequence of the functional domain of the first probe, which comprises a sequencing primer. the two sequencing primers may have the same sequence. The two sequencing primers may have different sequences.

Further steps of the methods described in this section include, for example, determining (i) all or a part of the sequence of the spatial barcode on either end of the sequence depicted in FIG. 19D, or a complement thereof, and (ii) all or a part of the sequence of the target nucleic acid, or a complement thereof, and using the sequences of (i) and (ii) to determine the location of the target nucleic acid in the biological samples.

The methods described in this section allows for the sequencing of the target nucleic acid from either the 3' end or the 5' end, or both the 3' and the 5' ends of the target nucleic acid. For target nucleic acids that have large sizes (e.g., larger than 1 kb), the methods allow more accurate spatial sequence information to be obtained.

Also described in this section is an array comprising a feature, where the feature comprises an attached first and second probe, wherein: a 5' end of the first probe is attached to the feature; the first probe comprises in a 5' to a 3' direction: a spatial barcode and a poly(T) capture domain, wherein the poly(T) capture domain binds specifically to the target nucleic acid; a 5' end of the second probe is attached to the feature; a 3' end of the second probe is reversibly blocked; and the second probe comprises a poly(GI) capture domain.

A feature can include two or more pairs of a first and a second probe (e.g., any of the first and second probes described in this section). A first pair of a first and a second probe at a feature, as compared to a second pair of a first and a second probe at the feature, can have a different first and/or second probe as compared to first and/or second probe of the second pair (e.g., a different capture domain in the first probe and/or a different barcode in the first and/or second probes). The spatial barcode in the first probe of the first pair and the spatial barcode in the first probe of the second pair may be the same. The spatial barcode in the first probe of the first pair and the spatial barcode in the first probe of the second pair may be different. The capture domain of the first probe of the first pair may be the same as the capture domain of the first probe of the second pair. The capture domain of the first probe of the first pair may be different from the capture domain of the first probe of the second pair.

The capture domain on the first probe may have a poly(T) capture domain, where the poly(T) capture domain is configured to interact with a target nucleic acid (e.g., positioned at the 3' end of the first probe). For example, the poly(T) capture domain specifically binds to a messenger RNA (mRNA), via the poly(A) tail of the mRNA. For example, a poly(T) capture domain can include at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, or at least 30 contiguous thymidines.

The poly(GI) capture domain may comprise a sequence of at least 5 contiguous guanosine(s) and/or inosine(s). For example, a poly(GI) capture domain may comprise a sequence of (GGI)n, wherein n is about 3 to about 20. The poly(GI) capture domain may comprise a sequence of (GGI)n, wherein n is 1, 2, 3, 4, 5, 6, 7, 8, 9, 10, 11, 12, 13, 14, 15, 16, 17, 18, 19, or 20. For example, a poly(GI) capture domain may comprise a sequence of (GI)n, wherein n is about 4 to about 30. For example, a poly(GI) capture domain may comprise a sequence of (GI)n, wherein n is 4, 5, 6, 7, 8, 9, 10, 11, 12, 13, 14, 15, 16, 17, 18, 19, 20, 21, 22, 23, 24, 25, 26, 27, 28, 29, or 30. For example, a poly(GI) capture domain may comprise a sequence of (IG)n, wherein n is about 4 to about 30. For example, a poly(GI) capture domain may comprise a sequence of (IG)n, wherein n is 4, 5, 6, 7, 8, 9, 10, 11, 12, 13, 14, 15, 16, 17, 18, 19, 20, 21, 22, 23, 24, 25, 26, 27, 28, 29, or 30.

The second probe can comprise a spatial barcode, which is positioned 5' to the poly(GI) capture domain. The spatial barcode in the first probe may be different from the spatial barcode sequence in the second probe. The spatial barcode in the first probe may be the same as the spatial barcode sequence in the second probe.

Both the first and the second probes may be cleavable. The first probe and the second probe may have different cleavage site and are cleavable using different methods. The first probe and the second probe may have the same cleavable site and are cleavable using the same method. The cleavage domain of the first probe may be 5' to the poly(T) capture domain and/or the cleavage domain of the second probe may be 5' to the poly(GI) capture domain.

The first probe may not cleavable and the second probe is cleavable. The cleavage site of the second probe may be 5' to the poly(GI) capture domain of the second probe. The cleavage site on the second probe may be a uracil. The uracil may be cleaved by USER (Uracil-Specific Excision Reagent).

The first probe may further comprise a unique molecular identifier (UMI). The second probe may further comprise a unique molecular identifier (UMI). The UMI in the first probe and the UMI in the second probe may comprise different sequences. The UMI in the first probe and the UMI in the second probe may comprise the same sequence.

The first and/or second probe can further include a functional domain (e.g., a sequencing handle). The first and second probe may comprise a functional domain. The functional domain the first and second probes may be the same. The functional domain in the first probe and the functional domain in the second probe may be different.

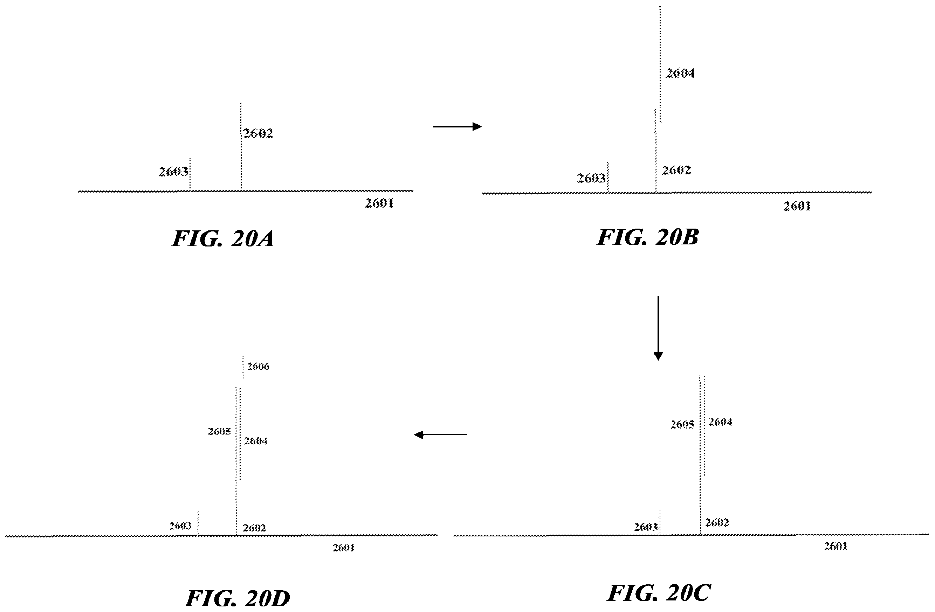

An exemplary, non-limiting workflow is depicted in FIGS. 20A - 20J. In the workflow depicted in FIGS. 20A - 20J, presence and/or location (e.g., spatial location) of a biological analyte may be determined and/or a 5' sequence (e.g., sequence of a 5' region) of a polynucleotide sequence of a biological analyte may be detected and/or determined. A "5' region" refers to a sequence that is at or near the 5' end of a polynucleotide sequence, or a sequence that is closer in proximity to the 5' end than the 3' end of a polynucleotide sequence. A biological sample is contacted with a substrate 2601. The substrate includes an attached first polynucleotide probe 2602 and an attached second polynucleotide probe 2603. (FIG. 20A) The first polynucleotide probe 2602 may include, in a 5' - 3' direction: a barcode (e.g., spatial barcode); a first capture domain; and an extendible 3' end. Depicted in FIGS. 20A - 20J, the second polynucleotide probe 2603 may include a second capture domain and an extendible 3' end, and both the first polynucleotide probe 2602 and the second polynucleotide 2603 may be attached at their 5' ends to the substrate 2601.

A biological sample is contacted with the substrate 2601 under conditions in which a target polynucleotide sequence 2604 of a biological analyte binds (e.g., hybridizes) to the first capture domain of the first polynucleotide probe 2602. (FIG. 20B) The first capture domain may include a sequence specific for an RNA molecule. The first capture domain may include a poly-T sequence. The first capture domain may include a sequence complementary to a region of an immune molecule, such as the constant region of a TCR or BCR sequence (as described herein). The first capture domain may include a sequence complementary to a region of an immunoglobulin molecule, such as one or more CDRs of an immunoglobulin heavy or light chain.

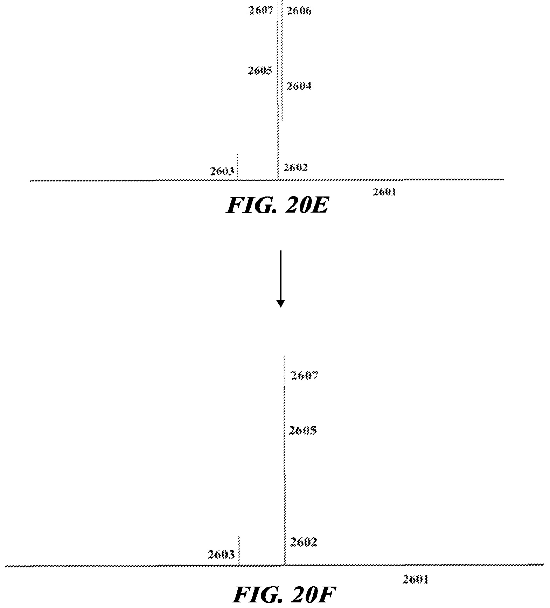

The extendible 3' end of the first polynucleotide probe 2602 is extended to produce a first extension product 2605. (FIG. 20C) An adapter is attached to the 3' end of the first extension product 2605. Depicted in FIG. 20D, untemplated nucleotides may be added to the 3' end of the first extension product 2605. A template switch oligonucleotide (TSO) 2606 binds (e.g., hybridizes) to the untemplated nucleotides, and then the 3' end of the first extension product is extended, producing a polynucleotide sequence 2607 that is complementary to the TSO sequence. (FIG. 20E). The target polynucleotide sequence 2604 and TSO 2606 are stripped away (e.g., denatured). (FIG. 20F)

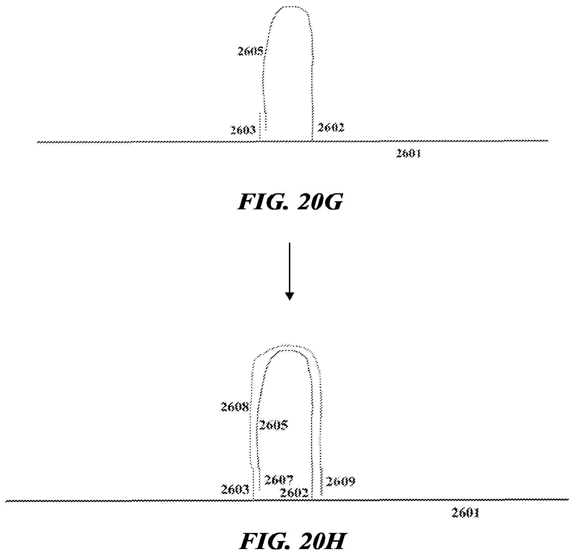

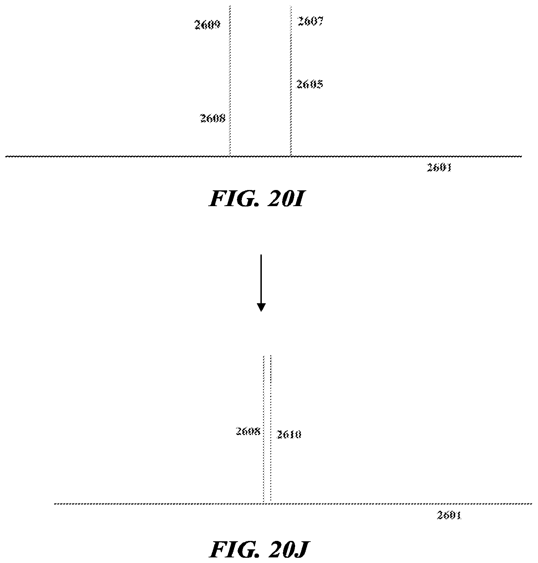

Depicted in FIG. 20G, the second capture domain of the second capture probe 2603 may include a sequence that is complementary to the adapter sequence 2607, i.e., the second capture domain contains the TSO sequence or a partial sequence thereof. The adapter 2607 at the 3' end of the first extension product 2605 binds to the second capture domain sequence at the 3' end of the second capture probe 2603. The 3' end of the second capture probe 2603 is extended, producing a second extension product 2608, which includes a 3' sequence that is complementary to the sequence of the first capture probe or a portion thereof 2609. (FIG. 20H) The first extension product 2605 with 3' adapter sequence 2607 includes a 3' sequence complementary to the target polynucleotide proximal to the first capture domain sequence, and may be used for preparation of a 3' sequence library; and/or the second extension product 2608 with 3' sequence complementary to the first polynucleotide probe 2607 includes a 5' sequence of the target polynucleotide proximal to the second capture domain sequence, and may be used for preparation of a 5' sequence library. (FIG. 20I). Depicted in FIG. 20J, a copy 2610 of the second extension product (e.g., amplification product) 2608 may be produced. The first and/or second extension product, and/or copy (e.g., amplification product thereof) may be detected and/or sequenced, and the resulting information obtained may be used to determine presence and/or location (e.g., spatial location) of the biological analyte in the biological sample.

The first analyte of the ABM-expressing cell may be a cDNA of an mRNA transcript encoding the ABM. The cDNA may be generated by in situ reverse transcription of the mRNA encoding the ABM. The in situ reverse transcription may comprise: contacting the tissue sample on the array with a first primer comprising a (i) sequence that hybridizes to a complementary sequence in the mRNA transcript encoding the ABM and (ii) a functional domain; hybridizing the first primer to the mRNA transcript encoding the ABM and extending the first primer using the mRNA transcript as a template to generate an extension product; attaching a homopolynucleotide sequence to the 3' end of the extension product; hybridizing a second primer to the 3' end of the homopolynucleotide sequence of the extension product of, wherein the second primer comprises a capture handle sequence; extending the extension product using the second primer as a template. The method may further comprise hybridizing the capture handle sequence of the extension product (or reverse complement thereof) to the capture sequence of the capture domain of the capture probe.

FIG. 2 is a schematic showing generation of a cDNA by in situ reverse transcription of a target nucleic acid (e.g., mRNA) from a first primer including a sequence complementary to the target nucleic acid and a functional domain and a second primer that includes a capture sequence (e.g., a capture sequence capable of hybridizing to a capture domain of a capture probe, or a complement thereof) and a sequence complementary a homopolynucleotide sequence.

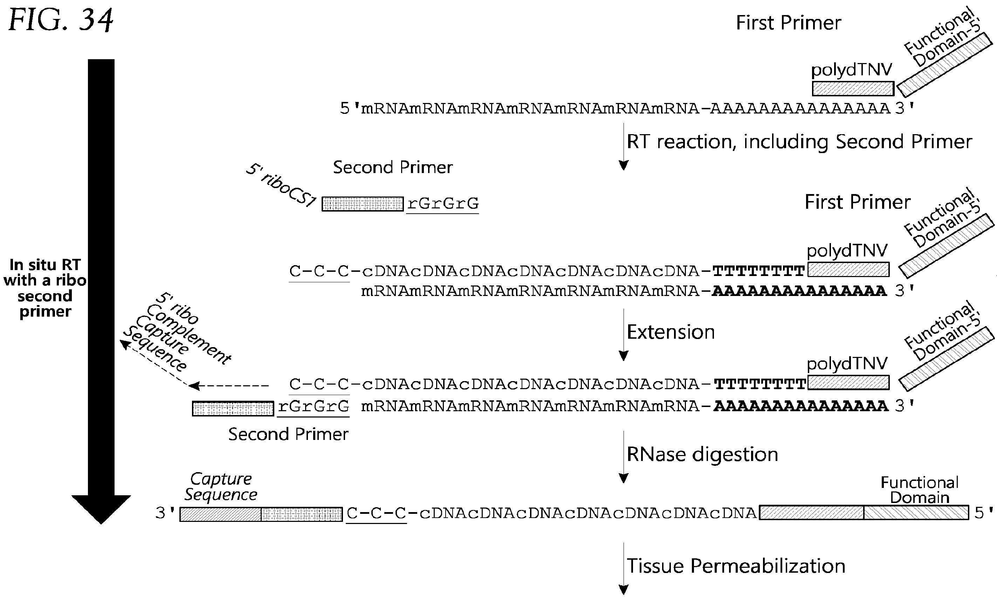

More specifically, target nucleic acids are contacted with a first primer that includes a sequence complementary to the target nucleic acid (e.g., poly(dT) sequence, a poly(dTNV) sequence) and a functional domain. In some examples, the functional domain is a primer binding site. In some examples, the functional domain is a sequencing specific site (e.g., Read2 site). The target nucleic acid is reverse transcribed and a homopolynucleotide sequence is added to the 3' end of the cDNA. FIG. 34 shows a homopolynucleotide sequence comprising cytosines, however, it is appreciated that other homopolynucleotide sequences can be added to the 3' end of the cDNA. In some examples, the homopolynucleotide sequence is added by the reverse transcriptase. In some examples, the homopolynucleotide sequence is added by a terminal transferase (e.g., terminal deoxynucleotidyl transferase).

A second primer is added where the second primer includes a sequence complementary to the homopolynucleotide sequence added to the 3' end of the cDNA and a capture sequence. In some examples, the second primer includes an RNA sequence (e.g., a ribo-functional sequence such as a linker sequence, a primer binding sequence, a sequence for use in next generation sequencing, etc.). After reverse transcription and extension of the 3' end of the cDNA using the second primer as an extension template, an RNase (e.g., RNase H) is contacted with the biological sample (e.g., a tissue section). The RNase degrades the RNA strand of the RNA/cDNA duplex, leaving a single-stranded cDNA product (e.g., an extension product) that includes the first primer at its 5' end and a capture sequence capable of hybridizing a capture domain of a capture probe.

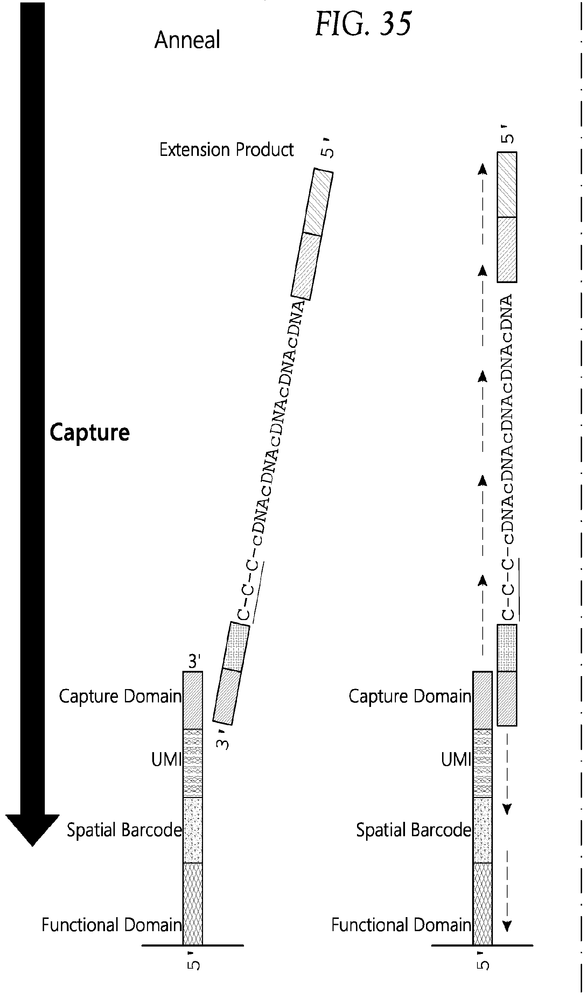

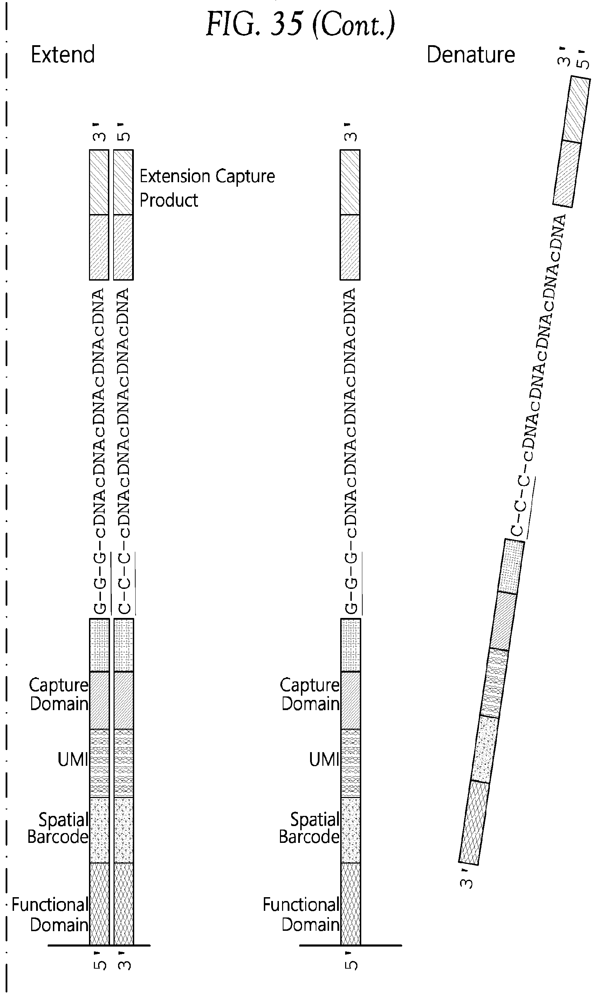

FIG. 35 is a schematic showing capture of the extension product (e.g., the single-stranded cDNA product shown in FIG. 34) by a capture probe on the substrate. The capture probe is attached to the substrate via its 5' end and can include one or more functional domains, a spatial barcode, a unique molecular identifier, and a capture domain. In some examples, the capture probe also includes a cleavage domain. The capture domain hybridizes to the capture sequence on the extension product (e.g., single-stranded cDNA product) from FIG. 34. In some examples, the 3' end of the capture probe is extended using the extension product as a template. In some examples, the 3' end of the extension product (e.g., single-stranded cDNA product) is extended using the capture probe as a template thereby generating an extended capture product. In some examples, the 3' end of the capture probe is extended using the extension product as a template and the 3' end of the extension product is simultaneously extended using the capture probe as a template (e.g., generating an extended capture product). In some examples, the extended capture product is released from the capture probe. In some examples, the extended capture product is released via heat. In some examples, the extended capture product is denatured from the capture probe. In some examples, the extended capture product is denatured from the capture probe with KOH.

The released, extended captured products can be prepared for downstream applications, such as generation of a sequencing library and next-generation sequencing.

### Exemplary Multiplexing Methods

The present disclosure provides methods and systems for multiplexing, and otherwise increasing throughput of samples for analysis. For example, a single or integrated process workflow may permit the processing, identification, and/or analysis of more or multiple analytes, more or multiple types of analytes, and/or more or multiple types of analyte characterizations. For example, in the methods and systems described herein, one or more labeling agents capable of binding to or otherwise coupling to one or more cells or cell features can be used to characterize cells and/or cell features. Cell features may include cell surface features. Cell features can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, an ABM, an antibody, a T-cell receptor, a B-cell receptor, a chimeric antigen receptor, a gap junction, an adherens junction, or any combination thereof. Cell features can include intracellular analytes, such as proteins, protein modifications (e.g., phosphorylation status or other post-translational modifications), nuclear proteins, nuclear membrane proteins, or any combination thereof. A cell feature can comprise an ABM, e.g., an antibody such as a secreted antibody, an immune receptor (e.g., a TCR, a BCR, or an FcR). A labeling agent can include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, an antigen (e.g., an AbTx or ADC), a monobody, an affimer, a Darpin, and a protein scaffold, or any combination thereof. The labeling agent may include an antigen such as an AbTx or ADC. The AbTx or ADC may be bound to its antigenic target (AgTx).

The labeling agents can include (e.g., be attached to or conjugated with) a reporter oligonucleotide that is indicative of the cell feature to which the labeling agent binds. For example, the reporter oligonucleotide can include a barcode sequence that permits identification of the labeling agent. For example, a labeling agent that is specific to one type of cell feature (e.g., a first cell feature) can have a first reporter oligonucleotide comprising a first reporter barcode sequence coupled thereto, while a labeling agent that is specific to a different cell feature (e.g., a second cell feature) can have a different reporter oligonucleotide comprising a different reporter barcode sequence coupled thereto. For a description of exemplary labeling agents, reporter oligonucleotides, and methods of use, see, e.g., U.S. Pat. 10,550,429; U.S. Pat. Pub. 20190177800; and U.S. Pat. Pub. 20190367969.

In a particular example, a library of potential cell feature labeling agents can be provided, where the respective cell feature labeling agents are associated with reporter oligonucleotides, such that a different reporter oligonucleotide sequence is associated with each labeling agent capable of binding to a specific cell feature. Different members of the library can be characterized by the presence of a different oligonucleotide sequence label. For example, an antibody capable of binding to a first protein can have associated with it a first reporter oligonucleotide sequence, while an antibody capable of binding to a second protein can have a different reporter oligonucleotide sequence associated with it. For other example, a first antigen that is an AbTx or ADC can have associated with it a first reporter barcode sequence, while a second antigen (that is a different AbTx or ADC) can have associated with it a second reporter barcode sequence. The presence of the particular barcode sequence can be indicative of the presence of a particular cell feature which can be recognized or bound by the particular labeling agent.

Labeling agents capable of binding to or otherwise coupling to one or more cells can be used to characterize a cell as belonging to a particular set of cells. For example, labeling agents can be used to label a sample of cells or a group of cells. In this way, a group of cells can be labeled as different from another group of cells. In an example, a first group of cells can originate from a first sample and a second group of cells can originate from a second sample. Labeling agents can allow the first group and second group to have a different labeling agent (or reporter oligonucleotide associated with the labeling agent). This can, for example, facilitate multiplexing, where cells of the first group and cells of the second group can be labeled separately and then pooled together for downstream analysis. The downstream detection of a label can indicate analytes as belonging to a particular group.

For example, a reporter oligonucleotide can be linked to an antibody or an epitope binding fragment thereof, and labeling a cell can include subjecting the antibody-linked barcode molecule or the epitope binding fragment-linked barcode molecule to conditions suitable for binding the antibody to a molecule present on a surface of the cell. The binding affinity between the antibody or the epitope binding fragment thereof and the molecule present on the surface can be within a desired range to ensure that the antibody or the epitope binding fragment thereof remains bound to the molecule. For example, the binding affinity can be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds can be less than about 100 µM, 90 µM, 80 µM, 70 µM, 60 µM, 50 µM, 40 µM, 30 µM, 20 µM, 10 µM, 9 µM, 8 µM, 7 µM, 6 µM, 5 µM, 4 µM, 3 µM, 2 µM, 1 µM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant can be less than about 10 µM.

In another example, a reporter oligonucleotide can be coupled to a cell-penetrating peptide (CPP), and labeling cells can include delivering the CPP coupled reporter oligonucleotide into an biological particle. Labeling biological particles can include delivering the CPP conjugated oligonucleotide into a cell and/or cell bead by the cell-penetrating peptide. A CPP that can be used in the methods provided herein can include at least one non-functional cysteine residue, which can be either free or derivatized to form a disulfide link with an oligonucleotide that has been modified for such linkage. Non-limiting examples of CPPs include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The CPP can be an arginine-rich peptide transporter. The CPP can be Penetratin or the Tat peptide.

In another example, a reporter oligonucleotide can be coupled to a fluorophore or dye, and labeling cells can include subjecting the fluorophore-linked barcode molecule to conditions suitable for binding the fluorophore to the surface of the cell. Fuorophores can interact strongly with lipid bilayers and labeling cells can include subjecting the fluorophore-linked barcode molecule to conditions such that the fluorophore binds to or is inserted into a membrane of the cell. In some cases, the fluorophore is a water-soluble, organic fluorophore. The fluorophore may be Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649 for a description of organic fluorophores.

A labeling agent, e.g., a reporter oligonucleotide conjugated antigen, can comprise a detectable label, e.g., a fluorophore or dye. Different labeling agents, e.g., different reporter oligonucleotide conjugated antigens, can comprise different detectable labels, e.g., different fluorophores or dyes.

A reporter oligonucleotide can be coupled to a lipophilic molecule, and labeling cells can include delivering the nucleic acid barcode molecule to a membrane of a cell or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and the cell or nuclear membrane can be such that the membrane retains the lipophilic molecule (e.g., and associated components, such as nucleic acid barcode molecules, thereof) during subsequent processing (e.g., partitioning, cell permeabilization, amplification, pooling, etc.). The reporter nucleotide can enter into the intracellular space and/or a cell nucleus. A reporter oligonucleotide coupled to a lipophilic molecule may be remain associated with and/or inserted into lipid membrane (as described herein) via the lipophilic molecule until lysis of the cell occurs, e.g., inside a partition. Examples of lipophilic molecules coupled to reporter oligonucleotides are described in PCT/US2018/064600.

A reporter oligonucleotide can be part of a nucleic acid molecule including any number of functional sequences, as described elsewhere herein, such as a target capture sequence, a random primer sequence, and the like, and coupled to another nucleic acid molecule that is, or is derived from, the analyte.

Prior to attachment of analytes from a tissue sample to capture probes, as disclosed herein, the tissue sample can be contacted with (e.g., incubated with) a library of labeling agents, that can be labeling agents to a broad panel of different cell features and which include their associated reporter oligonucleotides. Unbound labeling agents can be washed from the tissue sample, and the tissue sample can then be subjected to spatial analysis, as described in any one of the spatial analysis methods disclosed herein. As a result, analytes and reporter oligonucleotides from the labeling agents can be associated with spatial barcodes which can provide information on the spatial location of the analytes and labeling agents in the tissue sample.

In other examples, e.g., to facilitate sample multiplexing, an antigen that is specific to a particular cell feature can have a first plurality of the antigen (e.g., an antibody or lipophilic moiety) coupled to a first reporter oligonucleotide with a first reporter barcode and a second plurality of the antigen coupled to a second reporter oligonucleotide with a second reporter barcode. In this way, different samples or groups can be independently processed and subsequently combined together for pooled analysis (e.g., spatial analysis as described elsewhere herein). See, e.g., U.S. Pat. Pub. 20190323088.

These reporter oligonucleotides can include nucleic acid barcode sequences that permit identification of the labeling agent which the reporter oligonucleotide is coupled to. For example, reporter oligonucleotides include nucleic acid barcode sequences that permit identification of the antigen (e.g., AbTx or ADC) which the reporter oligonucleotide is coupled to. The use of oligonucleotides as the reporter can provide advantages of being able to generate significant diversity in terms of sequence, while also being readily attachable to most biomolecules, e.g., antibodies, etc., as well as being readily detected, e.g., using sequencing or array technologies.

Attachment (e.g., coupling, conjugation) of the reporter oligonucleotides to the labeling agents can be achieved through any of a variety of direct or indirect, covalent or non-covalent associations or attachments. For example, oligonucleotides can be covalently attached to a portion of a labeling agent (such a protein, e.g., an antigen, an antibody or antibody fragment) using chemical conjugation techniques (e.g., Lightning-Link^{®} antibody labeling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Two molecules may be "covalently linked" or "covalently attached" to one another when at least one atom in the first molecule shares at least one electron pair with at least one atom in the second molecule. A covalent linkage between two molecules can involve one or more intermediary molecules. For example, a first molecule and a second molecule may be considered covalently linked, if they are each covalently linked to a linker molecule. In such a circumstance, all three molecules (the first molecule, the second molecule, and the linker molecule) are covalently linked to one another.

Antibody and oligonucleotide biotinylation techniques are available. See, e.g., Fang, et al., "Fluoride-Cleavable Biotinylation Phosphoramidite for 5'-end-Labeling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat. No. 6,265,552. Furthermore, click reaction chemistry such as a Methyltetrazine-PEG5-NHS Ester reaction, a TCO-PEG4-NHS Ester reaction, or the like, can be used to couple reporter oligonucleotides to labeling agents. Commercially available kits, such as those from Thunderlink and Abcam, and techniques common in the art can be used to couple reporter oligonucleotides to labeling agents as appropriate. In another example, a labeling agent is indirectly (e.g., via hybridization) coupled to a reporter oligonucleotide including a barcode sequence that identifies the labeling agent. For instance, the labeling agent can be directly coupled (e.g., covalently bound) to a hybridization oligonucleotide that includes a sequence that hybridizes with a sequence of the reporter oligonucleotide. Hybridization of the hybridization oligonucleotide to the reporter oligonucleotide couples the labeling agent to the reporter oligonucleotide. The reporter oligonucleotides may be releasable from the labeling agent, such as upon application of a stimulus. For example, the reporter oligonucleotide can be attached to the labeling agent through a labile bond (e.g., chemically labile, photolabile, thermally labile, etc.) as generally described for releasing molecules from supports elsewhere herein. The reporter oligonucleotides described herein can include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

An antigen (e.g., a therapeutic antibody or antibody drug complex) can be conjugated to a reporter oligonucleotide. The reporter oligonucleotide can be conjugated anywhere along the amino acid chain of the antigen. The reporter oligonucleotide can be conjugated to the N terminus, the C terminus, or between the N terminus and the C terminus of the antigen.

The reporter oligonucleotide conjugated to the antigen may not interfere with binding of the antigen to an antigen binding molecule. A reporter oligonucleotide can be conjugated away from a site on the antigen that binds to the antigen binding molecule (e.g., an epitope). Such as when the binding site is unknown, a reporter oligonucleotide can be conjugated to different parts of the antigen on different copies of the antigen.

Either end (e.g., the 3' end or the 5' end) of the reporter oligonucleotide can be conjugated to the antigen. More than one reporter oligonucleotide can be conjugated to the antigen.

Conjugation of a reporter oligonucleotide to an antigen can preserve the tertiary and/or quaternary structure of the antigen. The structure of the antigen can be completely preserved. The structure of a binding site (e.g., a site where the antigen can bind to an antigen binding molecule such as an antibody) can be preserved. The location and/or orientation of surface residues of the antigen can be preserved.

The link between an antigen and a reporter oligonucleotide can be stable. Stability can be, for example, under physiological conditions (e.g., physiological pH, temperature, etc.), or under conditions of an assay. Such a link can remain stable for at least 1 hour, at least 6 hours, at least 12 hours, at least 1 day, at least 1 week, at least 1 month, at least 1 year, or a range between any two foregoing values.

The affinity between an antigen and antigen binding molecule may not be compromised by the conjugation of a reporter oligonucleotide to the antigen. The presence of the oligonucleotide or the process of conjugating the oligonucleotide to the antigen may not increase or decrease the affinity of the antigen to the antigen binding molecule.

A reporter oligonucleotide can be conjugated to an antigen directly using any suitable chemical moiety on the antigen. A reporter oligonucleotide can be conjugated to an antigen enzymatically, e.g., by ligation. In some cases, a reporter oligonucleotide can be linked indirectly to an antigen, for example via a non-covalent interaction such as a biotin/streptavidin interaction or an equivalent thereof, via an aptamer or secondary antibody, or via a protein-protein interaction such as a leucine-zipper tag interaction or the like.

A reporter oligonucleotide can be conjugated to an antigen using click chemistry, or a similar method. Click chemistry can refer to a class of biocompatible small molecule reactions that can allow the joining of molecules, such as a reporter oligonucleotide and an antigen. A click reaction can be a one pot reaction, and in some cases is not disturbed by water. A click reaction can generate minimal byproducts, non-harmful byproducts, or no byproducts. A click reaction can be driven by a large thermodynamic force. In some cases, a click reaction can be driven quickly and/or irreversibly to a high yield of a single reaction product (e.g., a reporter oligonucleotide conjugated to an antigen), and can have high reaction specificity. Click reactions can include but are not limited to [3+2] cycloadditions, thiol-ene reactions, Diels-Alder reactions, inverse electron demand Diels-Alder reactions, [4+1] cycloadditions, nucleophilic substitutions, carbonyl-chemistry-like formation of ureas, or addition reactions to carbon-carbon double bonds (e.g., dihydroxylation).

An antigen can be conjugated to a reporter oligonucleotide by a redox activated chemical tagging (ReACT) reaction. A react reaction can be a chemoselective methionine-bioconjugation that can employ redox reactivity. For example, oxaziridine-based reagents can enable highly selective, rapid, and robust conjugation. Further description of ReACT chemistry can be found, for example, in (Makishma, Akio. Biochemistry for Materials Science. Elsevier, 2019).

An antigen can be conjugated to a reporter oligonucleotide by a site-specific sortase motif-dependent conjugation. Site-specific sortase motif-dependent conjugation can be a highly specific platform for conjugation that can rely on the specificity of Sortase A for short peptide sequences (e.g., LPXTG AND GGG).

Sortase A can be a transpeptidase that can be adopted for site-specific protein modification. A reaction catalyzed by Sortase A can result in the formation of an amide bond between a C terminal sorting motif (e.g., LPXTG, where X can be any amino acid) and an N terminal oligoglycine. Such a conjugation reaction can proceed by first cleaving the peptide bond between the threonine and glycine residues with the sorting motif of Sortase A. Sortase A can be used to conjugate an oligonucleotide to either an N terminus or a C terminus of an antigen. Sortase A can retain its specificity while accepting a wide range of potential substrates.

An antigen can be conjugated to a reporter oligonucleotide by a site-specific photo-crosslinking-dependent conjugation. For example, such photo-crosslinking dependent conjugation can utilize unnatural amino acids or chemical crosslinking. Such photo-crosslinking can be mediated or directed by a peptide in some cases. For example, a peptide or other photosensitive molecule on the antigen can form a covalent bond with a molecule on the oligonucleotide upon activation by a specified wavelength of light. A peptide or other photosensitive molecule on the reporter oligonucleotide can form a covalent bond with a residue on the antigen upon activation by a specified wavelength of light.

An antigen can be conjugated to a reporter oligonucleotide by site-specific conformation-dependent conjugation (e.g., glycan-dependent Fc conjugation or GlyCLICK). Such conjugation can generate a reporter oligonucleotide conjugated antigen. For example, deglycosylation of the antigen can allow for site specific conjugation using click chemistry techniques. An antigen can be conjugated to a reporter oligonucleotide by nitrilotriacetate conjugation

An oligonucleotide can be conjugated to a constant region of an antigen. For example, an oligonucleotide can be conjugated to a constant region of a heavy chain or a constant region of a light chain of an antigen that is an antibody or antigen binding fragment thereof.

An oligonucleotide can be conjugated to a variable region of an antigen. For example, an oligonucleotide can be conjugated to a variable region of a heavy chain or a variable region of a light chain of an antigen that is an antibody or antigen binding fragment thereof.

The reporter oligonucleotide conjugated antigen can include one or more detectable tags. For example, the reporter oligonucleotide conjugated antigen can include a fluorophore, metal ion, or other detectable tag. The detectable tag can be conjugated to the reporter oligonucleotide, the antigen, or both.

In some cases, the labeling agent can include a reporter oligonucleotide and a detectable tag. A detectable tag can be fluorophore, a radioisotope, a molecule capable of a colorimetric reaction, a magnetic particle, or any other suitable molecule or compound capable of detection. The detectable tag can be conjugated to a labeling agent (or reporter oligonucleotide) either directly or indirectly (e.g., the label can be conjugated to a molecule that can bind to the labeling agent or reporter oligonucleotide). In some cases, a detectable tag is conjugated to an oligonucleotide that is complementary to a sequence of the reporter oligonucleotide, and the oligonucleotide can be allowed to hybridize to the reporter oligonucleotide.

In some cases, a reporter oligonucleotide can be attached (e.g., covalently linked such as conjugated, e.g., directly or indirectly via a linker, or non-covalently bound through a binding interaction) to an antibody (e.g., an AbTx or ADC) via an antibody-binding protein. For example, a reporter oligonucleotide and an antibody-binding protein can form a complex. The complex can bind to a respective antibody through the antibody-binding protein.

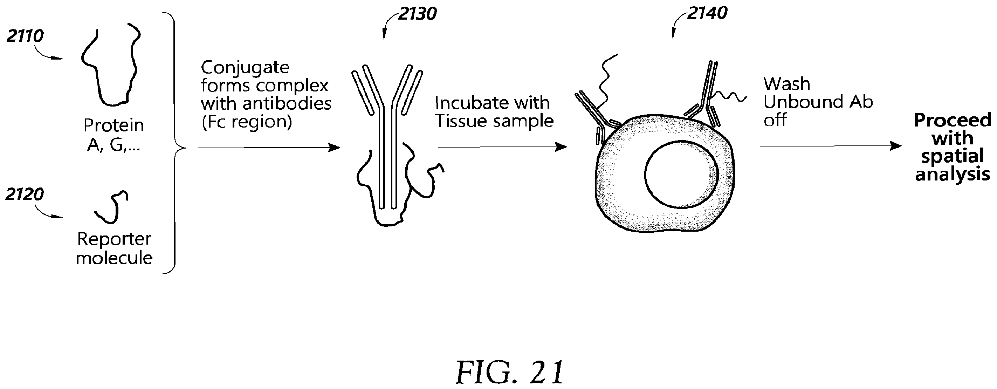

FIG. 21 shows an example workflow for associating a reporter molecule on an antibody (e.g., an ADA, and ADC, or an AbTx) using an antibody-binding protein. An antibody binding protein 2110, e.g., Protein A or Protein G, and a reporter molecule 2120 are conjugated to the Fc region of an antibody, forming a complex 2130 comprising the antibody, the antibody-binding protein 2110, and the reporter molecule 2120. The complex 2130 is incubated with a tissue sample, e.g., a tissue sample comprising one or more immune receptor expressing cells, and unbound antibody can be washed out. The complex 2130 can binds to a cell, e.g., an immune receptor expressing cell of the tissue sample, thereby providing a labelled immune receptor expressing cell. The tissue sample can be subjected to spatial analysis according to a method disclosed herein.

An antibody-binding protein can have fast adsorption kinetics, slow desorption kinetics, and/or a low binding equilibrium constant. Any methods for adding chemical functionality to peptides or protein can be used. Some methods can include attaching a reporter molecule to specific amino acids or chemical groups (e.g., chemical groups present in multiple types of protein) on the antibody-binding protein. The conjugation of antibody-binding proteins and reporter molecules can be performed using any known methods for forming antibody-nucleic acid conjugation, e.g., using click chemistry. Dissociation of the antibody-binding protein/reporter molecule complexes can be prevented by crosslinking (e.g., using a crosslinker such as formaldehyde), protein engineering, or adding the protein-binding proteins in excess.

Examples of antibody-binding proteins can include proteins that bind to the constant (Fc) region of antibodies, such as Protein A, Protein G, protein L, or fragments thereof. Other binding proteins (e.g., streptavidin) can be expressed as fusion proteins with antibody-binding proteins, and used to associate reporter molecules (e.g., by binding of biotinylated reporter molecules to a streptavidin-protein A fusion protein). Other antibody-binding proteins or domains can provide additional binding affinity for various antibody classes. In some cases, the antibody-binding protein can be an antibody, e.g., a secondary antibody for the antibody targeting the sample. The secondary antibody can include a reporter molecule described herein, e.g., an oligonucleotide with a reporter barcode and a poly-A or poly-T terminated sequence.

The antibody-binding proteins can be engineered to introduce additional functionalities. Antibody-binding proteins can be engineered to contain amino acids with functional groups amenable to conjugation with oligonucleotides (e.g., reporter molecules). For example, the antibody-binding proteins can naturally have or be engineered to have cysteine residues, e.g., for controlling stoichiometry and/or attachment location of the reporter molecules. The antibody-binding proteins can be engineered to have non-natural amino acid residues, e.g., for targeted crosslinking of binding proteins and antibodies. The antibody-binding proteins can be engineered to have tags, e.g., fluorescent tags (e.g., by fusing with a fluorescent protein such as green fluorescent protein (GFP), red fluorescence protein (RFP), yellow fluorescent protein (YFP)), and/or affinity tags for purification and visualization. The fluorescent tags and/or the affinity tags can be cleavable. In some cases, the antibody-binding protein can be engineered to have one or more barcode attachment sites per protein.

Also provided herein are kits comprising antibody-binding proteins conjugated with reporter molecules in e.g., well plates. Antibody for an assay can be incubated with the antibody-binding proteins conjugated with reporter molecules at a specified concentration without interfering with the antibody's binding site and/or without the need for any chemistry to be carried out in one's hands to conjugate the reporter molecule to the antibody.

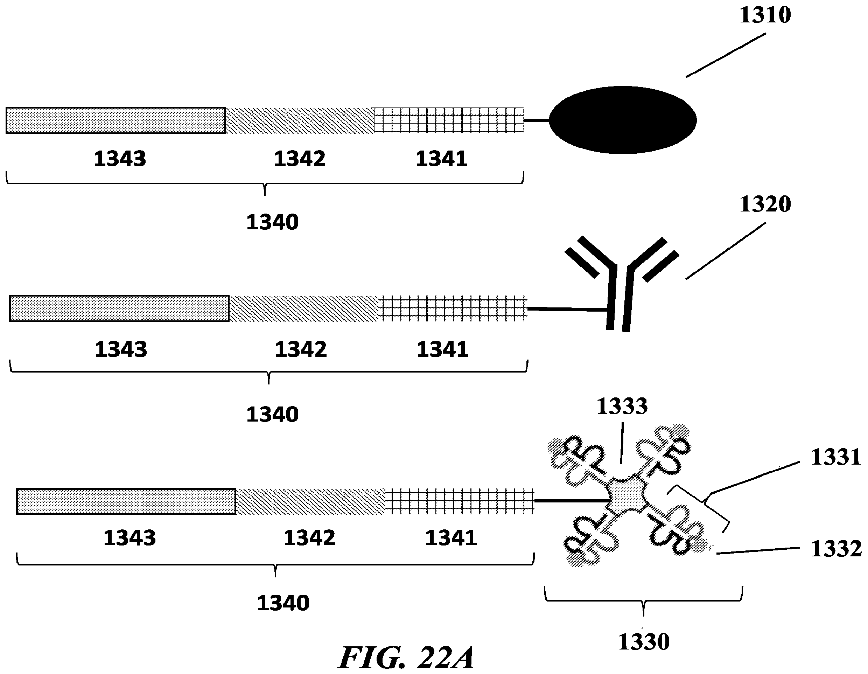

FIG. 22A describes exemplary antigens (1310, 1320, 1330) conjugated to a reporter oligonucleotide (1340) attached thereto. The antigen 1310, 1320, or 1330 is attached (either directly, e.g., covalently attached, or indirectly) to a reporter oligonucleotide 1340. A reporter oligonucleotide 1340 can contain a reporter barcode sequence 1342 that identifies the antigen 1310, 1320, or 1330. A reporter oligonucleotide 1340 can also contain one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to FIG. 22A, reporter oligonucleotide 1340 conjugated to an antigen (e.g., 1310, 1320, 1330) can include a functional sequence 1341 (e.g., an adaptor), a barcode sequence that identifies the antigen (e.g., 1310, 1320, 1330), and functional sequence (e.g., adaptor) 1343. Capture handle 1343 can be configured to hybridize to a complementary sequence (e.g., a capture sequence), such as a complementary sequence (e.g., capture sequence) of a capture domain of a capture probe, such as those described elsewhere herein. A capture handle can include a sequence that is complementary to a capture sequence of a capture domain of a capture probe. A capture probe may be attached to a substrate (e.g., substrate feature), such as those described elsewhere herein. A reporter oligonucleotide 1340 may include one or more additional functional sequences, such as those described above.

Referring to FIG. 22A, antigen 1310 may be a protein or polypeptide (e.g., an antigen or prospective antigen) conjugated to reporter oligonucleotide 1340. Reporter oligonucleotide 1340 contains reporter barcode sequence 1342 that identifies protein or polypeptide 1310 and can be used to infer the presence of, e.g., a binding partner of protein or polypeptide 1310 (i.e., a molecule or compound to which the protein or polypeptide binds). 1310 may be a lipophilic moiety (e.g., cholesterol) comprising reporter oligonucleotide 1340, where the lipophilic moiety is selected such that 1310 integrates into a membrane of a cell or nucleus. Reporter oligonucleotide 1340 contains reporter barcode sequence 1342 that identifies lipophilic moiety 1310 which may be used to tag cells (e.g., groups of cells, cell samples, etc.) for multiplex analyses as described elsewhere herein.

Referring to FIG. 22A, the antigen is an antibody (e.g., AbTx or ADC) 1320 (or an epitope binding fragment thereof) including reporter oligonucleotide 1340. Reporter oligonucleotide 1340 includes reporter barcode sequence 1342 that identifies antibody 1320 and can be used to infer the presence of, e.g., a target of antibody 1320 (e.g., a molecule or compound to which antibody 1320 binds, such as an ABM that binds (e.g., specifically binds) the antibody 1320).

Referring to FIG. 22A, antigen 1330 may include an MHC molecule 1331 including peptide 1332 and oligonucleotide 1340 that identifies peptide 1332. The MHC molecule may be coupled to a support 1333. Support 1333 may be streptavidin (e.g., MHC molecule 1331 can include biotin). Support 1333 may be a polysaccharide, such as dextran. Reporter oligonucleotide 1340 can be directly or indirectly coupled to MHC labeling agent 1330 in any suitable manner, such as to MHC molecule 1331, support 1333, or peptide 1332. Labeling agent 1330 may include a plurality of MHC molecules, i.e., is an MHC multimer, which can be coupled to a support (e.g., 1133). There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5^{®} MHC Class I Pentamers, (ProImmune, Ltd.), MHC octamers, MHC dodecamers, MHC decorated dextran molecules (e.g., MHC Dextramer^{®} (Immudex)), etc. For a description of exemplary labeling of various antigens, including antibody and MHC-based labeling agents, reporter oligonucleotides, and methods of use, see, e.g., U.S. Pat. 10,550,429 and U.S. Pat. Pub. 20190367969.

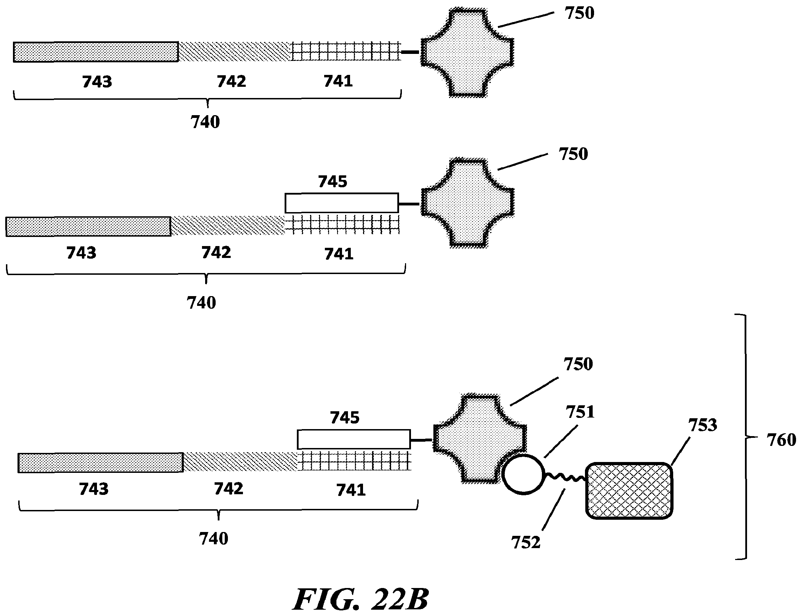

Referring to FIG. 22B, reporter oligonucleotide 740 may be conjugated to a support 750 that can be used to complex with or bind to an antigen (e.g., an antigen of interest or a non-target antigen). Reporter oligonucleotide 740 includes a functional sequence 741, a reporter barcode sequence 742 that identifies the antigen of interest, and reporter capture handle 743. Reporter capture handle sequence 743 can be configured to hybridize to a complementary sequence, such as a complementary sequence present on a capture domain of a capture probe, such as those described elsewhere herein (e.g., FIGS. 2-4). The capture probe may be attached to a substrate (e.g., a substrate feature), such as those described elsewhere herein. For example, capture probe can be attached to the support via a releasable linkage (e.g., including a labile bond), such as those described elsewhere herein. The reporter oligonucleotide 740 may include one or more additional functional sequences, such as those described above. Support 750 may comprise an anchor sequence 745 that is complementary to functional sequence 741. The reporter oligonucleotide 740 may be attached to support 750 via hybridization to anchor sequence 745. The anchor sequence 745 may further comprise (or may be) a functional sequence (similar to or equivalent to functional sequence 741) as described herein. The anchor sequence 745 may not comprise a functional sequence. Reporter oligonucleotide 740 may include a functional sequence (not shown). A support 750 may comprise a binding region that can be used to complex with (or bind to) an antigen of interest. The antigen of interest may comprise a ligand that can be bound by the binding region of support 750.

Referring to FIG. 22B again, labelling agent 760 comprises a support 750 that includes an antigen of interest 753 (e.g., an AbTx or ADC) and reporter oligonucleotide 740 that identifies the antigen 753 (e.g., via reporter barcode sequence 742). The support 750 may be coupled to, complexed with, or bound to a ligand 751. Support 750 can be a polypeptide. The polypeptide can be streptavidin. The polypeptide can be avidin. Support 750 can be a polysaccharide. The polysaccharide can be dextran. The polysaccharide can be a dextran. The ligand 751 can be a molecule with affinity for the binding region of the support 750. For example, the ligand 751 may be biotin and the support 750 may be a streptavidin support. The ligand 751 may be coupled to or conjugated to antigen 753 via a linker 752. Accordingly, the tissue sample may be contacted with one or more biotinylated antigens. The antigens can include Avitag biotinylation site and/or a His tag. Protein biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, and U.S. Pat. No. 6,265,552. Reporter oligonucleotide 740 can be directly or indirectly coupled to labelling agent 760 in any suitable manner. For example, reporter oligonucleotide 740 can be coupled to the antigen 753, support 750, anchor sequence 745, or ligand 751.

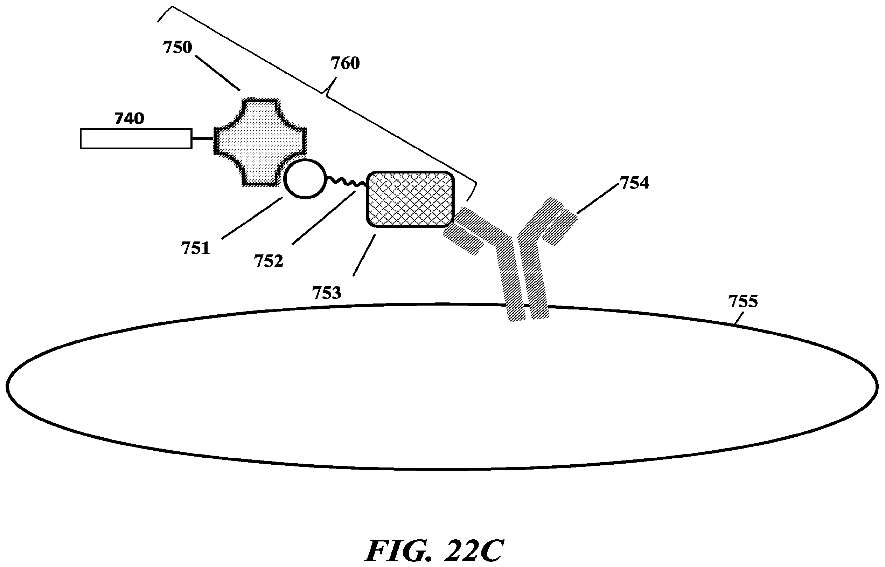

Referring to FIG. 22C, a labelled cell 755 comprising an ABM of interest 754 is depicted. The labelling agent 760 can be contacted with a tissue sample comprising one or more cells expressing an antigen-binding molecule (ABM). In one example, an ABM 754 is bound by or labeled with the labelling agent 760 via an interaction between the ABM 754 and the antigen 753. Further processing of the labelled cell 755 can be performed according to one or more methods and systems for spatial analysis as further described herein.

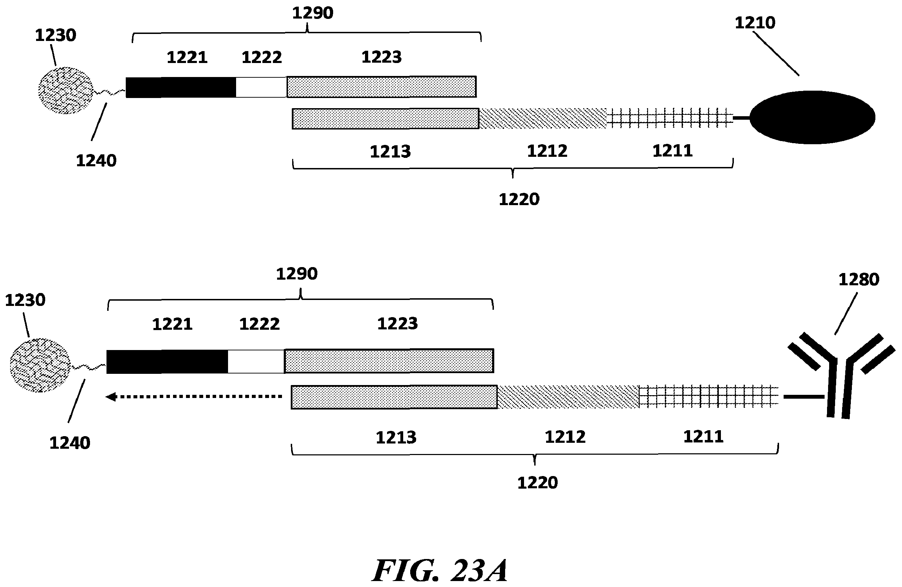

Analysis of multiple analytes (e.g., nucleic acids and one or more analytes using labeling agents described herein) can be performed. For example, the workflow can include a workflow as generally depicted in any of FIGS. 23A-23D, or a combination of workflows for an individual analyte, as described elsewhere herein. For example, by using a combination of the workflows as generally depicted in FIGS. 23A-23D, multiple analytes can be analyzed.

Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969.

### Oligonucleotides

An oligonucleotide can be a molecule which can be a chain of nucleotides. Oligonucleotides described herein can include ribonucleic acids. Oligonucleotides described herein can include deoxyribonucleic acids. In some cases, oligonucleotides can be of any sequence, including a user-specified sequence.

An oligonucleotide can include a sequence that can be used to isolate or identify the antigen. For example, an oligonucleotide can include a barcode sequence (e.g., a reporter barcode and/or a spatial barcode). Different reporter oligonucleotides (e.g., reporter oligonucleotides associated with different antigens) can contain different reporter barcode sequences. A reporter barcode sequence or other feature of an reporter oligonucleotide can be configured to be utilized to identify or isolate the reporter oligonucleotide and/or an antigen associated with the reporter oligonucleotide. For example, identification or isolation can be achieved through base pairing, amplification, sequencing, library creation, imaging (e.g., fluorescent imaging of a label conjugated to the oligonucleotide, for example via the barcode), or other methods.

An oligonucleotide can include G, A, T, U, C, or bases that are capable of base pairing reliably with a complementary nucleotide. 7-deaza-adenine, 7-deaza-guanine, adenine, guanine, cytosine, thymine, uracil, 2-deaza-2-thio-guanosine, 2-thio-7-deaza-guanosine, 2-thio- adenine, 2-thio- 7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7- deaza purine, 5- methyl-cytosine, 5-propynyl-uridine, 5-propynyl-cytidine, 2-thio-thymine or 2-thio-uridine are examples of such bases, although many others are known. An oligonucleotide can include an LNA, a PNA, a UNA, or an morpholino oligomer, for example. The oligonucleotides used herein can contain natural or non- natural nucleotides or linkages.

An oligonucleotide can be at least 5, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, or at least 100 nucleotides long. In some cases, an oligonucleotide can be between 10-30, between 10-50, between 10-70, between 10-100, between 20-50, between 20-70, between 20-100, between 30-50, between 30-70, between 30-100, between 40-70, between 40-100, between 50-70, between 50-100, between 60-70, between 60-80, between 60-90, or between 60-100 nucleotides in length. In some cases, an oligonucleotide can be no more than 5, no more than 10, no more than 15, no more than 20, no more than 25, no more than 30, no more than 35, no more than 40, no more than 45, no more than 50, no more than 55, no more than 60, no more than 65, no more than 70, no more than 75, no more than 80, no more than 85, no more than 90, no more than 95, or no more than 100 nucleotides long.

In some cases, an oligonucleotide can be wholly single stranded. In some cases, an oligonucleotide can be partially double stranded. A partially double stranded region can be at the 3' end of the oligonucleotide, at the 5' end of the oligonucleotide, or between the 5' end and 3' end of the oligonucleotide. In some cases, there can be more than one double stranded region.

In some cases, an oligonucleotide can have a secondary structure. In some cases, an oligonucleotide can have a tertiary structure. Some oligonucleotides can have a structure such that it can fold on itself (e.g. if one region of the oligonucleotide is complementary to another region of the oligonucleotide) to produce one or more double stranded regions comprising a single strand.

In some cases, a segment of an oligonucleotide able to bind a circular nucleic acid primer can be exposed in a single stranded region of the oligonucleotide or an unfolded region of the oligonucleotide. In some cases, a segment of an oligonucleotide able to bind a circular nucleic acid primer can be in a double stranded or folded region of the oligonucleotide, such that upon melting of the oligonucleotide, such a circular nucleic acid primer can bind.

An oligonucleotide can include any nucleic acid based feature provided herein.

### Contacting with Reporter Oligonucleotide Conjugated Antigens

Contacting a tissue sample containing at least one antigen binding molecule with a reporter oligonucleotide conjugated antigen can include contacting the tissue sample with the antigen (such as a therapeutic antibody or antibody-drug conjugate) such that the antigen can interact with the antigen binding molecule. Upon contacting, the antigen can bind to the antigen binding molecule.

Contacting can occur in a vessel, such as a well, a tube, a bead, a partition, a microfluidic system, or in another vessel. Contacting can occur on a membrane or a column. Contacting can occur on a surface or support, such as on a slide, a plate, or a dish.

Contacting can include exposing the at least one antigen binding molecule to the antigen under conditions that can allow binding if the antigen binding molecule has affinity for the antigen. Contacting can occur under physiological conditions, or under conditions that approximate physiological conditions.

Contacting can be performed under conditions that do not significantly alter the structure and/or function of the antigen or the antigen binding molecule. Contacting can be performed under conditions that do not significantly alter (e.g., increase or decrease) the affinity of the antigen binding molecule for the antigen. Conditions can include a pH, a temperature, or other conditions.

Contacting can occur in a buffer having a pHFor example, contacting can occur in a buffer having a pH of about 4.5, about 5.0, about 5.5, about 6.0, about 6.5, about 7.0, about 7.5, about 8.0, about 8.5, about 9.0, or a range between any two foregoing values.

Contacting can occur at a temperature that can allow protein-protein interactions. Contacting can occur at a temperature of about 10°C, about 15°C, about 20°C, about 25°C, about 30°C, about 35°C, about 40°C, about 45°C, about 50°C, or a range between any two foregoing values.

A method can include contacting the tissue sample comprising at least one antigen binding molecule with a plurality of antigens, wherein the antigens can be conjugated to reporter oligonucleotides. In some such cases, an antigen of the plurality of antigens can be conjugated to a reporter oligonucleotide that is different than a reporter second oligonucleotide that is conjugated to a second antigen. One or more such antigens can bind one or more antigen binding molecules in the tissue sample. One antigen can bind an antigen binding molecule in the tissue sample more strongly than a second antigen.

A method can include contacting the tissue sample including at least one antigen binding molecule with a plurality of variants of an antigen, wherein the variants of antigens can be conjugated to reporter oligonucleotides. In some such cases, a variant of the plurality of variants of an antigen can be conjugated to a reporter oligonucleotide that is different than a second reporter oligonucleotide that is conjugated to a second variant of the antigen. One or more such variants of an antigen can bind an antigen binding molecule in the composition. One variant of an antigen can bind an antigen binding molecule more strongly than a second variant of the antigen.

Variants of an antigen can include, for example, an amino acid mutation (e.g., an insertion, deletion, or point mutation) or differences in glycosylation or other modification to an amino acid. A variant of an antigen can include a non-natural amino acid. A variant can, for example, be a variant of a therapeutic (e.g., an antibody or antibody-based drug) that is modified. A modified therapeutic can be modified to reduce recognition of the therapeutic by the immune system of a subject. A modified therapeutic can be modified to reduce binding of the therapeutic to an antigen binding molecule, such as an antibody or antigen binding fragment thereof.

### Exemplary barcoding methods

Antigen binding molecules can be analyzed based at least in part on reporter oligonucleotides conjugated to antigens. Such analysis can allow for identification and/or characterization of an antigen binding molecule that has affinity for the antigen.

Analyzing an antigen binding molecule bound to an antigen (such as a BCR of a B cell or an anti-drug antibody (ADA) bound to a therapeutic antibody or antibody-drug conjugate) can include attaching (e.g., hybridizing or ligating) a reporter oligonucleotide of a reporter oligonucleotide conjugated antigen to a capture probe comprising a capture domain and a spatial barcode. Exemplary capture probes are described further herein.

Isolating an antigen binding molecule can include pulling down at least one antigen binding molecule using its unique sequence. The at least one antigen binding molecule can be pulled down without using the capture probe. Isolating an antigen binding molecule can include pulling down the at least one antigen binding molecule using the capture probe. The capture probe can be attached to a bead, such as those described elsewhere herein. The capture probe can be affixed to a slide, affixed to a well, affixed to a tube, or conjugated to a magnetic molecule.

An antigen binding molecule can be isolated using a pull-down assay. For example, the oligonucleotide can be hybridized to a capture probe that is conjugated to a protein or a magnetic particle. Upon hybridization, the protein or magnetic particle can be used to separate the antigen binding molecule from other components of the composition, for example by contacting the protein or magnetic particle with a binding partner to the protein or a magnetic field, respectively, and washing away other components of the composition. For example, when an antigen binding molecule is on a cell surface, the cell can be lysed or permeabilized prior to performing such a pull-down assay.

For example when an antigen binding molecule is on a cell surface, the cell can be lysed or permeabilized, or the binding molecule can otherwise be removed from the cell. Such lysis, permeabilization, or removal can occur before or after the antigen binding molecule is isolated using the capture probe. An antigen binding molecule can be isolated from a composition with the cell (i.e., the cell can remain intact).

Analysis of one or more antigen binding molecules (e.g., using the oligonucleotide labeled antigens described herein) may include a workflow as generally depicted in FIGS. 23A-23D. For example, a tissue sample, e.g., a tissue sample comprising one or more immune receptor expressing cells, may be contacted with one or more labeling agents, e.g., one or more reporter oligonucleotide 1220 conjugated antigens 1210 (e.g., polypeptide, antibody (1280), antibody therapeutic, antibody drug conjugate, or pMHC molecule or complex) and optionally further processed prior to barcoding. The contacting may provide a labeled cell, e.g., a labeled immune receptor expressing cell. Optional processing steps can include one or more washing steps. A labelled cell may be coupled to a capture probe 1290 that is attached to a substrate 1230, e.g., a substrate comprising an array of capture probes, or a feature of the substrate. Capture probe 1290 may be attached to substrate or feature 1230 via a releasable linkage 1240 (e.g., comprising a labile bond) as described elsewhere herein.

With continued reference to FIG. 23A, oligonucleotide 1220 conjugated to antigen 1210 (e.g., polypeptide, an antibody (1280), pMHC molecule such as an MHC multimer, etc.) may include a functional sequence 1211 (e.g., an adaptor sequence), a reporter barcode sequence 1212 that identifies the antigen 1210 (e.g., the polypeptide, antibody (1280), AbTx, or ADC, or peptide of a pMHC molecule or complex), and capture handle sequence 1213. Capture handle sequence 1213 can be configured to hybridize to a capture domain comprising a capture sequence, such as capture sequence 1223 present capture probe 1290. A capture handle can include a sequence that is complementary to a capture sequence on a capture probe. A capture probe 1290 can include a capture sequence 1223, a spatial barcode (e.g., a spatial barcode sequence 1222), and a functional sequence 1221. Reporter oligonucleotide 1220 may include one or more additional functional sequences, such as those described above.

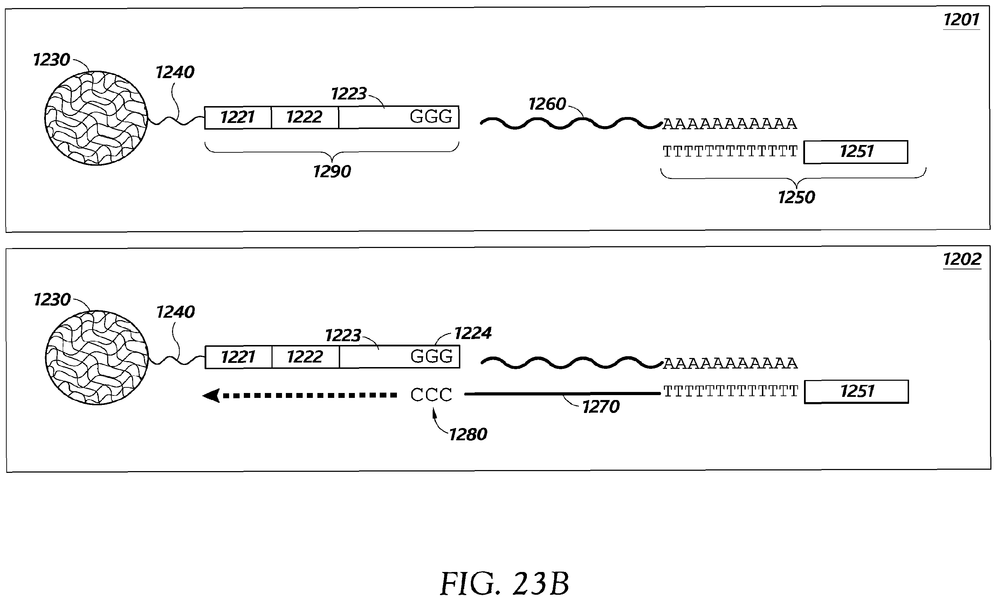

Referring to FIGS. 23B-C, nucleic acid molecules derived from a cell (such as RNA molecules) can be similarly processed to append the spatial barcode sequence 1222 to these molecules or derivatives thereof (e.g., cDNA molecules). For example, referring to FIG. 23B, primer 1250 may include a sequence complementary to a sequence of RNA molecule 1260 (such as an RNA encoding for a BCR sequence) from a cell. Primer 1250 may include one or more adapter sequences 1251 that are not complementary to RNA molecule 1260. Primer 1250 may include a poly-T sequence. Primer 1250 may include a sequence complementary to a target sequence in an RNA molecule. Primer 1250 may include a sequence complementary to a region of an immune molecule, such as the constant region of a TCR or BCR sequence. Primer 1250 is hybridized to RNA molecule 1260 and cDNA molecule 1270 is generated in a reverse transcription reaction. The reverse transcriptase enzyme may be selected such that several non-templated bases 1280 (e.g., a poly-C sequence) are appended to the cDNA. Capture probe 1290 includes a sequence 1224 complementary to the non-templated bases, and the reverse transcriptase performs a template switching reaction onto capture probe 1290 to generate a nucleic acid molecule including spatial barcode sequence 1222 (or a reverse complement thereof) and a sequence of cDNA 1270 (or a portion thereof).

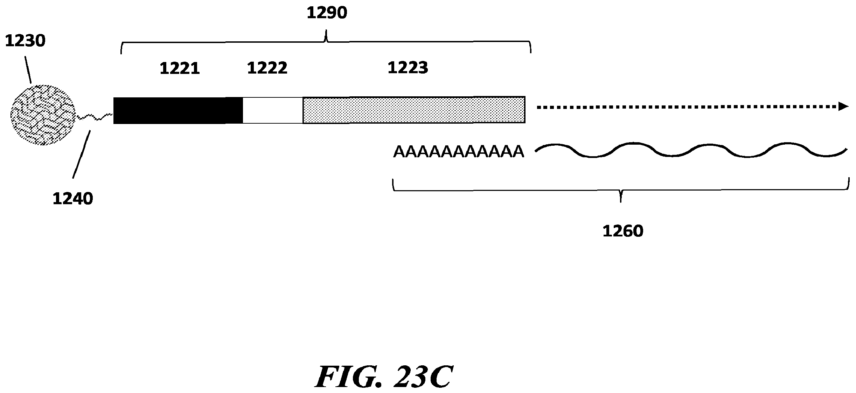

In another example, referring to FIG. 23C, capture probe 1290 may include capture sequence 1223 complementary to a sequence of RNA molecule 1260 from a cell. Capture sequence 1223 may include a sequence specific for an RNA molecule. Capture sequence 1223 may include a poly-T sequence. Capture sequence 1223 may include a sequence specific for an RNA molecule. Capture sequence 1223 may include a sequence complementary to a region of an immune molecule, such as the constant region of a TCR or BCR sequence. Capture sequence 1223 can be hybridized to RNA molecule 1260 and a cDNA molecule 1270 (FIG. 23B) is generated in a reverse transcription reaction, generating a spatially barcoded polynucleotide including spatial barcode sequence 1222 (or a reverse complement thereof) and a sequence of cDNA 1270 (FIG. 23B) (or a portion thereof). The spatially barcoded polynucleotide including spatial barcode 1222 (or reverse complement thereof) can then be optionally processed as described elsewhere herein, e.g., to enrich for target sequences of interest, to amplify the molecules and/or append sequencing platform specific sequences to the fragments. See, e.g., U.S. Pat. Pub. 20180105808. The spatially barcoded polynucleotides, or derivatives or library members generated therefrom, can then be sequenced on a suitable sequencing platform.

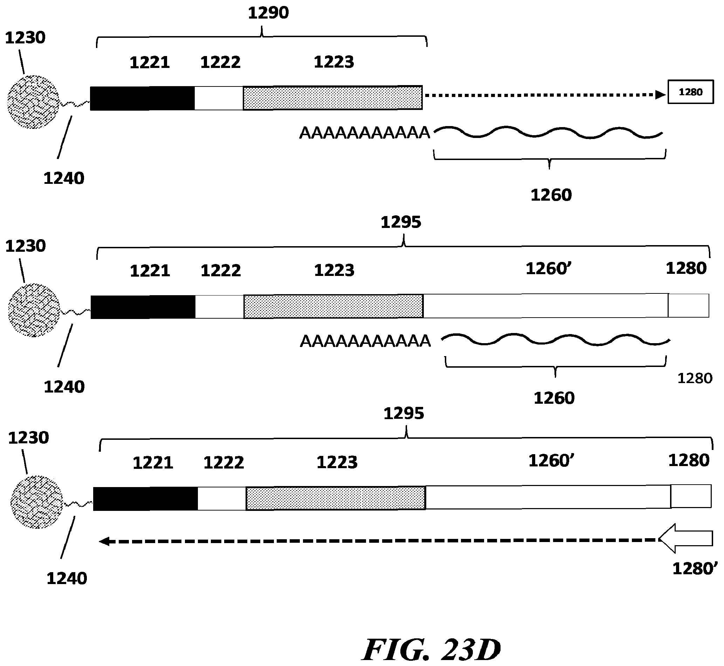

In another example, referring to FIG. 23D, capture probe 1290 may include capture sequence 1223 complementary to a sequence of RNA molecule 1260 from a cell. Capture sequence 1223 may include a sequence specific for an RNA molecule. Capture sequence 1223 may include a poly-T sequence. Capture sequence 1223 may include a sequence specific for an RNA molecule. Capture sequence 1223 may include a sequence complementary to a region of an immune molecule, such as the constant region of a TCR or BCR sequence. Capture sequence 1223 can be hybridized to RNA molecule 1260 and extended, e.g., via a reverse transcriptase. The reverse transcriptase may add template switching sequence 1280, generating a spatially barcoded polynucleotide 1295 comprising 1221, 1222, 1223, 1260' cDNA sequence, template switching sequence 1280. Template switching sequence 1280 may comprise non-templated bases (e.g., polyC) added to the end of the 1260' cDNA sequence. Spatially barcoded polynucleotide 1295 may be subjected to second strand synthesis using a primer 1280' comprising a sequence complementary to template switching sequence 1280, thereby generating a double-stranded spatially barcoded polynucleotide. The double-stranded spatially barcoded polynucleotide can be optionally processed as described herein, e.g., to enrich for target sequences of interest, to amplify the molecules and/or append sequencing platform specific sequences to the fragments. See, e.g., U.S. Pat. Pub. 20180105808. The spatially barcoded polynucleotides, or derivatives or library members generated therefrom, can then be sequenced on a suitable sequencing platform.

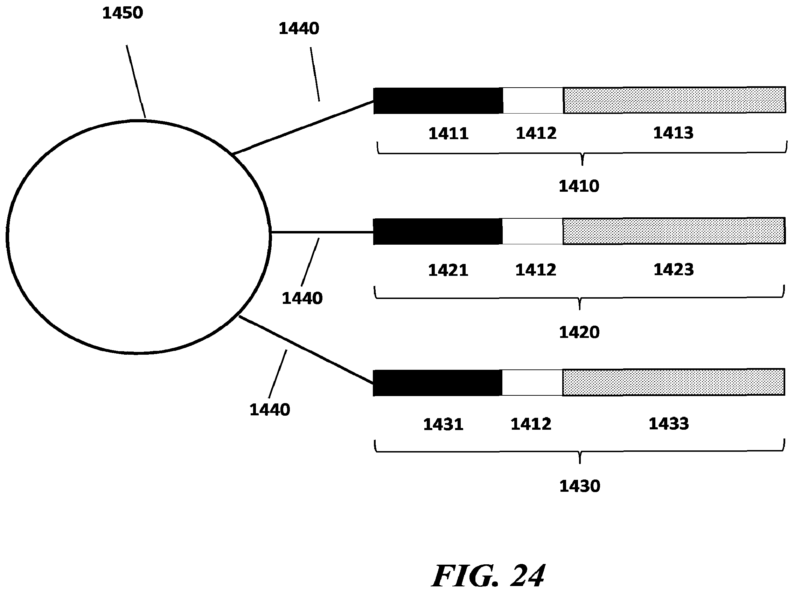

Exemplary capture probes attached to a substrate (e.g., a substrate feature) is shown in FIG. 24. Capture probe 1410 can include functional sequence 1411, spatial barcode 1412 and capture sequence 1413. Capture probe 1420 can include functional sequence 1421, spatial barcode 1412, and capture sequence 1423, wherein capture sequence 1423 includes a different sequence than capture sequence 1413. Functional sequence 1411 and functional sequence 1421 may include the same sequence. Functional sequence 1411 and functional sequence 1421 may include different sequences. Although substrate (e.g., substrate feature) 1450 is shown including capture probe 1410 and 1420, any suitable number of capture probes including spatial barcode 1412 are contemplated herein. For example, substrate or substrate feature 1450 may further include capture probe 1430. Capture probe 1430 can include functional sequence 1431, spatial barcode 1412 and capture sequence 1433, wherein capture sequence 1433 includes a different sequence than capture sequence 1413 and 1423. Capture probes (e.g., 1410, 1420, 1430) may include one or more additional functional sequences, such as a UMI or other sequences described herein. Any one or more of capture probes 1410, 1420, and 1430 may be attached to substrate or substrate feature 1450 via a linker 1440.

### Identifying Antigen Binding Molecules

Identifying an antigen binding molecule can include determining the identity of the antigen binding molecule. An antigen binding molecule can be determined by comparing the antigen binding molecule to a library of antigen binding molecules, by sequencing the antigen binding molecule, using imaging, using histochemical techniques, using spectrometry, or by another technique. An antigen binding molecule can be identified using a barcode associated with the reporter oligonucleotide (e.g., a reporter barcode on the oligonucleotide conjugated to the antigen, or a spatial barcode appended to a capture probe comprising a sequence complementary to a sequence of the reporter oligonucleotide conjugated to the antigen).

The sequence (e.g., the amino acid sequence) of the antigen binding molecule can be determined. The sequence of the entire antibody can be determined. A portion of the sequence of the antibody, such as the variable region of the light chain or a fragment thereof, the variable region of the heavy chain or a fragment thereof, the constant region of the light chain or a fragment thereof, the constant region of the heavy chain or a fragment thereof, or a combination thereof can be determined. This can be accomplished, for example, by using mass spectrometry or by Edman degradation using a protein sequenator (sequencer).

An isotype of the antigen binding molecule (e.g., IgA, IgD, IgG, IgE, or IgM) can be determined. Isotype can be determined, for example, by sequencing or by an assay designed to determine the isotype (e.g., an antibody based assay or other assay).

The antigen binding molecule can be identified based on its affinity to an epitope of the antigen. In some such cases, the sequence of the antigen can be known, and techniques such as epitope mapping techniques can be implemented to identify the antigen binding molecule.

More than one antigen binding molecules can be identified. For example, a sample from a subject can include more than one antigen binding molecule that can bind to an antigen (e.g., a therapeutic antibody) that can be identified by methods described herein. In some such cases, a first identified antigen binding molecule can have a higher affinity for the antigen than a second identified antigen binding molecule. The more than one antigen binding molecule can bind to the same epitope on the antigen or to different epitopes on the antigen. 2, 3, 4, 5, 10, 50, 100, or more antibodies can be identified.

### Identification of Mutations in Therapeutic Antibodies or Antibody-Drug Conjugates

One or more mutations in therapeutic antibodies or antibody-drug conjugates that eliminate the recognition of the mutated therapeutic antibodies or antibody-drug conjugates comprising such mutations by the preexisting anti-drug antibodies can be determined by methods provided herein.

A count matrix of one or more cells and drug-reactive antibodies conjugated to reporter oligonucleotides can be derived. A count matrix of cells expressing drug-reactive antibodies, and therapeutic antibodies conjugated to reporter oligonucleotides can be derived. A count matrix of cells expressing unmodified or mutated therapeutic antibodies and drug-reactive antibodies conjugated to reporter oligonucleotides can be derived. A count matrix of cells expressing drug-reactive antibodies, therapeutic antibodies conjugated to reporter oligonucleotides, and therapeutic ligands conjugated to reporter oligonucleotides can be derived. A count matrix of cells expressing mutated therapeutic antibodies, drug-reactive antibodies conjugated to reporter oligonucleotides, and therapeutic ligands conjugated to reporter oligonucleotides can be derived. The count matrix can, for example, provide data regarding cells (e.g., T cells or B cells) from a composition that containing antibodies that can bind to a therapeutic antibody, antibody-drug conjugate, control antigen, viral antigen, or other etiologic antigen, such as a cancer antigen, control antigen, or decoy antigen.

Cells can be filtered to retain cells that have both a detected VDJ/antibody sequence and non-zero drug-reactive antibody counts and can be sequenced to an appropriate depth. Cells can be sequenced at approximately 5,000 to 100,000 reads per cell for both antigen and VDJ libraries. Cells can be sequenced at approximately 5,000 to 20,000 reads per cell for both antigen and VDJ libraries. Cells can be sequenced at approximately 20,000 to 40,000 reads per cell for both antigen and VDJ libraries. Cells can be sequenced at approximately 40,000 to 60,000 reads per cell for both antigen and VDJ libraries. Cells can be sequenced at approximately 60,000 to 80,000 reads per cell for both antigen and VDJ libraries. Cells can be sequenced at approximately 80,000 to 100,000 reads per cell for both antigen and VDJ libraries. Saturation can occur rapidly.

The count matrix can be retained from cells that are removed. For each antigen, background concentration and ambient concentration can be calculated (e.g., mean, median, or hypergeometric mean), and appropriate deviation for non-cell-associated (empty barcodes) can be calculated. For a given cell retained by the filter, a logarithm (e.g., natural, base 10, or base 2) or similar transformation (e.g., variance stabilizing transformation, square root, or cubic root) can be performed, a pseudocount (e.g., ranging from 1-30) can be added, and the background can be subtracted. The value can then be scaled (e.g., by division) by the deviation or variance of the given antigen. Further, the value can be modified using a Gaussian mixture model for each antigen on a count vector for each cell, and can include, for example, but not limited to, covariates for sequencing depth or number of detected unique molecular identifiers, cell annotations, or other technical or biological covariates, such as the expression level of the BCR within the cell.

Alternatively, background and/or ambient concentration can be calculated in empty features, and subtracted for each antigen. Features of the capture array may not covered by tissue can be subtracted. See, e.g., PCT Publication No. WO2021102003A1. A pseudocount can be added (e.g., ranging from 1-50). The center log ratio or other appropriate compositional transformation can then be applied.

The resulting matrix and cellular populations thereof can be visualized using standard dimensional reduction models, for example PCA, UMAP, t-SNE, or Poincare disks. Further application of spectral clustering, granularity/resolution-based/community detections algorithms, or other algorithms can be used to separate cells into groups of cells with distinct drug reactive antibody profiles.

### Enzyme-Tagged Therapeutic Antibodies or Antibody-Drug Conjugates

Methods provided herein can be used for enzyme-tagged therapeutic antibodies or antibody-drug conjugates, wherein the therapeutic antibodies or antibody-drug conjugates can be tagged for example with a sortase motif (LPXTG). a therapeutic antibody or antibody-drug conjugate can be bound by a cell expressing an anti-drug antibody and captured. Incubation with a protein tag or peptide tag including an N-terminal glycine and addition of a bacterial SrtA protein or an engineered variant thereof (e.g., P94, D160N, or K196T) can confer a protein tag which can then be recognized by a barcoded antibody with an additional round of staining. For example, the covalently or non-covalently attached protein tag is selected from BCCP (biotin carboxyl carrier protein) tag, glutathione-S-transferase tag, green fluorescent protein tag, halo-tag, SNAP tag, CLIP tag, HUH tag, maltose binding protein tag, Nus tag, thioredoxin tag, Fc tag, and CRDSAT tag. For example, the covalently or non-covalently attached peptide tag is selected from ALFA tag, AviTag, C-tag, calmodulin tag, polyglutamate tag, polyarginine tag, E tag, FLAG tag, HA tag, His tag, Myc tag, NE tag, Rho1D4 tag, S tag, SBP tag, Softag 1, Softag 3, Spot tag, Strep tag, T7 tag, TC tag, Ty tag, V5 tag, VSV tag, Xpress tag, Isopeptag, Spy tag, Snoop tag, DogTag, and SdyTag.

### Capture of Anti-Drug Antibodies Using a Splint Oligonucleotide

A splint oligonucleotide can be used to capture and/or detect both an anti-drug antibody RNA transcript and genomic DNA. RTX polymerase can be used to produce a clonable and uniquely amplifiable anti-drug antibody VDJ sequence that can be cell associated. Such sequences can be amplified after next-generation sequencing and analysis, and can enable rapid production of anti-drug antibodies for research or screening purposes.

### Efficacy Validation and Re-engineering Therapeutic Antibodies and Antibody Drug Conjugates

Anti-drug antibodies isolated using a method provided herein can be used to validate the efficacy of a therapeutic antibody or antibody-drug conjugate re-engineering. By expressing and labeling the anti-drug antibodies with oligonucleotides and staining engineered cells or a tissue sample comprising such engineered cells expressing mutants of the therapeutic antibody or antibody drug conjugate of choice, a workflow described herein, e.g., in example 1, can be used to confirm that the new therapeutic antibody or antibody-drug variant is not bound by the same antibodies as the original therapeutic antibody or antibody-drug variant. Furthermore, use of such antibodies in functional assays (e.g., ADCC, ADCP, ADCD, or other assays) can be used to identify anti-drug antibodies with therapeutic value. For example, such anti-drug antibodies can be developed as drugs to be administered to patients experiencing side effects induced by a therapeutic antibody or antibody-drug conjugate by neutralizing the drug or promoting its destruction. Such anti-drug antibodies can also be deployed as reference standards in traditional serum-based anti-drug antibody assays. This can be effective for therapeutic antibodies or antibody-drug conjugates that have been configured to be recycled efficiently, such as by the addition of one or more Fc domains that can be recognized by a neonatal Fc receptor (FcRn, product of the FCGRT gene). Antigens more broadly can also be used to characterize the response in patients with diverse immunoglobulin haplotypes, and in combination with traditional statistical models such as contingency tests, GWAS, PheWAS, and/or cellular assays discover genomic or other elements associated with the development of anti-drug antibodies.

### Identification of Re-engineered Antigens Recognized by Antigen Binding Molecules

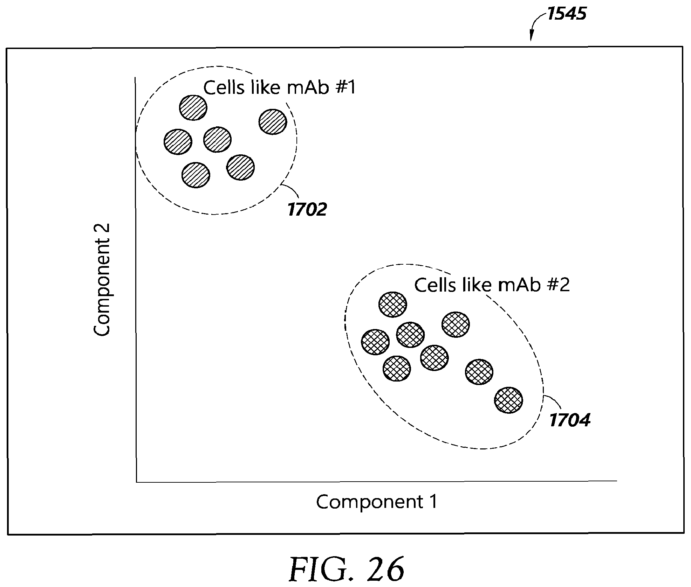

The methods provided herein can be implemented to perform an experiment with 5 different point mutants of adalimumab (FIG. 25). Following the procedure of the method, each point mutant can be labeled with its own unique reporter barcode oligonucleotide population. The presence of 2 distinct populations of cells in the resulting clustering and visualization analyses can indicate that these populations of cells can recognize distinct (e.g., disjoint) sets of the 5 antigens used (FIG. 26). Mutation or masking of the identified amino acids in the parent antigen (i.e., adalimumab) can ablate recognition of the drug by patient derived anti-drug antibodies, guiding the drug engineering and development process accordingly. A re-engineered antibody can be knocked into a cell line, expressed, and purified to produce a new antigen (e.g., adalimumab variant) to be used in the same patient samples.

### Analysis of Anti-Drug Antibodies (ADA) or Drug-Reactive Antibodies (DRA)

The methods provided herein can be implemented to isolate and characterize anti-drug antibodies (ADAs) or drug-reactive antibodies (DRAs) via barcoding. For example, an antigen conjugated to a reporter molecule can be used to profile samples from a subject treated with an antibody-drug conjugate (ADC) and/or an antibody therapeutic (AbTx). An exemplary workflow for associating the reporter molecule with an antibody (e.g., an ADA, an ADC, or an AbTx) is shown in FIG. 21. For example, the reporter molecule 2120 can be conjugated with e.g., an antibody-binding protein (e.g., Protein A or Protein G), and the complex 2130 can be formed by non-covalent association between the antibody binding protein and the ADC and/or the AbTX. The complex 2130 can be incubated with a tissue sample. The tissue sample may be isolated from a subject. Cells of the tissue sample express an AbTx which can be unmodified or modified. Unbound 2130 is washed off, and 2140 can be partitioned, e.g., into a droplet 2150 for further analysis.

### Identification of Anti-Drug Antibodies

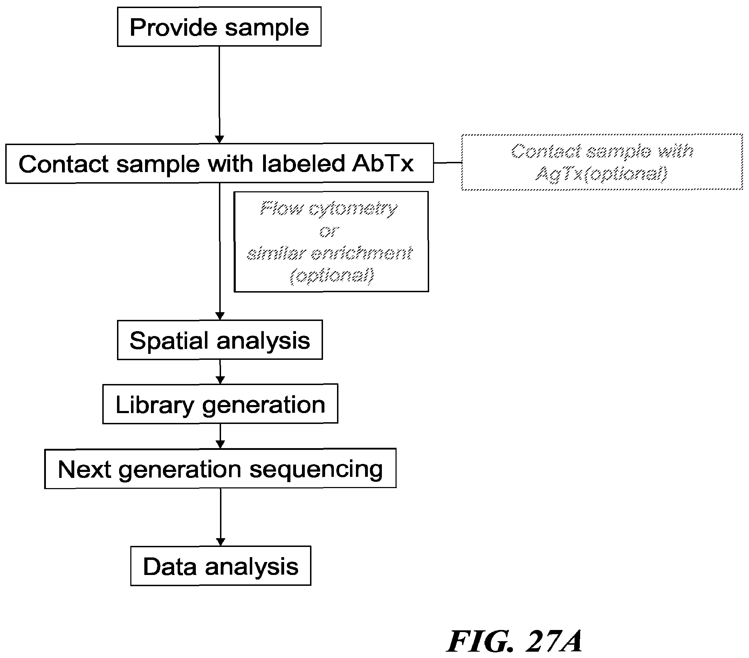

The methods provided herein can be implemented to identify ADAs to an AbTx via barcoding. An exemplary workflow of identifying ADAs is described in FIG. 27A. A tissue sample can be obtained from a patient treated with a known AbTx.

The known AbTx can be conjugated to a reporter oligonucleotide according to the methods described herein. The AbTx can also be labeled with a reporter fluorophore or any other detectable labels that can enable identification of the AbTx by, for example, flow cytometry or any other known similar methods. The AbTx can be pre-incubated with its labeled antigenic target (AgTx), enabling identification of ADAs binding to AbTx-AgTx complex rather than the AbTx alone.

The tissue sample can be incubated with the reporter oligonucleotide conjugated AbTx. The tissue sample can be contacted with a reporter oligonucleotide conjugated AbTx panel comprising unmodified and modified (or mutated) AbTx conjugated with reporter oligonucleotides. The tissue sample can also be contacted with a panel of additional labeling agents comprising, e.g., reporter oligonucleotide antibody panels for cell surface markers used, e.g., for cell classification. The tissue sample can be processed for spatial analysis according to a method disclosed herein. For example, the tissue sample can be contacted with a substrate comprising an array of capture probes. Analytes from an ABM-expressing cell of the tissue sample, as well as reporter oligonucleotides from the AbTx panel, and optionally reporter oligonucleotides from the panel of additional labeling agents, can be attached to capture probes disclosed herein, e.g., according to a spatial analysis method disclosed herein. This can generate a reporter barcode library, a V(D)J library, and optionally a gene expression library. The reporter barcode library can include spatially barcoded polynucleotides or amplicons or library members thereof comprising (i) a reporter barcode sequence or reverse complement thereof and (ii) a spatial barcode sequence or reverse complement thereof. The V(D)J library can include native sequences of ABMs that bind an AbTx, expressed antibody sequences in engineered cells.

The generated libraries can be sequenced. Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}).

A count matrix of one or more cells expressing ADAs and reporter barcodes associated with the AbTx or AbTx-AgTx complex can be generated from the sequence data. The count matrix may be a count matrix described in FIG. 25, wherein each row corresponds to one or more cells expressing an antigen binding molecule (i.e., an ADA), and each column corresponds to an antigen (i.e., the known AbTx (unmodified), one or more modified (mutated) AbTx with at least one point mutation, or the AbTx-AgTx complex). Each element of the count matrix described in FIG. 25 corresponds to a count of quantity of times the antigen bound to the one or more cells expressing a particular antigen binding molecule (i.e., a particular ADA). BCR or TCR sequences from cells having a threshold number of AbTx reporter oligonucleotide UMI counts can be used to identify the particular ADA bound to the unmodified AbTx, modified AbTx, or the AbTx-AgTx complex.

### Identification of a Patient-Specific Antibody Therapeutic

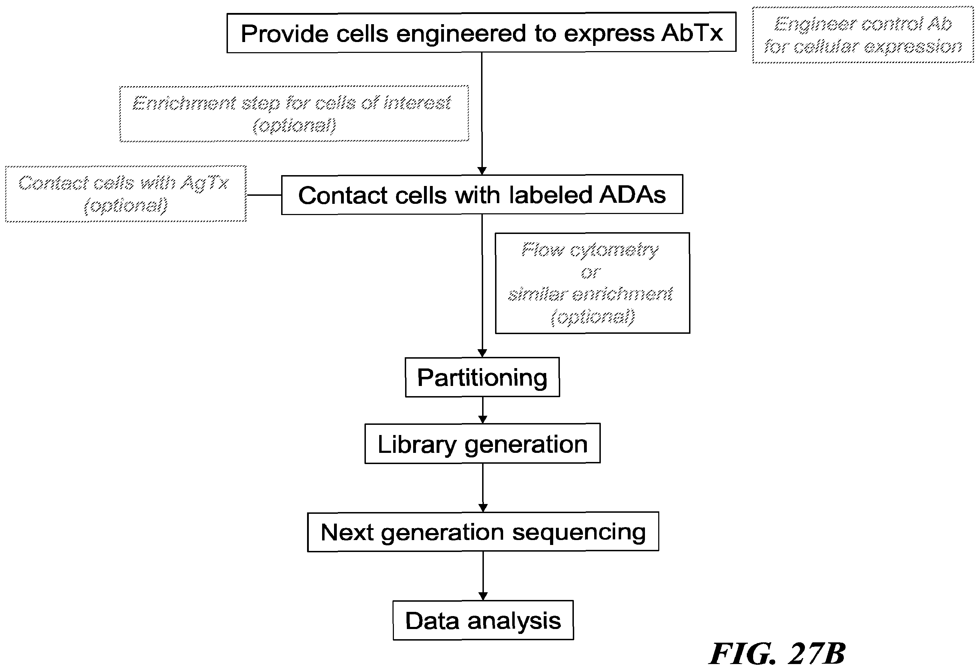

The methods provided herein can be implemented to identify a patient specific engineered AbTx that does not elicit an immune response in the patient. An exemplary workflow of identifying the patient specific AbTx is described in FIG. 27B.

The ADAs identified according to the methods disclosed herein, e.g., the methods disclosed in Example 7, can be conjugated to reporter oligonucleotides as described herein. The ADAs can also be labeled with a reporter fluorophore or any other detectable labels that can enable identification of the ADA by, for example flow cytometry, imaging, or any other known similar methods.

Cells can be engineered to express the AbTx and/or the AbTx comprising one or more point mutations. The AbTx and/or the modified (mutated) AbTx can be tethered to the cell surface when expressed from constructs comprising the 5th exon, but not the 6th exon, of an IgG1 constant region. The AbTx and/or the modified (mutated) AbTx can be secreted when expressed from constructs comprising the 6th exon, but not the 5th exon, of an IgG1 constant region. The engineered cells expressing the AbTx or the modified (mutated) AbTx can be pre-incubated with its labeled antigenic target (AgTx), thereby forming a AbTx-AgTx complex. The engineered cells can be enriched in order to identify a modified (mutated) AbTx with a low ADA activity or a high ADA activity.

The engineered cells can be incubated with the reporter oligonucleotide conjugated ADAs. The engineered cells can be contacted with a panel of reporter oligonucleotide conjugated ADAs and a control antibody.

The engineered cells bound to the reporter oligonucleotide conjugated ADAs can be partitioned and subjected to, for example, 10x 5'V2 Single Cell Immune Profiling kit per manufacturer's instructions. Additional information in this regard can be found at "support.10xgenomics.com/permalink/getting-started-immune-profiling-feature-barcoding." This can generate a reporter barcode library, a V(D)J library, and optional a gene expression library. The AbTx or modified (mutated) AbTx can be detected using the V(D)J library and/or the gene expression library. Reporter barcoded ADAs can be detected using the reporter barcode library. Reporter barcoded AbTx-AgTx can be detected using the reporter barcode library.

Alternatively, a tissue sample comprising the engineered cells may be used. The tissue sample can be incubated with the reporter oligonucleotide conjugated ADAs. The tissue sample can be contacted with a panel of reporter oligonucleotide conjugated ADAs and a control antibody. The tissue sample can also be contacted with a panel of additional labeling agents comprising, e.g., reporter oligonucleotide antibody panels for cell surface markers used, e.g., for cell classification. The tissue sample can be processed for spatial analysis according to a method disclosed herein. For example, the tissue sample can be contacted with a substrate comprising an array of capture probes. Analytes from an ABM-expressing cell of the tissue sample, as well as reporter oligonucleotides from the AbTx panel, and optionally reporter oligonucleotides from the panel of additional labeling agents, can be attached to capture probes disclosed herein, e.g., according to a spatial analysis method disclosed herein. This can generate a reporter barcode library, a V(D)J library, and optionally a gene expression library. The reporter barcode library can include spatially barcoded polynucleotides or amplicons or library members thereof comprising (i) a reporter barcode sequence or reverse complement thereof and (ii) a spatial barcode sequence or reverse complement thereof. The AbTx or modified (mutated) AbTx can be detected using the V(D)J library and/or the gene expression library. Reporter barcoded ADAs can be detected using the reporter barcode library. Reporter barcoded AbTx-AgTx can be detected using the reporter barcode library.

The libraries can be enriched for targets of interest, which can include any combination of ADA antibody sequences identified using methods disclosed herein, and known AbTx or AbTx mutant sequences. An exemplary target enrichment method can comprise providing a plurality of barcoded nucleic acid molecules (e.g., library members) and hybridizing barcoded nucleic acid molecules comprising targeted regions of interest to oligonucleotide probes ("baits") which can be complementary to the targeted regions of interest (or to regions near or adjacent to the targeted regions of interest). Baits can be attached to a capture molecule, including without limitation a biotin molecule. The capture molecule (e.g., biotin) can be used to selectively pull down the targeted regions of interest (for example, with magnetic streptavidin beads) to thereby enrich the resultant population of barcoded nucleic acid molecules for those containing the targeted regions of interest.

The generated libraries can be sequenced on, for example, a NovaSeq 3 using a NovaSeq S4 200 cycles 2020 v1.5 kit per manufacturer's instructions. Sequencing can also be performed by various systems currently available, such as, without limitation, a sequencing system by Illumina^{®}, Pacific Biosciences (PacBio^{®}), Oxford Nanopore^{®}, or Life Technologies (Ion Torrent^{®}). The binding affinity of ADAs to the AbTx, the modified (mutated) AbTx, or the AbTx-AgTx complex can be determined based on quantity/numbers of unique molecular identifiers (UMIs) associated with each of the antigen binding molecules bound to the target antigen.

A count matrix of one or more cells expressing the AbTx or the modified (mutated) AbTx and reporter barcodes associated with the ADAs can be generated. The count matrix may be a count matrix described in FIG. 25, wherein each row corresponds to one or more cells expressing an antigen binding molecule (i.e., the known AbTx (unmodified), one or more modified (mutated) AbTx with at least one point mutation, or the AbTx-AgTx complex), and each column corresponds to an antigen (i.e., an ADA). Each element of the count matrix described in FIG. 25 corresponds to a count of quantity of times the antigen bound to the one or more cells expressing a particular antigen binding molecule (i.e., a particular AbTx). This data can be used to identify the epitope (the antigen binding site) of the unmodified AbTx, the modified (mutated) AbTx, or the AbTx-AgTx bound by the ADA. This data can be used to identify a patient-specific AbTx, engineered to not elicit an immune response in the patient when treated with the identified patient-specific AbTx.

### Characterizing Binding of Anti-Drug Antibody to Antibody Therapeutic

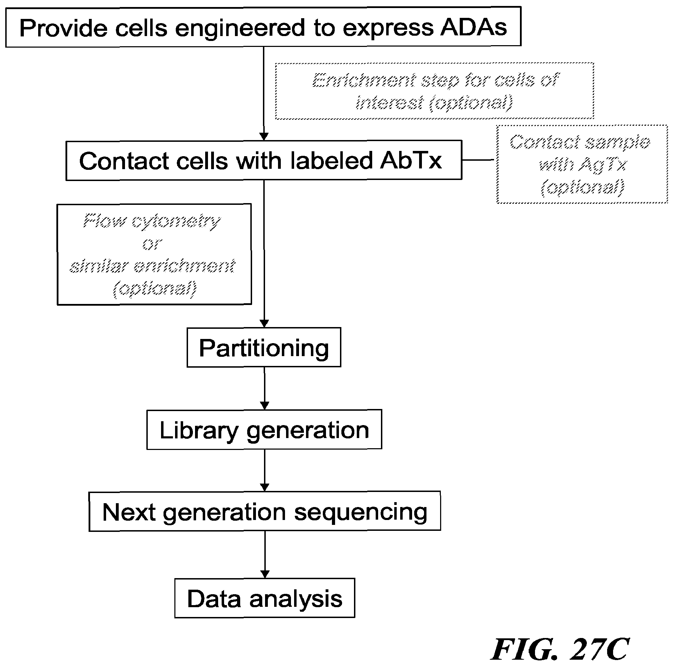

The methods provided herein can be implemented to identify a patient specific engineered AbTx that does not elicit an immune response in patient. In some cases, the methods identify a patient-specific engineered AbTx that retains its binding profile to its AgTx and does not elicit an immune response in the patient. Exemplary workflows of identifying the patient specific AbTx are described in FIGS. 27C-27D.

Referring to FIG. 27C, the AbTx can be conjugated to reporter oligonucleotides as described herein. Optionally, the AbTx is modified (mutated) to comprise at least one mutation, wherein each modified AbTx is conjugated with a unique reporter oligonucleotide. For example, a panel of AbTx's, e.g., including an unmodified AbTx and one or more modified AbTx's, can be conjugated with reporter oligonucleotides that identify the AbTx. The AbTx and/or modified AbTx can also be labeled with a reporter fluorophore or any other detectable labels that can enable identification of the AbTx by, for example flow cytometry, imaging, or any other known similar methods. The AbTx and/or modified AbTx can be also labeled with an enrichment molecule, which can, for example, without limitation, activate FRET-based fluorescence upon binding its target ligand. The target ligand can also be labeled with a reporter oligonucleotide or a fluorescent molecule that can be used in downstream enrichment steps.

Continuing with FIG. 27C, cells can be engineered to express the ADAs identified in Example 7. Optionally, cells engineered to express a control antibody can be provided. The control antibody can be a non-ADA that can bind an irrelevant target that is not the AbTx (e.g., a cell surface protein, a murine version of a human protein). The control antibodies can be a commercially available clone against, for example, without limitation, CD3 or CD8. IThe ADAs can be tethered to the cell surface when expressed from constructs comprising the 5th exon, but not the 6th exon, of an IgG1 constant region. The ADAs can be secreted when expressed from constructs comprising the 6th exon, but not the 5th exon, of an IgG1 constant region.

The engineered cells can be incubated with the AbTx and/or the modified (mutated) AbTx conjugated with reporter oligonucleotides. The engineered cells can be contacted with a panel of the AbTx and/or the modified (mutated) AbTx conjugated with reporter oligonucleotides. The engineered cells expressing the ADAs can be pre-incubated with its labeled antigenic target, which can optionally comprise a reporter oligonucleotide that can identify the antigenic target. The engineered cells can be enriched in order to identify a modified (mutated) AbTx that poorly or strongly binds ADAs. This enrichment process can optionally be repeated in a panning-like process.

The engineered cells bound to the AbTx and/or modified (mutated) AbTx conjugated with reporter oligonucleotides are partitioned and subjected to, for example, 10x 5'V2 Single Cell Immune Profiling kit per manufacturer's instructions. Additional information in this regard can be found at "support.10xgenomics.com/permalink/getting-started-immune-profiling-feature-barcoding." This can generate a reporter barcode library, a V(D)J library, and optional a gene expression library. The reporter barcode library can be used to detect the presence of the AbTx, the modified (mutated) AbTx, and optionally the AgTx ligand. The V(D)J library and the gene expression library can be used to detect the presence of ADAs and the control antibodies expressed by the cells. The libraries can be enriched for targets of interest, which can include any combination of ADA antibody sequences identified using methods disclosed herein, and known AbTx or AbTx mutant sequences. Exemplary methods for enrichment of libraries for targets of interest are disclosed herein.

Alternatively, a tissue sample comprising the engineered cells may be used. The tissue sample can be incubated with the reporter oligonucleotide conjugated AbTx. The tissue sample can be contacted with a panel of reporter oligonucleotide conjugated AbTx. The tissue sample can be pre-incubated with its labeled antigenic target, which can optionally comprise a reporter oligonucleotide that can identify the antigenic target. The tissue sample can also be contacted with a panel of additional labeling agents comprising, e.g., reporter oligonucleotide antibody panels for cell surface markers used, e.g., for cell classification. The tissue sample can be processed for spatial analysis according to a method disclosed herein. For example, the tissue sample can be contacted with a substrate comprising an array of capture probes. Analytes from the tissue sample, as well as reporter oligonucleotides from the reporter oligonucleotide conjugated AbTx panel, and optionally reporter oligonucleotides from the panel of additional labeling agents, can be attached to capture probes disclosed herein, e.g., according to a spatial analysis method disclosed herein. This can generate a reporter barcode library, a V(D)J library, and optionally a gene expression library. The reporter barcode library can be used to detect the presence of the AbTx, the modified (mutated) AbTx, and optionally the AgTx ligand. The V(D)J library and the gene expression library can be used to detect the presence of ADAs and the control antibodies expressed by the cells. The libraries can be enriched for targets of interest, which can include any combination of ADA antibody sequences identified using methods disclosed herein, and known AbTx or AbTx mutant sequences. Exemplary methods for enrichment of libraries for targets of interest are disclosed herein.

The generated libraries can be sequenced on, for example, a NovaSeq 3 using a NovaSeq S4 200 cycles 2020 v1.5 kit per manufacturer's instructions. Sequencing can also be performed by various systems currently available, such as, without limitation, a sequencing system by Illumina^{®}, Pacific Biosciences (PacBio^{®}), Oxford Nanopore^{®}, or Life Technologies (Ion Torrent^{®}). The binding affinity of ADAs to the AbTx, the modified (mutated) AbTx, or the AbTx-AgTx complex can be determined based on quantity/numbers of unique molecular identifiers (UMIs) associated with each of the antigen binding molecules bound to the target antigen.

A count matrix of one or more cells expressing the ADAs and the AbTx or the modified (mutated) AbTx conjugated with and reporter barcodes can be generated. The count matrix may be a count matrix described in FIG. 25, wherein each row corresponds to one or more cells expressing an antigen binding molecule (i.e., the known AbTx (unmodified), one or more modified (mutated) AbTx with at least one point mutation, or the AbTx-AgTx complex), and each column corresponds to an antigen (i.e., an ADA). Each element of the count matrix described in FIG. 25 corresponds to a count of quantity of times the antigen bound to the one or more cells expressing a particular antigen binding molecule (i.e., a particular AbTx). This data can be used to identify (1) cases where each AbTx or each modified (mutated) AbTx is bound by one or more ADAs; (2) whether each AbTx or each modified (mutated) AbTx binds the AgTx ligand; (3) whether an ADA binding of each AbTx or each modified (mutated) AbTx can abrogate AgTx ligand binding; and (4) whether a particular AbTx-ADA binding is dependent on a AgTx binding to AbTx.

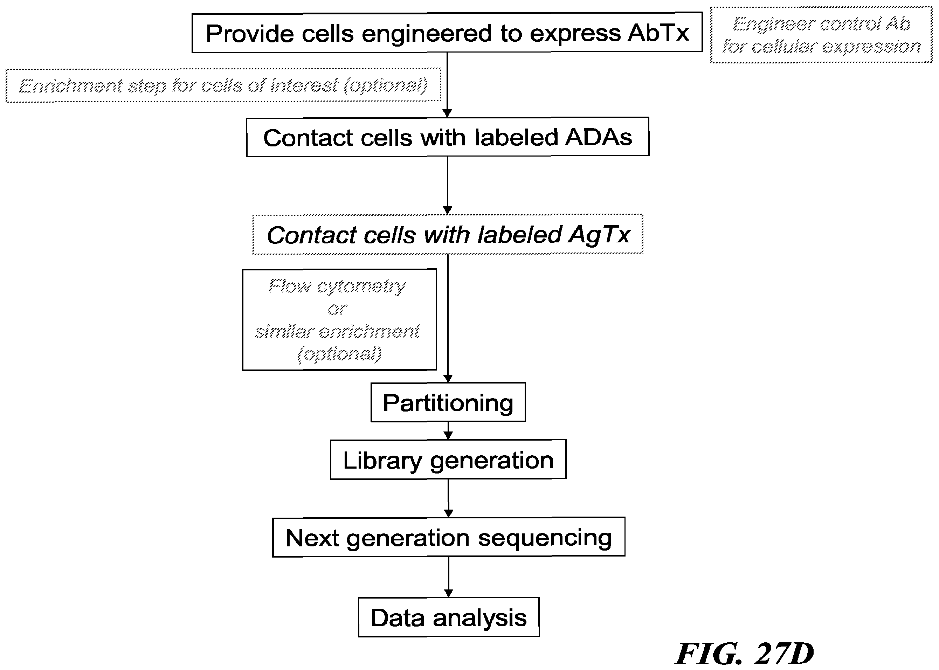

The ADAs and AbTx can be reversed in this workflow, where a library of ADAs can bind the cells expressing the AbTx and/or the modified (mutated) AbTx, the process of which is described in FIG. 27D.

### Determining an Epitope

Methods described herein can include identifying a site on the antigen binding to the antigen binding molecule. Identification of a site can include identification of at least one amino acid(s) that contribute of binding of the antigen to the antigen binding molecule.

Methods described herein can include identifying an epitope on an antigen that has affinity to the antigen binding molecule. Such an epitope can partially or fully confer affinity of the antigen binding molecule to the antigen.

An epitope can be a portion of an antigen or other macromolecule capable of forming a binding interaction with the variable region binding pocket of an antigen binding molecule such as an antibody or antigen binding fragment thereof. Such binding interactions can be manifested as an intermolecular contact with one or more amino acid residues of one or more CDRs. Antigen binding can involve, for example, a CDR3, a CDR3 pair or, in some cases, interactions of up to all six CDRs of the VH and VL chains. Antigen binding can involve framework regions (FWRs) that scaffold the CDRs. An epitope can be a linear peptide sequence (i.e., "continuous") or can be composed of noncontiguous amino acid sequences (i.e., "conformational" or "discontinuous"). An antigen binding molecule such as an antibody or antigen binding fragment thereof can recognize one or more amino acid sequences; therefore, an epitope can define more than one distinct amino acid sequence. Epitopes recognized by antigen binding molecules such as antibodies or antigen binding fragments thereof can be determined by peptide mapping or sequence analysis techniques. Binding interactions can be manifested as intermolecular contacts between an epitope on an antigen and one or more amino acid residues of a CDR. Epitopes recognized by antigen binding molecules such as antibodies or antigen binding fragments thereof can be determined, for example, by peptide mapping or sequence analysis techniques. Binding interactions can manifest as intermolecular contacts between an epitope on an antigen and one or more amino acid residues of a complementarity determining region (CDR).

An epitope can be determined, for example, using one or more epitope mapping techniques. Epitope mapping can include experimentally identifying the epitope on an antigen. Epitope mapping can be performed by any acceptable method, for example, but not limited to, X-ray co-crystallography, cryogenic electron microscopy, array-based oligopeptide scanning, site-directed mutagenesis mapping, high-throughput shotgun mutagenesis epitope mapping, hydrogen-deuterium exchange, cross-linking coupled mass spectrometry, yeast display, phage display, proteolysis, SPR chip, next generation sequencing, or a combination thereof.

### Epitope Modification

Methods described herein can include modifying an epitope of the antigen. An epitope can be modified to reduce the affinity of the antigen binding molecule for the antigen. In some such cases, an epitope can be modified to partially or fully prevent binding of the antigen binding molecule to the antigen.

Modification of an epitope can be based on the identity of the antigen binding molecule or the epitope. In some cases, an epitope determined, for example by epitope mapping as provided herein, can be modified. After modification of the epitope, the modified antigen can be contacted with the antigen binding molecule, and affinity between the modified antigen and antigen binding molecule can be determined. The affinity between the modified antigen and antigen binding molecule can be significantly reduced or physiologically negligible.

Epitope modification can include one or more of: a modification (e.g., insertion, deletion, or mutation) of the amino acid sequence of the epitope, a post-translational modification to the epitope (e.g., glycosylation, ubiquitination, phosphorylation, myristoylation, palmitoylation, isoprenylation, farnesylation, geranylgeranylation, glipyation, lipoylation, attachment of a flavin moiety, attachment of heme C, phosphopantetheinylation, retinylidene Schiff base formation, diphthamide formation, ethanolamide phosphoglycerol attachment, hypusine formation, beta-Lysine addition, acylation, alkylation, amidation, amide bond formation, arginylation, polyglutamylation, polyglycylation, butyrylation, gamma-carboxylation, malonylation, hydroxylation, iodination, nucleotide addition, phosphate ester or phosphoramidate formation, adenylation, uridylylation, propionylation, pyroglutamate formation, S-glutathionylation, S-nitrosylation, S-sulfenylation, S-sulfinylation, succinylation, sulfation, glycation, carbamylation, carbonylation, spontaneous isopeptide bond formation, biotinylation, carbamylation, oxidation, pegylation, ISGylation, SUMOylation, neddylation, pupylation, citrullination, deamidation, eliminylation, formation of a disulfide bridge, proteolytic cleavage, isoaspartate formation, racemization, protein splicing, or a combination thereof), or binding of a molecule to the epitope to block the antigen binding molecule from binding the epitope.

For example, an antigen binding molecule such as an antibody from a subject can be identified as having affinity to an antibody (e.g., an antibody therapeutic) or antibody-based drug to be administered to the subject. Administering that antibody or antibody-based drug to the subject can result in binding of the antibody (e.g., an antibody therapeutic) or antibody-based drug to the binding molecule in the subject, thereby reducing the therapeutic effect of the antibody (e.g., an antibody therapeutic) or antibody-based drug, undesirable side effects, or other undesirable effects. An epitope of the antibody (e.g., an antibody therapeutic) or antibody-based drug can be modified such that the affinity of one or more antigen binding molecules of the subject for the antibody (e.g., an antibody therapeutic) or antibody-based drug is reduced. In some such cases, reduction in therapeutic effect of the antibody (e.g., an antibody therapeutic) or antibody-based drug, undesirable side effects, or other undesirable effects can be ameliorated. The epitope can be modified without significantly reducing the efficacy of the antibody (e.g., an antibody therapeutic) or antibody-based drug. The modification to the epitope of the antibody (e.g., antibody therapeutic) or antibody-based drug may comprise a modification to one or more framework (FWR) or CDR sequences of the antibody (e.g., antibody therapeutic) or antibody-based drug, such as CDRH3 or CDRL3. These modifications to the heavy chain or light chain FWR and CDR sequences of the antibody (e.g., antibody therapeutic) or antibody-based drug may not affect its binding to the antigenic target (AgTx) of the antibody (e.g., antibody therapeutic) or antibody-based drug.

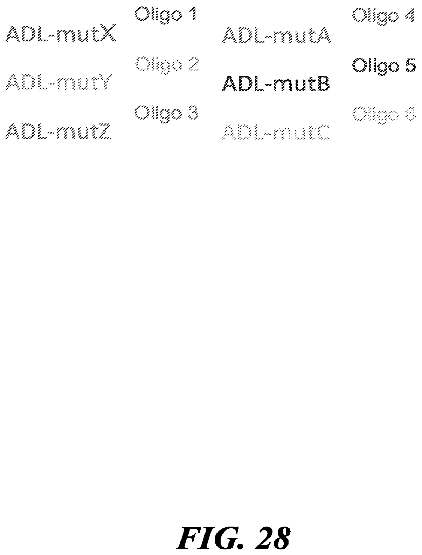

One or more antigens having one or more modified epitopes (e.g., antigen variants) can be conjugated to reporter oligonucleotides and used as antigens in methods provided herein. Antigens having one or more modified epitopes can have reduced affinity for the antigen-binding molecule. Antigens having one or more modified epitopes can have no affinity for the antigen-binding molecule. Examples of such antigen variants conjugated to oligonucleotides are provided in FIG. 28.

### Antigens and Antigen Binding Molecules

### Antigens

An antigen can include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a Darpin, and a protein scaffold, or any combination thereof.

An antigen can be a molecule that can have affinity to an antigen binding molecule. For example, an antigen can have affinity to an antibody or antigen binding fragment thereof. In some, when contacted with an antigen binding molecule, the antigen can bind to the antigen binding molecule. An antigen can be a biomolecule, such as a biologic therapeutic molecule. Examples of biologic therapeutic molecules can be, for example, a drug-reactive antibody or anti-drug antibody that is produced from a living organism or that contains one or more components of a living organism. A biologic therapeutic molecule can be derived from a human, animal, or microorganism using biotechnology techniques. Examples of biologic therapeutic molecules can include, for example, an immunological molecule (e.g. an antibody (such as a monoclonal antibodies), a fusion protein, a protein product of a gene therapy, a peptide, or other biologic molecule.

An antigen can be an antibody or antigen binding fragment thereof. An antigen can be an antibody-drug conjugate. An antigen can be a therapeutic antibody or antigen binding fragment thereof (e.g., a monoclonal antibody). An antigen can be a chimeric antigen receptor T cell therapy. The chimeric antigen receptor T cell therapy may be selected from the group consisting of axicabtagene ciloleucel (YESCARTA^{®}), tisagenlecleucel (KYMRIAH^{®}), brexucabtagene autoleucel (TECARTUS), idecabtagene vicleucel (ABECMA^{®}), and lisocabtagene maraleucel. (BREYANZI^{®}).

### Antigen Binding Molecules

An antigen binding molecule can be a molecule capable of binding an antigen. An antigen binding molecule can be an antibody or antigen binding fragment thereof. Examples of antibodies are provided herein.

An antigen binding molecule can be an antibody (such as an ADA) or antigen binding fragment thereof produced by a subject. The antibody or antigen binding fragment thereof can have affinity to an antigen provided herein. The antibody or antigen binding fragment thereof can have affinity to an antibody or antibody-based drug, for example an antibody or antibody-based drug that can be administered to a subject. The antigen binding molecule can have affinity to an antigen that is a biologic or a small molecule. For example, the antigen binding molecule can have affinity to a component of a vaccine composition.

### Antibodies

In methods provided herein, an antigen or an antigen binding molecule can be an antibody or antigen-binding fragment thereof. As used herein, the term "antibody" can refer to an immunoglobulin (Ig), polypeptide, or a protein having a binding domain which is, or is homologous to, an antigen-binding domain. The term can further include "antigen-binding fragments" and other interchangeable terms for similar binding fragments as described herein.

An antigen or an antigen binding molecule can be a therapeutic antibody or antigen binding fragment thereof (e.g., a monoclonal antibody). A therapeutic antibody or antigen binding fragment thereof can be a drug candidate or an FDA approved drug or therapeutic, such as a monoclonal antibody that is approved by the FDA for therapeutic use. Non-limiting examples of FDA approved monoclonal antibodies are provided in **Table 1.**

**Table 1. FDA Approved Therapeutic Monoclonal Antibodies and other immunotherapies**

| **Antibody** | **Brand name** | **Type** | **Target** |
|---|---|---|---|
| abciximab | ReoPro | chimeric Fab | GPIIb/IIIa |
| adalimumab | Humira | fully human | TNF |
| adalimumab-atto | Amjevita | fully human, biosimilar | TNF |
| ado-trastuzumab emtansine | Kadcyla | humanized, antibody-drug conjugate | HER2 |
| alemtuzumab | Campath, Lemtrada | humanized | CD52 |
| alirocumab | Praluent | fully human | PCSK9 |
| atezolizumab | Tecentriq | humanized | PD-L1 |
| atezolizumab | Tecentriq | humanized | PD-L1 |
| avelumab | Bavencio | fully human | PD-L1 |
| basiliximab | Simulect | chimeric | IL2RA |
| belimumab | Benlysta | fully human | BLyS |
| bevacizumab | Avastin | humanized | VEGF |
| bezlotoxumab | Zinplava | fully human | Clostridium difficile toxin B |
| blinatumomab | Blincyto | mouse, bispecific | CD19 |
| brentuximab vedotin | Adcetris | chimeric, antibody-drug conjugate | CD30 |
| brodalumab | Siliq | chimeric | IL17RA |
| canakinumab | Ilaris | fully human | IL1B |
| capromab pendetide | ProstaScint | murine, radiolabeled | PSMA |
| certolizumab pegol | Cimzia | humanized | TNF |
| cetuximab | Erbitux | chimeric | EGFR |
| daclizumab | Zenapax | humanized | IL2RA |
| daclizumab | Zinbryta | humanized | IL2R |
| daratumumab | Darzalex | fully human | CD38 |
| denosumab | Prolia, Xgeva | fully human | RANKL |
| dinutuximab | Unituxin | chimeric | GD2 |
| dupilumab | Dupixent | fully human | IL4RA |
| durvalumab | Imfinzi | fully human | PD-L1 |
| eculizumab | Soliris | humanized | Complement component 5 |
| elotuzumab | Empliciti | humanized | SLAMF7 |
| evolocumab | Repatha | fully human | PCSK9 |
| golimumab | Simponi | fully human | TNF |
| golimumab | Simponi Aria | fully human | TNF |
| ibritumomab tiuxetan | Zevalin | murine, radioimmunotherapy | CD20 |
| idarucizumab | Praxbind | humanized Fab | dabigatran |
| infliximab | Remicade | chimeric | TNF alpha |
| infliximab-abda | Renflexis | chimeric, biosimilar | TNF |
| infliximab-dyyb | Inflectra | chimeric, biosimilar | TNF |
| ipilimumab | Yervoy | fully human | CTLA-4 |
| ixekizumab | Taltz | humanized | IL17A |
| mepolizumab | Nucala | humanized | IL5 |
| natalizumab | Tysabri | humanized | alpha-4 integrin |
| necitumumab | Portrazza | fully human | EGFR |
| nivolumab | Opdivo | fully human | PD-1 |
| nivolumab | Opdivo | fully human | PD-1 |
| obiltoxaximab | Anthem | chimeric | Protective antigen of the Anthrax toxin |
| obinutuzumab | Gazyva | humanized | CD20 |
| ocrelizumab | Ocrevus | humanized | CD20 |
| ofatumumab | Arzerra | fully human | CD20 |
| olaratumab | Lartruvo | fully human | PDGFRA |
| omalizumab | Xolair | humanized | IgE |
| palivizumab | Synagis | humanized | F protein of RSV |
| panitumumab | Vectibix | fully human | EGFR |
| pembrolizumab | Keytruda | humanized | PD-1 |
| pertuzumab | Perjeta | humanized | HER2 |
| ramucirumab | Cyramza | fully human | VEGFR2 |
| ranibizumab | Lucentis | humanized | VEGFR1, VEGFR2 |
| raxibacumab | Raxibacumab | fully human | Protective antigen of Bacillus anthracis |
| reslizumab | Cinqair | humanized | IL5 |
| rituximab | Rituxan | chimeric | CD20 |
| secukinumab | Cosentyx | fully human | IL17A |
| siltuximab | Sylvant | chimeric | IL6 |
| tocilizumab | Actemra | humanized | IL6R |
| tocilizumab | Actemra | humanized | IL6R |
| trastuzumab | Herceptin | humanized | HER2 |
| ustekinumab | Stelara | fully human | IL12 |
| ustekinumab | Stelara | fully human | IL12, IL23 |
| vedolizumab | Entyvio | humanized | integrin receptor |
| sarilumab | Kevzara | fully human | IL6R |
| rituximab and hyaluronidase | Rituxan Hycela | chimeric, co-formulated | CD20 |
| guselkumab | Tremfya | fully human | IL23 |
| inotuzumab ozogamicin | Besponsa | humanized, antibody-drug conjugate | CD22 |
| adalimumab-adbm | Cyltezo | fully human, biosimilar | TNF |
| gemtuzumab ozogamicin | Mylotarg | humanized, antibody-drug conjugate | CD33 |
| bevacizumab-awwb | Mvasi | humanized, biosimilar | VEGF |
| benralizumab | Fasenra | humanized | interleukin-5 receptor alpha subunit |
| emicizumab-kxwh | Hemlibra | humanized, bispecific | Factor IXa, Factor X |
| trastuzumab-dkst | Ogivri | humanized, biosimilar | HER2 |
| infliximab-qbtx | Ixifi | chimeric, biosimilar | TNF |
| ibalizumab-uiyk | Trogarzo | humanized | CD4 |
| tildrakizumab-asmn | Ilumya | humanized | IL23 |
| burosumab-twza | Crysvita | fully human | FGF23 |
| erenumab-aooe | Aimovig | fully human | CGRP receptor |

An antigen or an antigen binding molecule can be similar to an FDA approved therapeutic monoclonal antibody. In some such cases, an antigen or an antigen binding molecule can have at least 75% identity, at least 80% identity, at least 85% identity, at least 90% identity, or at least 95% identity to an FDA approved therapeutic monoclonal antibody or a range between any two foregoing values. An antigen or an antigen binding molecule can have a heavy chain variable region that is at least 75% identity, at least 80% identity, at least 85% identity, at least 90% identity, or at least 95% identity to the heavy chain variable region of an FDA approved therapeutic monoclonal antibody or a range between any two foregoing values. An antigen or an antigen binding molecule can have a heavy chain constant region that is at least 75% identity, at least 80% identity, at least 85% identity, at least 90% identity, or at least 95% identity to the heavy chain constant region of an FDA approved therapeutic monoclonal antibody or a range between any two foregoing values. An antigen or an antigen binding molecule can have a light chain variable region that is at least 75% identity, at least 80% identity, at least 85% identity, at least 90% identity, or at least 95% identity to a light chain variable region of an FDA approved therapeutic monoclonal antibody or a range between any two foregoing values. An antigen or an antigen binding molecule can have a light chain constant region that is at least 75% identity, at least 80% identity, at least 85% identity, at least 90% identity, or at least 95% identity to the light chain constant region of an FDA approved therapeutic monoclonal antibody or a range between any two foregoing values. An antigen or an antigen binding molecule can be an antibody or antigen binding fragment thereof that has a target that is the same as the target of an FDA approved therapeutic monoclonal antibody. The antibody can be a secreted antibody. The antibody can be a surface-bound antibody.

Native antibodies and native immunoglobulins (Igs) can be heterotetrameric glycoproteins of about 150,000 Daltons, composed of two identical light chains and two identical heavy chains. Antibodies can further refer to camelid antibodies, which can be not tetrameric. Each light chain can be typically linked to a heavy chain by one covalent disulfide bond, while the number of disulfide linkages can vary among the heavy chains of different immunoglobulin isotypes. Each heavy and light chain can have regularly spaced intrachain disulfide bridges. Each heavy chain can have at one end a variable domain ("VH") followed by a number of constant domains ("CH"). Each light chain can have a variable domain at one end ("VL") and a constant domain ("CL") at its other end; the constant domain of the light chain can be aligned with the first constant domain of the heavy chain, and the light-chain variable domain can be aligned with the variable domain of the heavy chain. Particular amino acid residues can form an interface between the light- and heavy-chain variable domains.

An antibody or an antigen-binding fragment thereof may include an isolated antibody or antigen-binding fragment thereof, a purified antibody or antigen-binding fragment thereof, a recombinant antibody or antigen-binding fragment thereof, a modified antibody or antigen-binding fragment thereof, or a synthetic antibody or antigen-binding fragment thereof.

Antibodies and antigen-binding fragments herein can be partly or wholly synthetically produced. An antibody or antigen-binding fragment can be a polypeptide or protein having a binding domain which can be, or can be homologous to, an antigen binding domain. An antibody or an antigen-binding fragment thereof can be produced in an appropriate in vivo animal model and then isolated and/or purified.

Depending on the amino acid sequence of the constant domain of its heavy chains, immunoglobulins (Igs) can be assigned to different classes. Major classes of immunoglobulins can include: IgA, IgD, IgE, IgG, and IgM, and several of these may be further divided into subclasses (isotypes), e.g., IgG1, IgG2, IgG3, IgG4, IgA1 and IgA2. An Ig or portion thereof can, in some cases, be a human Ig. A CH3 domain can be from an immunoglobulin. In some cases, a chain or a part of an antibody or antigen binding fragment thereof, a modified antibody or antigen-binding fragment thereof, or a binding agent can be from an Ig. In such cases, an Ig can be IgG, an IgA, an IgD, an IgE, or an IgM. In cases where the Ig is an IgG, it can be a subtype of IgG, wherein subtypes of IgG can include IgG1, an IgG2a, an IgG2b, an IgG3, and an IgG4. In some cases, a CH3 domain can be from an immunoglobulin selected from the group consisting of an IgG, an IgA, an IgD, an IgE, and an IgM.

The "light chains" of antibodies (immunoglobulins) from any vertebrate species can be assigned to one of two clearly distinct types, called kappa ("x" or "K") or lambda ("λ"), based on the amino acid sequences of their constant domains.

A "variable region" of an antibody can refer to the variable region of the antibody light chain or the variable region of the antibody heavy chain, either alone or in combination. The variable regions of the heavy and light chain can consist of four framework regions (FR) connected by three complementarity determining regions (CDRs) also known as hypervariable regions. The CDRs in each chain can be held together in close proximity by the FRs and, with the CDRs from the other chain, contribute to the formation of the antigen-binding site of antibodies. CDRs can be determined by methods such as: (1) an approach based on cross-species sequence variability (i.e., Kabat et al., Sequences of Proteins of Immunological Interest, (5th ed., 1991, National Institutes of Health, Bethesda Md.)); and (2) an approach based on crystallographic studies of antigen-antibody complexes (Al-Iazikani et al. (1997) J. Molec. Biol. 273:927-948)). As used herein, a CDR may refer to CDRs defined by either approach or by a combination of both approaches.

With respect to antibodies, the term "variable domain" can refer to the variable domains of antibodies that are used in the binding and specificity of each particular antibody for its particular antigen. In some cases, the variability is not evenly distributed throughout the variable domains of antibodies. In some cases, it is concentrated in three segments called hypervariable regions (also known as CDRs) in both the light chain and the heavy chain variable domains. More highly conserved portions of variable domains can be called the "framework regions" or "FWRs" or "FRs." The variable domains of unmodified heavy and light chains can contain four FRs (FR1, FR2, FR3, and FR4), largely adopting a β-sheet configuration interspersed with three CDRs which can form loops connecting and, in some cases, part of the β-sheet structure. The CDRs in each chain can be held together in close proximity by the FRs and, with the CDRs from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669).

"Antibodies" useful in the present disclosure can encompass monoclonal antibodies, polyclonal antibodies, chimeric antibodies, bispecific antibodies, multispecific antibodies, heteroconjugate antibodies, humanized antibodies, human antibodies, deimmunized antibodies, mutants thereof, fusions thereof, immunoconjugates thereof, antigen-binding fragments thereof, and/or any other modified configuration of the immunoglobulin molecule that includes an antigen recognition site of the required specificity, including glycosylation variants of antibodies, amino acid sequence variants of antibodies, and covalently modified antibodies. An antibody can be a murine antibody.

An antibody can be a human antibody. As used herein, a "human antibody" can be an antibody having an amino acid sequence corresponding to that of an antibody produced by a human and/or that has been made using any suitable technique for making human antibodies. Human antibodies can include antibodies comprising at least one human heavy chain polypeptide or at least one human light chain polypeptide. One such example is an antibody comprising murine light chain and human heavy chain polypeptides. The human antibody may be selected from a phage library, where that phage library expresses human antibodies (Vaughan et al., 1996, Nature Biotechnology, 14:309-314; Sheets et al., 1998, PNAS USA, 95:6157-6162; Hoogenboom and Winter, 1991, J. Mol. Biol., 227:381; Marks et al., 1991, J. Mol. Biol., 222:581). Human antibodies can also be made by introducing human immunoglobulin loci into transgenic animals, e.g., mice in which the endogenous immunoglobulin genes have been partially or completely inactivated. This approach is described in U.S. Pat. Nos. 5,545,807; 5,545,806; 5,569,825; 5,625,126; 5,633,425; and 5,661,016. Alternatively, the human antibody may be prepared by immortalizing human B lymphocytes that produce an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., 1991, J. Immunol., 147 (1):86-95; and U.S. Pat. No. 5,750,373.

Any of the antibodies herein can be bispecific. Bispecific antibodies can be antibodies that have binding specificities for at least two different antigens and can be prepared using the antibodies disclosed herein. Exemplary methods for making bispecific antibodies are described (see, e.g., Suresh et al., 1986, Methods in Enzymology 121:210). The recombinant production of bispecific antibodies can be based on the coexpression of two immunoglobulin heavy chain-light chain pairs, with the two heavy chains having different specificities (Millstein and Cuello, 1983, Nature, 305, 537-539). Bispecific antibodies can be composed of a hybrid immunoglobulin heavy chain with a first binding specificity in one arm, and a hybrid immunoglobulin heavy chain-light chain pair (providing a second binding specificity) in the other arm. This asymmetric structure, with an immunoglobulin light chain in only one half of the bispecific molecule, can facilitate separation of the desired bispecific compound from unwanted immunoglobulin chain combinations. This approach is described, for example, in PCT Publication No. WO 94/04690.

Functional fragments of any of the antibodies herein are also contemplated. The terms "antigen-binding portion of an antibody," "antigen-binding fragment," "antigen-binding domain," "antibody fragment," or a "functional fragment of an antibody" can refer to one or more fragments of an antibody that retain the ability to specifically bind to an antigen. Representative antigen-binding fragments include a Fab, a Fab', a F(ab')2, a Fv, a scFv, a dsFv, a variable heavy domain, a variable light domain, a variable NAR domain, bi-specific scFv, a bi-specific Fab2, a tri-specific Fab3, an AVIMER^{®}, a minibody, a diabody, a maxibody, a camelid, a VHH, a minibody, an intrabody, fusion proteins comprising an antibody portion (e.g., a domain antibody), and a single chain binding polypeptide.

"F(ab')2" and "Fab'" moieties can be produced by treating an Ig with a protease such as pepsin and papain, and include antibody fragments generated by digesting immunoglobulin near the disulfide bonds existing between the hinge regions in each of the two heavy chains. For example, papain can cleave IgG upstream of the disulfide bonds existing between the hinge regions in each of the two heavy chains to generate two homologous antibody fragments in which an light chain composed of VL and CL (light chain constant region), and a heavy chain fragment composed of VH and CHγ1 (γ1) region in the constant region of the heavy chain) are connected at their C terminal regions through a disulfide bond. Each of these two homologous antibody fragments can be called Fab'. Pepsin can also cleave IgG downstream of the disulfide bonds existing between the hinge regions in each of the two heavy chains to generate an antibody fragment slightly larger than the fragment in which the two above-mentioned Fab' are connected at the hinge region. This antibody fragment can be called F(ab')2.

The Fab fragment can also contain the constant domain of the light chain and the first constant domain (CH1) of the heavy chain. Fab' fragments can differ from Fab fragments by the addition of a few residues at the carboxyl terminus of the heavy chain CH1 domain including one or more cysteine(s) from the antibody hinge region. Fab'-SH can be a Fab' in which the cysteine residue(s) of the constant domains bear a free thiol group. F(ab')2 antibody fragments can be produced, for example, as pairs of Fab' fragments which have hinge cysteines between them. Other chemical couplings of antibody fragments can also be employed.

A "Fv" as used herein can refer to an antibody fragment which contains a complete antigen-recognition and antigen-binding site. This region can consist of a dimer of one heavy chain and one light chain variable domain in tight, non-covalent or covalent association (disulfide linked Fvs have been described, see, e.g., Reiter et al. (1996) Nature Biotechnology 14:1239-1245). In this configuration that the three CDRs of each variable domain can interact to define an antigen-binding site on the surface of the VH-VL dimer. Collectively, a combination of one or more of the CDRs can from each of the VH and VL chains confer antigen-binding specificity to the antibody. For example, the CDRH3 and CDRL3 can be sufficient to confer antigen-binding specificity to an antibody when transferred to VH and VL chains of a recipient antibody or antigen-binding fragment thereof and this combination of CDRs can be tested for binding, specificity, affinity, etc. using, for example, techniques described herein. In some cases, even a single variable domain (or half of an Fv comprising only three CDRs specific for an antigen) can have the ability to recognize and bind antigen, although likely at a lower specificity or affinity than when combined with a second variable domain. Furthermore, although the two domains of a Fv fragment (VL and VH) can be coded for by separate genes, they can be joined using recombinant methods, for example by a synthetic linker that enables them to be made as a single protein chain in which the VL and VH regions pair to form monovalent molecules (known as single chain Fv (scFv); Bird et al.

(1988) Science 242:423-426; Huston et al. (1988) Proc. Natl. Acad. Sci. USA 85:5879-5883; and Osbourn et al. (1998) Nat. Biotechnol. 16:778). Such scFvs can be encompassed within the term "antigen-binding portion" of an antibody. Any VH and VL sequences of specific scFv can be linked to an Fc region cDNA or genomic sequences in order to generate expression vectors encoding complete Ig (e.g., IgG) molecules or other isotypes. VH and VL can also be used in the generation of Fab, Fv, or other fragments of Igs using either protein chemistry or recombinant DNA technology.

"Single-chain Fv" or "sFv" antibody fragments can include the VH and VL domains of an antibody, wherein these domains can be present in a single polypeptide chain. The Fv polypeptide can further include a polypeptide linker between the VH and VL domains which enables the sFv to form the desired structure for antigen binding. For a review of sFvs, see, e.g., Pluckthun in The Pharmacology of Monoclonal Antibodies, Vol. 113, Rosenburg and Moore eds. Springer-Verlag, New York, pp. 269-315 (1994).

Also contemplated herein is the use of an AVIMER^{®} as an antigen or antigen binding moiety. The term "AVIMER^{®}" can refer to a class of therapeutic proteins of human origin, which can be unrelated to antibodies and antibody fragments, and can be composed of several modular and reusable binding domains, referred to as A-domains (also referred to as class A module, complement type repeat, or LDL-receptor class A domain). They can be developed from human extracellular receptor domains by in vitro exon shuffling and phage display (Silverman et al., 2005, Nat. Biotechnol. 23:1493-1494; Silverman et al., 2006, Nat. Biotechnol. 24:220). The resulting proteins can contain multiple independent binding domains that can exhibit improved affinity and/or specificity compared with single-epitope binding proteins. Each of the known 217 human A-domains can include ~35 amino acids (~4 kDa); and these domains can be separated by linkers that can average five amino acids in length. Native A-domains fold quickly and efficiently to a uniform, stable structure mediated primarily by calcium binding and disulfide formation. A conserved scaffold motif of only 12 amino acids can be required for this common structure. The end result can be a single protein chain containing multiple domains, each of which represents a separate function. Each domain of the proteins can bind independently, and the energetic contributions of each domain can be additive.

Antigen-binding polypeptides can also include heavy chain dimers such as, for example, antibodies from camelids and sharks. Camelid and shark antibodies can include a homodimeric pair of two chains of V-like and C-like domains (neither has a light chain). Since the VH region of a heavy chain dimer IgG in a camelid does may not have to make hydrophobic interactions with a light chain, the region in the heavy chain that normally contacts a light chain can be changed to hydrophilic amino acid residues in a camelid. VH domains of heavy-chain dimer IgGs can be called VHH domains. Shark Ig-NARs can include a homodimer of one variable domain (termed a V-NAR domain) and five C-like constant domains (C-NAR domains). In camelids, the diversity of antibody repertoire can be determined by the CDRs 1, 2, and 3 in the VH or VHH regions. The CDR3 in the camel VHH region can be characterized by its relatively long length, averaging 16 amino acids (Muyldermans et al., 1994, Protein Engineering 7(9): 1129). This can be in contrast to CDR3 regions of antibodies of many other species. For example, the CDR3 of mouse VH can have an average of 9 amino acids. Libraries of camelid-derived antibody variable regions, which can maintain the in vivo diversity of the variable regions of a camelid, can be made by, for example, the methods disclosed in U.S. Patent Application Ser. No. 20050037421.

As used herein, a "maxibody" can refer to a bivalent scFv covalently attached to the Fc region of an immunoglobulin, see, e.g., Fredericks et al., Protein Engineering, Design & Selection, 17:95-106 (2004) and Powers et al., Journal of Immunological Methods, 251:123-135 (2001).

As used herein, a "dsFv" can be a Fv fragment obtained, for example, by introducing a Cys residue into a suitable site in each of a heavy chain variable region and a light chain variable region, and then stabilizing the heavy chain variable region and the light chain variable region by a disulfide bond. The site in each chain, into which the Cys residue can be introduced, can be determined based on a conformation predicted by molecular modeling. In the present disclosure, for example, a conformation can be predicted from the amino acid sequences of the heavy chain variable region and light chain variable region of the above-described antibody, and DNA encoding each of the heavy chain variable region and the light chain variable region, into which a mutation has been introduced based on such prediction, can be then constructed. The DNA construct can be incorporated then into a suitable vector and prepared from a transformant obtained by transformation with the aforementioned vector.

Single chain variable region fragments ("scFv") of antibodies are described herein. Single chain variable region fragments may be made by linking light and/or heavy chain variable regions by using a short linking peptide. Bird et al. (1988) Science 242:423-426. The single chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect, or mammalian cells, or prokaryotic, such as E. coli. Polynucleotides encoding the scFv of interest can be made by routine manipulations such as ligation of polynucleotides. The resultant scFv can be isolated using any suitable protein purification techniques.

Diabodies can be single chain antibodies. Diabodies can be bivalent, bispecific antibodies in which VH and VL domains can be expressed on a single polypeptide chain, but using a linker that is too short to allow for pairing between the two domains on the same chain, thereby forcing the domains to pair with complementary domains of another chain and creating two antigen binding sites (see, e.g., Holliger, P., et al., Proc. Natl. Acad. Sci. USA, 90:6444-6448 (1993); and Poljak, R. J., et al., Structure, 2:1121-1123 (1994)).

As used herein, a "minibody" can refer to a scFv fused to CH3 via a peptide linker (hingeless) or via an IgG hinge has been described in Olafsen, et al., Protein Eng Des Sel. April 2004; 17(4):315-23. As used herein, an "intrabody" can refer to a single chain antibody which can demonstrate intracellular expression and can manipulate intracellular protein function (Biocca, et al., EMBO J. 9:101-108, 1990; Colby et al., Proc Natl Acad. Sci. USA. 101:17616-21, 2004). Intrabodies, which can include cell signal sequences which can retain the antibody construct in intracellular regions, may be produced, for example, as described in Mhashilkar et al., (EMBO J., 14:1542-51, 1995) and Wheeler et al. (FASEB J. 17:1733-5. 2003). Transbodies can be cell-permeable antibodies in which a protein transduction domains (PTD) can be fused with single chain variable fragment (scFv) antibodies as in, for example, Heng et al. (Med Hypotheses. 64:1105-8, 2005).

### Computer Systems

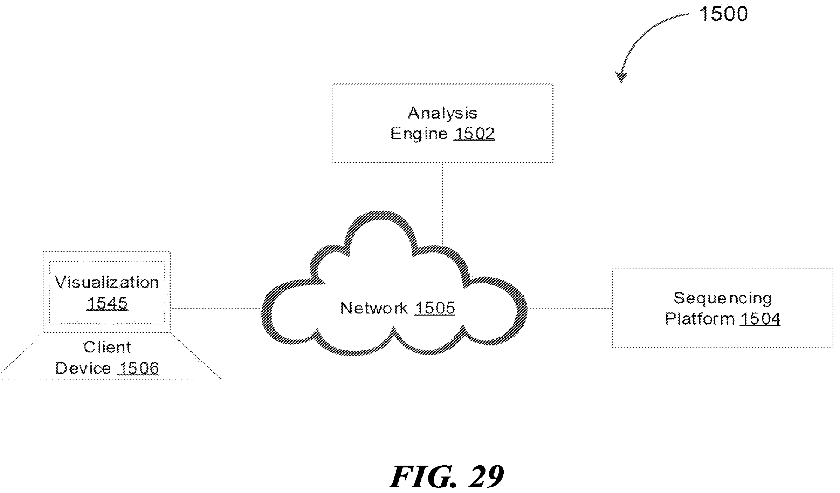

The present disclosure also provides computer systems configured to implement the various methods disclosed herein including, for example, methods to identify an antigen binding molecule, quantify a subject's response to an antigen, determine a diversity of a subject's immune response to an antigen, monitor a subject's response to an antibody therapeutic or antibody drug conjugate (ADC), characterize cells expressing one or more antigen binding molecules that bind to an antigen, identify an antibody therapeutic that does not elicit an immune response, and/or the like. FIG. 29 depicts a system diagram illustrating an example of an analysis system 1500. Referring to FIG. 29, the analysis system 1500 may include an analysis engine 1502, a sequencing platform 1504, and a client device 1506. As shown in FIG. 29, the analysis engine 1502, the sequencing platform 1504, and the client device 1506 may be communicatively coupled via a network 1505. The network 1505 may be a wired network and/or a wireless network including, for example, a local area network (LAN), a virtual local area network (VLAN), a wide area network (WAN), a public land mobile network (PLMN), the Internet, and/or the like.

Referring again to FIG. 29, the analysis engine 1502 may receive, from the sequencing platform 1504, data associated with spatially barcoded polynucleotides containing sequence information from, e.g., a spatial barcode sequence and a reporter oligonucleotide conjugated to each of a plurality of antigens. Alternatively, again referring to FIG. 29, the analysis engine 1502 may receive, from the sequencing platform 1504, data associated with a nucleic acid barcode molecule containing sequence information from a partition-specific barcode molecule and a reporter oligonucleotide conjugated to each of a plurality of antigens. As noted, the reporter oligonucleotide conjugated to an antigen may include a sequence, such as a reporter barcode, that enables an identification of the antigen. Conjugating each of the plurality of antigens with a reporter oligonucleotide that includes a reporter barcode may further enable a differentiation between different antigens, for example, during a multiplexed antigen assay. To further facilitate the processing and identification of the reporter barcode (e.g., through nucleic acid sequencing), the reporter oligonucleotide may be coupled with a capture probe that includes one or more of a spatial barcode, and optionally a template switching oligonucleotide (TSO) site.

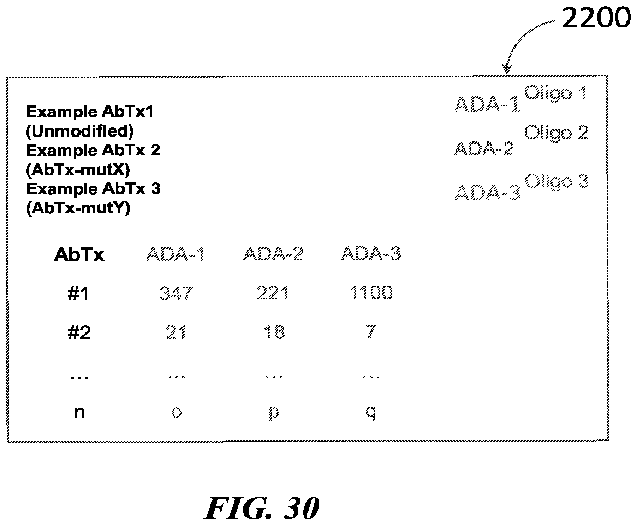

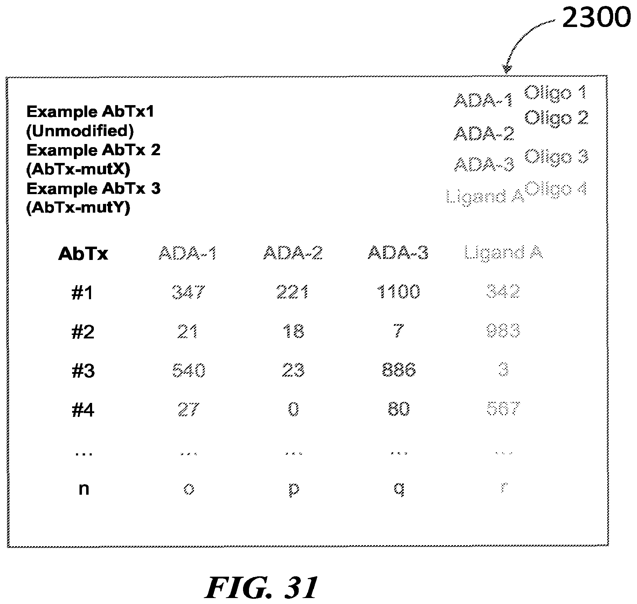

The analysis engine 1502 may generate, based at least on the data received from the sequencing platform 1504, a count matrix indicating a count of a quantity of times each of the plurality of antigens bound to each of a plurality of cells expressing one or more antigen binding molecules. The count matrix may contain information relating to, but not limited to, surface protein (non-antigen), intracellular protein (non-antigen), gene expression (targeted or untargeted RNA), DNA accessibility/ATAC, metabolite data, CRISPR guide, and/or DNA repair activity. To further illustrate, FIG. 25 depicts an example of a count matrix 1600 (see Example 7). Referring to FIG. 25, each row of the count matrix 1600 may correspond to one or more cells expressing an antigen binding molecule (e.g., mAb₁, mAb₂, and/or the like) while each column of the count matrix 1600 may correspond to an antigen (e.g., Ag₁, Ag₂, Ag₃, Ag₄, Ag₅, and/or the like). Accordingly, each element of the count matrix 1600 may correspond to a count of a quantity of times an antigen bound to the one or more cells expressing an antigen binding molecule. For example, according to the example of the count matrix 1600 shown in FIG. 25, indicates that a first antigen Ag₁ bound to a first feature corresponding to an antigen binding molecule mAb₁ 347 times while the first antigen Ag₁ bound to a second feature corresponding to an antigen binding module mAb₂ 21 times. The antigen binding molecule may be a secreted antibody. The antigen binding molecule may be a surface-bound antibody. FIG. 30 depicts another example of a count matrix 2200 (see Example 8). Referring to FIG. 30, each row of the count matrix 2200 may correspond to a cell expressing an antibody therapeutic (e.g., AbTx 1 (unmodified), AbTx 2 (containing an engineered mutation X),and AbTx 3 (containing an engineered mutation Y), while each column of the count matrix may correspond to an anti-drug antibody (ADA) identified according to the methods disclosed herein (e.g., ADA-1, ADA-2, ADA-3, and/or the like). Accordingly, each element of the count matrix may correspond to a count of a quantity of times an ADA bound to one or more cells expressing a particular AbTx. For example, according to the example of the count matrix shown in FIG. 30, indicates that a first ADA (ADA-1) bound to a first cell expressing AbTx 1 347 times while the ADA (ADA-1) bound to a second cell expressing AbTx 2 21 times. FIG. 31 depicts yet another example of a count matrix 2300 (see Example 9). Referring to FIG. 31, each row of the count matrix 2300 may correspond to a cell expressing an antibody therapeutic (e.g., AbTx 1 (unmodified), AbTx 2 (containing an engineered mutation X),and AbTx 3 (containing an engineered mutation Y), while each column of the count matrix may correspond to an anti-drug antibody (ADA) identified according to the methods disclosed herein (e.g., ADA-1, ADA-2, ADA-3, and/or the like, and a ligand A). Accordingly, each element of the count matrix may correspond to a count of a quantity of times an ADA bound to a cell expressing a particular AbTx. For example, according to the example of the count matrix shown in FIG. 31, indicates that a first ADA (ADA-1) bound to a first feature corresponding to AbTx 1 347 times while the ADA (ADA-1) bound to a second feature corresponding to AbTx 2 21 times.

The analysis engine 1502 may embed the count matrix, for example, the count matrix 1600, in a lower dimensional space in order to identify one or more distinct populations of cells expressing an antigen binding molecule capable of binding to one or more of a same antigen. The count matrix may provide high dimensional raw data as each cell expressing an antigen many be capable of binding to many different antigens, each of antigen adding to the dimensionality of the raw data. The high dimensionality of the data associated with the count matrix may obscure the similarity between populations of cells that are capable of binding to one or more of the same antigens. Embedding the count matrix in the lower dimensional space, which reduces the dimensionality of the data associated with the count matrix, may therefore enable the identification of one or more distinct populations of cells expressing an antigen binding molecule capable of binding to one or more of a same antigen.

The analysis engine 1502 may preprocess the count matrix prior to generating a reduced dimension representation of the count matrix. For example, the analysis engine 1502 may preprocess the count matrix by applying various techniques such as filtering, transforming, and/or denoising the data associated with the count matrix. The preprocessing of the count matrix may minimize the sources of unwanted variations in the data associated with the count matrix. Preprocessing the count matrix may therefore improve the performance of subsequent dimensionality reduction techniques such as, for example, principal component analysis (PCA), uniform manifold approximation and projection (UMAP), T-distributed Stochastic Neighbor Embedding (t-SNE), Poincare disks, and/or the like.

The analysis engine 1502 may preprocess the count matrix by at least filtering the count matrix to retain one or more cells expressing one or more antigen binding molecules having a detected variability, diversity, and joining (VDJ) sequence and/or an antibody sequence, a non-zero binding count, and a sufficient sequencing depth (e.g., 5,000 to 20,000 reads per cell for antigen and VDJ libraries).

Furthermore, the analysis engine 1502 may preprocess the count matrix, for example, the filtered count matrix, by calculating an ambient concentration (e.g., in mean, median, hypergeometric mean, and/or the like) of one or more antigens and/or the reporter oligonucleotides conjugated to the antigens as well as an deviation for non-cell-associated (e.g., empty) barcodes. The count matrix may be further preprocessed by applying a transformation, adding a pseudocount, subtracting the ambient concentration, and scaling. Examples of transformations may include variance stabilizing transformation, square root transformation, cubic root transformation, and log transformation. The analysis engine 1502 may apply the transformation to each element that is included in the filtered count matrix before adding a pseudocount (e.g., ranging from 1 to 30), subtracting the ambient concentration, and scaling (e.g., dividing) by the deviation or variance of the corresponding antigen. The analysis engine 1502 may further modify the count matrix by fitting the count vector for each cell to a mixture model (e.g., a Gaussian mixture model) including one or more covariates such as a quantity of detected reads, a quantity of detected genes, a quantity of detected gene unique molecular identifiers (UMIs), a quantity of detected antigens, a quantity of detected antigen unique molecular identifiers (UMIs), a quantity of detected surface or intracellular protein unique molecular identifiers, a quantity of detected ATAC peaks, a quantity of detected surface proteins, a quantity of detected intracellular proteins, B-cell phenotypes, an IgG constant region, a quantity of mutations in antibody sequence, sequencing depth, quantity of detected unique molecular identifiers (UMIs), cell annotations, and/or the like.

To further illustrate, referring again to FIG. 25 and the example of the count matrix 1600, once the count matrix 1600 is filtered to retain, for example, at least the first cell mAb₁ and the second cell mAb₂, the analysis engine 1502 may further preprocess the count matrix 1600 by calculating an ambient concentration of each of the first antigen Ag₁, the second antigen Ag₂, the third antigen Ag₃, the fourth antigen Ag₄, the fifth antigen Ag₅, and/or the like. The analysis engine 1502 may further transform each element in the count matrix 1600 before adding a pseudocount (e.g., ranging from 1 to 30), subtracting the ambient concentration, and scaling (e.g., dividing) by the deviation or variance of the corresponding antigen. For example, the analysis engine 1502 may transform the count indicating the first antigen Ag₁ bound to the first feature corresponding to mAb₁ 347 times by applying a variance stabilizing transformation, a square root transformation, a cubic root transformation, a log transformation, and/or the like. The analysis engine 1502 may add a pseudocount (e.g., ranging from 1 to 30), subtract the ambient concentration (e.g., of the first antigen Ag₁), and scale (e.g., divide) by the deviation or variance of the first antigen Ag₁. Moreover, the analysis engine 1502 may further modify the count matrix 1600 by fitting the count vector associated with each of the first feature corresponding to mAb₁ and the second feature corresponding to mAb₂, which includes the quantity of times each cell bound to each of the antigens Ag₁, Ag₂, Ag₃, Ag₄, Ag₅, to a mixture model (e.g. a Gaussian mixture model and/or the like) that includes various covariates such as a quantity of detected reads, a quantity of detected genes, a quantity of detected gene unique molecular identifiers (UMIs), a quantity of detected antigens, a quantity of detected antigen unique molecular identifiers (UMIs), a quantity of detected surface or intracellular protein unique molecular identifiers, a quantity of detected ATAC peaks, a quantity of detected surface proteins, a quantity of detected intracellular proteins, B-cell phenotypes, an IgG constant region, a quantity of mutations in antibody sequence, sequencing depth, quantity of detected unique molecular identifiers (UMIs), cell annotations, and/or the like.

Alternatively, the analysis engine 1502 may preprocess the count matrix, for example, the filtered count matrix, by calculating an ambient concentration (e.g., in mean, median, hypergeometric mean, and/or the like) of one or more antigens and/or the reporter oligonucleotides conjugated to the antigens before subtracting the ambient concentration from each antigen included in the count matrix (e.g., antigens Ag₁, Ag₂, Ag₃, Ag₄, and Ag₅ in the count matrix 1600). The analysis engine 1502 may, optionally, add a pseudocount (e.g., ranging from 1 to 50) before applying a transformation such as, for example, a variance stabilizing transformation, a square root transformation, a cubic root transformation, a log transformation, and/or the like.

The analysis engine 1502 may generate a reduced dimension representation of the count matrix by applying, to the preprocessed count matrix, one or more dimensionality reduction techniques such as, for example, principal component analysis (PCA), uniform manifold approximation and projection (UMAP), T-distributed Stochastic Neighbor Embedding (t-SNE), Poincare disks, and/or the like. Generating the reduced dimension representation of the count matrix may enable the analysis engine 1502 to generate, for example, for display at the client device 1506, a corresponding visualization 1545 of the count matrix and/or the populations of cells included in the count matrix. Further application of a clustering technique (e.g., spectral clustering and/or the like) or a community detection algorithm (e.g., granularity-based community detection, resolution-based community detection, and/or the like) may enable the analysis engine 1502 to generate the visualization 1545 to depict groups of cells that have been separated into distinct populations of cells having a similar binding profile (e.g., capable of binding to one or more of a same antigen).

FIG. 26 depicts an example of the visualization 1545, which may be presented as part of a graphic user interface (GUI) at the client device 1506. The example of the visualization 1545 may be generated based on the example of the count matrix 1600 shown in FIG. 25. For example, the visualization 1545 may be generated based on a reduced dimension representation of the count matrix 1600 generated by applying, to the count matrix 1600, one or more of a principal component analysis (PCA), a uniform manifold approximation and projection (UMAP), a T-distributed Stochastic Neighbor Embedding (t-SNE), Poincare disks, and/or the like. The visualization 1545 may be further generated by applying a clustering technique (e.g., spectral clustering and/or the like) or a community detection algorithm (e.g., granularity based community detection, resolution based community detection). Accordingly, the visualization 1545 may depict distinct populations of cells having a similar binding profile (e.g., capable of binding to one or more of a same antigen) including, for example, a first population of cells 1702 having a similar binding profile as the first cell mAb₁ and a second population of cells 1704 having a similar binding profile as the second cell mAb₂.

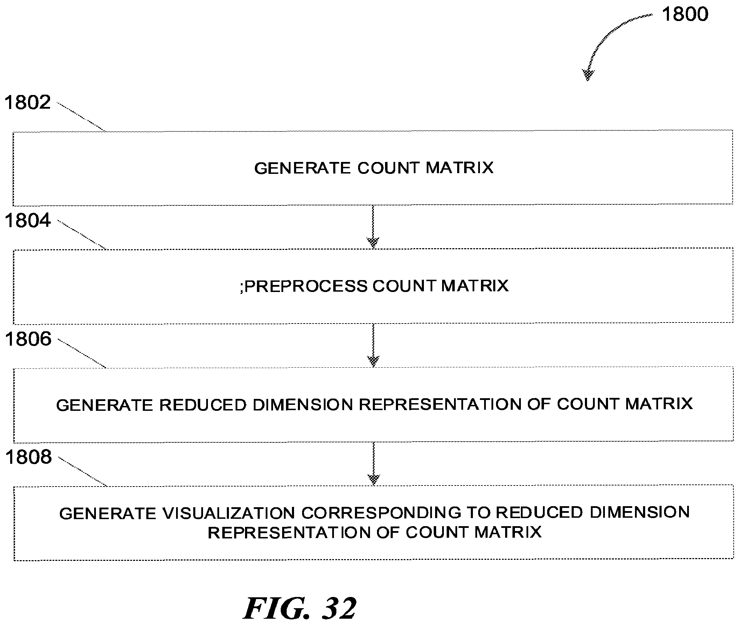

FIG. 32 depicts a flowchart illustrating an example of a process 1800 for analyzing and visualizing data associated with cells expressing one or more antigen binding molecules. Referring to FIGS. 29 and 32, the process 1800 may be performed by the analysis engine 1502 to generate, analyze, and visualize, for example, the count matrix 1600, which may include data indicating a quantity of an antigen (e.g., Ag₁, Ag₂, Ag₃, Ag₄, Ag₅, and/or the like) bound to one or more features associated with an antigen binding molecule (e.g., the first feature associated with mAb₁, the second feature associated with mAb₂, and/or the like).

At 1802, the analysis engine 1502 may generate a count matrix. For example, the analysis engine 1502 may receive, from the sequencing platform 1504, data associated with spatially barcoded polynucleotides or amplicons thereof containing sequence information from a spatial barcode sequences and a reporter oligonucleotide conjugated to each of a plurality of antigens. The reporter oligonucleotide conjugated to an antigen may include a unique sequence, such as a reporter barcode, that enables an identification of the antigen as well as a differentiation between different antigens. As such, the analysis engine 1502 may generate, based at least on the data received form the sequencing platform 1504, a count matrix indicating a count of a quantity of times each of the plurality of antigens bound to each of a plurality of cells expressing one or more antigen binding molecules. For instance, in the example of the count matrix 1600 shown in FIG. 25, the first antigen Ag₁ bound to the first cell mAb₁ 347 times and to the second cell mAb₂ 21 times.

At 1804, the analysis engine 1502 may preprocess the count matrix. The analysis engine 1502 may, prior to generating a reduced dimension representation of the count matrix, preprocess the count matrix by applying various techniques such as such as filtering, transforming, and/or denoising the data associated with the count matrix. The analysis engine 1502 may preprocess the count matrix by at least filtering the count matrix to retain one or more cells expressing one or more antigen binding molecules having a detected variability, diversity, and joining (VDJ) sequence and/or an antibody sequence, a non-zero binding count, and a sufficient sequencing depth (e.g., 5,000 to 20,000 reads per cell for antigen and VDJ libraries). Moreover, the analysis engine 1502 may preprocess the count matrix, for example, the filtered count matrix, by calculating an ambient concentration (e.g., in mean, median, hypergeometric mean, and/or the like) of one or more antigens and/or the reporter oligonucleotides conjugated to the antigens. The analysis engine 1502 may apply a transformation (e.g., a variance stabilizing transformation, a square root transformation, a cubic root transformation, a log transformation, and/or the like) to each cell that is included in the filtered count matrix, add a pseudocount (e.g., ranging from 1 to 30), subtract the ambient concentration, and/or scale (e.g., divide) by the deviation or variance of the corresponding antigen. Alternatively and/or additionally, the analysis engine 1502 may modify the count matrix by fitting the count vector for each cell to a mixture model (e.g., a Gaussian mixture model) including one or more covariates such as a quantity of detected reads, a quantity of detected genes, a quantity of detected gene unique molecular identifiers (UMIs), a quantity of detected antigens, a quantity of detected antigen unique molecular identifiers (UMIs), a quantity of detected surface or intracellular protein unique molecular identifiers, a quantity of detected ATAC peaks, a quantity of detected surface proteins, a quantity of detected intracellular proteins, B-cell phenotypes, an IgG constant region, a quantity of mutations in antibody sequence, sequencing depth, quantity of detected unique molecular identifiers (UMIs), cell annotations, and/or the like.

At 1806, the analysis engine 1502 may generate a reduced dimension representation of the count matrix. For example, the analysis engine 1502 may generate a reduced dimension representation of the count matrix by applying, to the preprocessed count matrix, one or more dimensionality reduction techniques such as principal component analysis (PCA), uniform manifold approximation and projection (UMAP), T-distributed Stochastic Neighbor Embedding (t-SNE), Poincare disks, and/or the like. As noted, the high dimensionality of the raw data associated with the count matrix may obscure the similarity between populations of cells that are capable of binding to one or more of the same antigens. Reducing the dimensionality of the data associated with the count matrix, for example, by generating a corresponding reduced dimension representation, may therefore enable the identification of one or more distinct populations of cells expressing an antigen binding molecule capable of binding to one or more of a same antigen.

At 1808, the analysis engine 1502 may generate a visualization corresponding to the reduced dimension representation of the count matrix. For example, the analysis engine 1502 may generate, based on the reduced dimension representation of the count matrix, the visualization 1545 to depict the count matrix and/or the populations of cells included in the count matrix. By applying a clustering technique (e.g., spectral clustering and/or the like) or a community detection algorithm (e.g., granularity based community detection, resolution based community detection), the analysis engine 1502 may further generate the visualization 1545 to depict groups of cells having a similar binding profile (e.g., capable of binding to one or more of a same antigen). For instance, the example of the visualization 1545 shown in FIG. 26 may depict the first population of cells 1702 having a similar binding profile as the first cell mAb₁ and the second population of cells 1704 having a similar binding profile as the second cell mAb₂. The visualization 1545 may be presented as part of the graphic user interface (GUI) at the client device 1506.

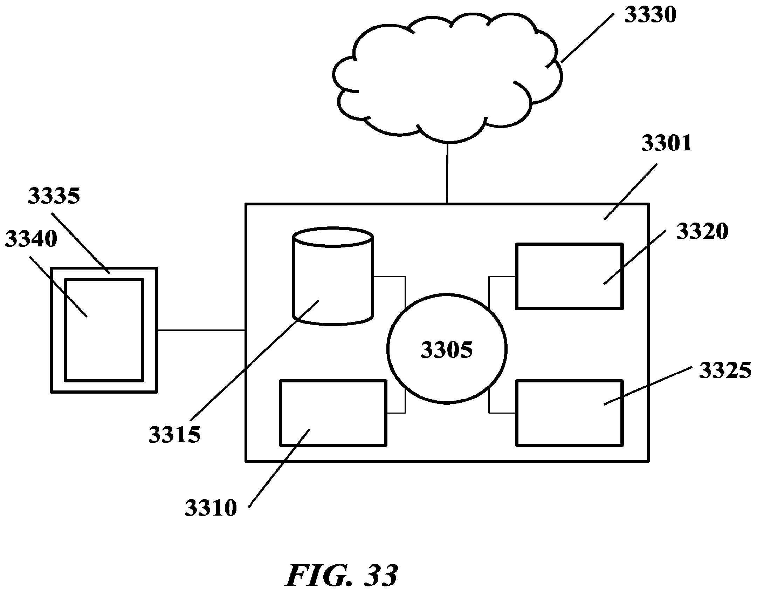

FIG. 33 depicts a block diagram illustrating an example of a computer system 3301. Referring to FIGS. 33 and 29, the computer system 3301 may be configured to implement one or more of the analysis engine 1502, the sequencing platform 1504, and the client device 1506. The computer system 3301 can be an electronic device of a user or a computer system that is remotely located with respect to the electronic device. The electronic device can be a mobile electronic device.

The computer system 3301 includes a central processing unit (CPU, also "processor" and "computer processor" herein) 3305, which can be a single core or multi core processor, or a plurality of processors for parallel processing. The computer system 3301 also includes memory or memory location 3310 (e.g., random-access memory, read-only memory, flash memory), electronic storage unit 3315 (e.g., hard disk), communication interface 3320 (e.g., network adapter) for communicating with one or more other systems, and peripheral devices 3325, such as cache, other memory, data storage and/or electronic display adapters. The memory 3310, storage unit 3315, interface 3320 and peripheral devices 3325 are in communication with the CPU 3305 through a communication bus (solid lines), such as a motherboard. The storage unit 3315 can be a data storage unit (or data repository) for storing data. The computer system 3301 can be operatively coupled to a computer network ("network") 3330 with the aid of the communication interface 3320. The network 3330 can be the Internet, an internet and/or extranet, or an intranet and/or extranet that is in communication with the Internet. The network 3330 in some cases is a telecommunication and/or data network. The network 3330 can include one or more computer servers, which can enable distributed computing, such as cloud computing. The network 3330, in some cases with the aid of the computer system 3301, can implement a peer-to-peer network, which may enable devices coupled to the computer system 3301 to behave as a client or a server.

The CPU 3305 can execute a sequence of machine-readable instructions, which can be embodied in a program or software. The instructions may be stored in a memory location, such as the memory 3310. The instructions can be directed to the CPU 3305, which can subsequently program or otherwise configure the CPU 3305 to implement methods of the present disclosure. Examples of operations performed by the CPU 3305 can include fetch, decode, execute, and writeback.

The CPU 3305 can be part of a circuit, such as an integrated circuit. One or more other components of the system 3301 can be included in the circuit. In some cases, the circuit is an application specific integrated circuit (ASIC).

The storage unit 3315 can store files, such as drivers, libraries and saved programs. The storage unit 3315 can store user data, e.g., user preferences and user programs. The computer system 3301 in some cases can include one or more additional data storage units that are external to the computer system 3301, such as located on a remote server that is in communication with the computer system 3301 through an intranet or the Internet.

The computer system 3301 can communicate with one or more remote computer systems through the network 3330. For instance, the computer system 3301 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 3301 via the network 3330.

Methods as described herein can be implemented by way of machine (e.g., computer processor) executable code stored on an electronic storage location of the computer system 3301, such as, for example, on the memory 3310 or electronic storage unit 3315. The machine executable or machine readable code can be provided in the form of software. During use, the code can be executed by the processor 3305. In some cases, the code can be retrieved from the storage unit 3315 and stored on the memory 3310 for ready access by the processor 3305. In some situations, the electronic storage unit 3315 can be precluded, and machine-executable instructions are stored on memory 3310.

The code can be pre-compiled and configured for use with a machine having a processor adapted to execute the code, or can be compiled during runtime. The code can be supplied in a programming language that can be selected to enable the code to execute in a pre-compiled or as-compiled fashion.

The systems and methods described herein, such as the computer system 3301, can be embodied in programming. Various examples of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that include a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 3301 can include or be in communication with an electronic display 3335 that includes a user interface (UI) 3340 for providing, for example, results of the assay, such as a summary of one or more antigen binding molecules that bind the antigens, a summary of one or more antigens not bound by an antigen binding molecule in the composition, a site on the antigen that binds to the antigen binding molecule, or proposed modifications to the antigen that can reduce affinity of the antigen binding fragment for the antigen. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit 3305. The algorithm can, for example, contact an antigen with an antigen binding molecule, isolate the antigen binding molecule, or identify the antigen binding molecule as described herein. An algorithm can determine a site on the antigen binding to the antigen binding molecule, such as an epitope. An algorithm can determine a modification of an antigen or to modify an antigen to reduce affinity of the antigen binding molecule for the antigen.

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) from a single cell. For example, a biological particle (e.g., a cell or cell bead) is partitioned in a partition (e.g., droplet), and multiple analytes from the biological particle are processed for subsequent processing. The multiple analytes may be from the single cell. This may enable, for example, simultaneous proteomic, transcriptomic and genomic analysis of the cell.

No admission is made that any reference cited herein constitutes prior art. The discussion of the references states what their authors assert, and the Applicant reserves the right to challenge the accuracy and pertinence of the cited documents. It will be clearly understood that, although a number of information sources, including scientific journal articles, patent documents, and textbooks, are referred to herein; this reference does not constitute an admission that any of these documents forms part of the common general knowledge in the art.

### EXAMPLES

### Example 1: Identification of mutations in therapeutic antibodies or antibody-drug conjugates

One or more mutations in therapeutic antibodies or antibody-drug conjugates that eliminate the recognition of the mutated therapeutic antibodies or antibody-drug conjugates comprising such mutations by the preexisting anti-drug antibodies can be determined by methods provided herein.

A count matrix of cells and drug-reactive antibodies conjugated to reporter oligonucleotides is derived. In some embodiments, a count matrix of cells expressing drug-reactive antibodies, and therapeutic antibodies conjugated to reporter oligonucleotides is derived. In some embodiments, a count matrix of cells expressing unmodified or mutated therapeutic antibodies and drug-reactive antibodies conjugated to reporter oligonucleotides is derived. In some embodiments, a count matrix of cells expressing drug-reactive antibodies, therapeutic antibodies conjugated to reporter oligonucleotides, and therapeutic ligands conjugated to reporter oligonucleotides is derived. In some embodiments, a count matrix of cells expressing mutated therapeutic antibodies, drug-reactive antibodies conjugated to reporter oligonucleotides, and therapeutic ligands conjugated to reporter oligonucleotides is derived. The count matrix provides data regarding cells (*e.g.,* T cells or B cells) from a composition that containing antibodies that can bind to a therapeutic antibody, antibody-drug conjugate, control antigen, viral antigen, or other etiologic antigen, such as a cancer antigen, control antigen, or decoy antigen.

In some embodiments, cells are filtered to retain cells that have both a detected VDJ/antibody sequence and non-zero drug-reactive antibody counts and sequenced to an appropriate depth. In some embodiments, cells are sequenced at approximately 5,000 to 100,000 reads per cell for both antigen and VDJ libraries. In some embodiments, cells are sequenced at approximately 5,000 to 20,000 reads per cell for both antigen and VDJ libraries. In some embodiments, cells are sequenced at approximately 20,000 to 40,000 reads per cell for both antigen and VDJ libraries. In some embodiments, cells are sequenced at approximately 40,000 to 60,000 reads per cell for both antigen and VDJ libraries. In some embodiments, cells are sequenced at approximately 60,000 to 80,000 reads per cell for both antigen and VDJ libraries. In some embodiments, cells are sequenced at approximately 80,000 to 100,000 reads per cell for both antigen and VDJ libraries. In some embodiments, saturation can occur rapidly.

The count matrix is retained from cells that are removed. For each antigen, background concentration and ambient concentration are calculated (e.g., mean, median, or hypergeometric mean), and appropriate deviation for non-cell-associated (empty barcodes) is calculated. For a given cell retained by the filter, a logarithm (e.g., natural, base 10, or base 2) or similar transformation (e.g., variance stabilizing transformation, square root, or cubic root) is performed, a pseudocount (e.g., ranging from 1-30) is added, and the background is subtracted. The value is scaled (e.g., by division) by the deviation or variance of the given antigen. Further, the value is modified using a Gaussian mixture model for each antigen on a count vector for each cell, and can include, for example, but not limited to, covariates for sequencing depth or number of detected unique molecular identifiers, cell annotations, or other technical or biological covariates, such as the expression level of the BCR within the cell.

Alternatively, in some embodiments, background and/or ambient concentration is calculated in empty droplets, and subtracted for each antigen. A pseudocount is added (e.g., ranging from 1-50) in some embodiments. The center log ratio or other appropriate compositional transformation can then be applied.

In some embodiments, the resulting matrix and cellular populations thereof are visualized using standard dimensional reduction models, for example PCA, UMAP, t-SNE, or Poincare disks. Further application of spectral clustering, granularity/resolution-based/community detections algorithms, or other algorithms are used to separate cells into groups of cells with distinct drug reactive antibody profiles.

### Example 2: Enzyme-tagged therapeutic antibodies or antibody-drug conjugates

Methods provided herein can be used for enzyme-tagged therapeutic antibodies or antibody-drug conjugates, wherein the therapeutic antibodies or antibody-drug conjugates are tagged for example with a sortase motif (LPXTG). A therapeutic antibody or antibody-drug conjugate is bound by a cell expressing an anti-drug antibody and captured. Incubation with a protein tag or peptide tag including an N-terminal glycine and addition of a bacterial SrtA protein or an engineered variant thereof (e.g., P94, D160N, or K196T) confers a protein tag which is recognized by a barcoded antibody with an additional round of staining. For example, the covalently or non-covalently attached protein tag is selected from BCCP (biotin carboxyl carrier protein) tag, glutathione-S-transferase tag, green fluorescent protein tag, halo-tag, SNAP tag, CLIP tag, HUH tag, maltose binding protein tag, Nus tag, thioredoxin tag, Fc tag, and CRDSAT tag. For example, the covalently or non-covalently attached peptide tag is selected from ALFA tag, AviTag, C-tag, calmodulin tag, polyglutamate tag, polyarginine tag, E tag, FLAG tag, HA tag, His tag, Myc tag, NE tag, Rho1D4 tag, S tag, SBP tag, Softag 1, Softag 3, Spot tag, Strep tag, T7 tag, TC tag, Ty tag, V5 tag, VSV tag, Xpress tag, Isopeptag, Spy tag, Snoop tag, DogTag, and SdyTag.

### Example 3: Capture of anti-drug antibodies using a splint oligonucleotide

In some embodiments, a splint oligonucleotide is used to capture and/or detect both an anti-drug antibody RNA transcript and genomic DNA. In some embodiments, RTX polymerase is used to produce a clonable and uniquely amplifiable anti-drug antibody VDJ sequence that is cell associated. Such sequences are amplified after next-generation sequencing and analysis, and can enable rapid production of anti-drug antibodies for research or screening purposes.

### Example 4: Efficacy validation and re-engineering therapeutic antibodies and antibody drug conjugates

Anti-drug antibodies isolated using a method provided herein can be used to validate the efficacy of a therapeutic antibody or antibody-drug conjugate re-engineering. By expressing and labeling the anti-drug antibodies with oligonucleotides and staining engineered cells expressing mutants of the therapeutic antibody or antibody drug conjugate of choice, the workflow described in example 1 is used to confirm that the new therapeutic antibody or antibody-drug variant is not bound by the same antibodies as the original therapeutic antibody or antibody-drug variant. Furthermore, use of such antibodies in functional assays (e.g., ADCC, ADCP, ADCD, or other assays) are used to identify anti-drug antibodies with therapeutic value. For example, such anti-drug antibodies are developed as drugs to be administered to patients experiencing side effects induced by a therapeutic antibody or antibody-drug conjugate by neutralizing the drug or promoting its destruction. Such anti-drug antibodies can also be deployed as reference standards in traditional serum-based anti-drug antibody assays. In some embodiments, this can be effective for therapeutic antibodies or antibody-drug conjugates that have been configured to be recycled efficiently, such as by the addition of one or more Fc domains that are recognized by a neonatal Fc receptor (FcRn, product of the FCGRT gene). Antigens more broadly can also be used to characterize the response in patients with diverse immunoglobulin haplotypes, and in combination with traditional statistical models such as contingency tests, GWAS, PheWAS, and/or cellular assays discover genomic or other elements associated with the development of anti-drug antibodies.

### Example 5: Identification of re-engineered antigens recognized by antigen binding molecules

The methods provided herein can be implemented to perform an experiment with 5 different point mutants of adalimumab (FIG. 25). Following the procedure of the method, each point mutant is labeled with its own unique reporter barcode oligonucleotide population. The presence of 2 distinct populations of single cells in the resulting clustering and visualization analyses indicates that these populations of cells can recognize distinct (e.g., disjoint) sets of the 5 antigens used (FIG. 26). Mutation or masking of the identified amino acids in the parent antigen (i.e., adalimumab) ablates recognition of the drug by patient derived anti-drug antibodies, guiding the drug engineering and development process accordingly. Optionally, a re-engineered antibody is knocked into a cell line, expressed, and purified to produce a new antigen (e.g., adalimumab variant) to be used in the same patient samples.

### Example 6: Analysis of anti-drug antibodies (ADA) or drug-reactive antibodies (DRA)

The methods provided herein can be implemented to isolate and characterize anti-drug antibodies (ADAs) or drug-reactive antibodies (DRAs) via barcoding. For example, an antigen conjugated to a reporter molecule is used to profile samples from a subject treated with an antibody-drug conjugate (ADC) and/or an antibody therapeutic (AbTx). An exemplary workflow for associating the reporter molecule with an antibody (e.g., an ADA, an ADC, or an AbTx) is shown in FIG. 21. For example, the reporter molecule 2120 is conjugated with e.g., an antibody-binding protein (e.g., Protein A or Protein G), and the complex 2130 is formed by non-covalent association between the antibody binding protein and the ADC and/or the AbTX. The complex 2130 is incubated with a tissue sample, e.g., a tissue sample comprising one or more immune receptor expressing cells, and unbound antibody can be washed out. The complex 2130 can binds to a cell, e.g., an immune receptor expressing cell of the tissue sample, thereby providing a labelled immune receptor expressing cell. The tissue sample can be subjected to spatial analysis according to a method disclosed herein.

### Example 7: Identification of anti-drug antibodies (ADAs)

The methods provided herein can be implemented to identify ADAs to an AbTx via barcoding. An exemplary workflow of identifying ADAs is described in FIG. 27A.

A tissue sample is obtained from a patient treated with a known AbTx.

The known AbTx is conjugated to a reporter oligonucleotide according to the methods described herein. In some embodiments, the AbTx can also be labeled with a reporter fluorophore or any other detectable labels that can enable identification of the AbTx by, for example, flow cytometry or any other known similar methods. In some embodiments, the AbTx is pre-incubated with its labeled antigenic target (AgTx), enabling identification of ADAs binding to AbTx-AgTx complex rather than the AbTx alone.

The tissue sample is incubated with the reporter oligonucleotide conjugated AbTx. In some embodiments, the tissue sample is contacted with a reporter oligonucleotide conjugated AbTx panel comprising unmodified and modified (or mutated) AbTx conjugated with reporter oligonucleotides. In some embodiments, the tissue sample can also be contacted with a panel of additional labeling agents comprising, e.g., reporter oligonucleotide antibody panels for cell surface markers used, e.g., for cell classification. The tissue sample is processed for spatial analysis according to a method disclosed herein. For example, the tissue sample can be contacted with a substrate comprising an array of capture probes. Analytes from an ABM-expressing cell of the tissue sample, as well as reporter oligonucleotides from the AbTx panel, and optionally reporter oligonucleotides from the panel of additional labeling agents, are attached to capture probes disclosed herein, e.g., according to a spatial analysis method disclosed herein. This can generate a reporter barcode library, a V(D)J library, and optionally a gene expression library. The reporter barcode library can include spatially barcoded polynucleotides or amplicons or library members thereof comprising (i) a reporter barcode sequence or reverse complement thereof and (ii) a spatial barcode sequence or reverse complement thereof. The V(D)J library can include native sequences of ABMs that bind an AbTx, expressed antibody sequences in engineered cells.

The generated libraries are sequenced. Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}).

A count matrix of cells expressing ADAs and reporter barcodes associated with the AbTx or AbTx-AgTx complex is generated from the sequence data. In some embodiments, the count matrix is a count matrix described in FIG. 25, wherein each row corresponds to a cell expressing an antigen binding molecule (i.e., an ADA), and each column corresponds to an antigen (i.e., the known AbTx (unmodified), one or more modified (mutated) AbTx with at least one point mutation, or the AbTx-AgTx complex). Each element of the count matrix described in FIG. 25 corresponds to a count of quantity of times the antigen bound to a cell expressing a particular antigen binding molecule (i.e., a particular ADA). BCR or TCR sequences from cells having a threshold number of AbTx reporter oligonucleotide UMI counts are used to identify the particular ADA bound to the unmodified AbTx, modified AbTx, or the AbTx-AgTx complex.

### Example 8: Identification of a patient specific engineered antibody therapeutic (AbTx)

The methods provided herein can be implemented to identify a patient specific engineered AbTx that does not elicit an immune response in the patient. An exemplary workflow of identifying the patient specific AbTx is described in FIG. 24B.

The ADAs identified in Example 7 are conjugated to reporter oligonucleotides as described herein. In some embodiments, the ADAs can also be labeled with a reporter fluorophore or any other detectable labels that can enable identification of the ADA by, for example flow cytometry or any other known similar methods.

Cells are engineered to express the AbTx and/or the AbTx comprising one or more point mutations. In some embodiments, the AbTx and/or the modified (mutated) AbTx are tethered to the cell surface when expressed from constructs comprising the 5th exon, but not the 6th exon, of an IgG1 constant region. In some embodiments, the AbTx and/or the modified (mutated) AbTx are secreted when expressed from constructs comprising the 6th exon, but not the 5th exon, of an IgG1 constant region. In some embodiments, the engineered cells expressing the AbTx or the modified (mutated) AbTx are pre-incubated with its labeled antigenic target (AgTx), thereby forming a AbTx-AgTx complex. In some embodiments, the engineered cells are enriched in order to identify a modified (mutated) AbTx with a low ADA activity or a high ADA activity.

The engineered cells are incubated with the reporter oligonucleotide conjugated ADAs. In some embodiments, the engineered cells are contacted with a panel of reporter oligonucleotide conjugated ADAs and a control antibody.

The engineered cells bound to the reporter oligonucleotide conjugated ADAs are partitioned and subjected to, for example, 10x 5'V2 Single Cell Immune Profiling kit per manufacturer's instructions. Additional information in this regard can be found at "support.10xgenomics.com/permalink/getting-started-immune-profiling-feature-barcoding." This can generate a reporter barcode library, a V(D)J library, and optional a gene expression library. The AbTx or modified (mutated) AbTx are detected by V(D)J chemistry or by targeted GEX chemistry. Reporter barcoded ADAs are detected by the reporter barcode chemistry. Reporter barcoded AbTx-AgTx can be detected by the reporter barcode chemistry.

Optionally, the libraries are enriched for targets of interest, which includes any combination of ADA antibody sequences identified using methods disclosed herein, and known AbTx or AbTx mutant sequences. An exemplary target enrichment method may comprise providing a plurality of barcoded nucleic acid molecules (e.g., library members) and hybridizing barcoded nucleic acid molecules comprising targeted regions of interest to oligonucleotide probes ("baits") which are complementary to the targeted regions of interest (or to regions near or adjacent to the targeted regions of interest). Baits may be attached to a capture molecule, including without limitation a biotin molecule. The capture molecule (e.g., biotin) can be used to selectively pull down the targeted regions of interest (for example, with magnetic streptavidin beads) to thereby enrich the resultant population of barcoded nucleic acid molecules for those containing the targeted regions of interest.

The generated libraries are sequenced on, for example, a NovaSeq 3 using a NovaSeq S4 200 cycles 2020 v1.5 kit per manufacturer's instructions. Sequencing can also be performed by various systems currently available, such as, without limitation, a sequencing system by Illumina^{®}, Pacific Biosciences (PacBio^{®}), Oxford Nanopore^{®}, or Life Technologies (Ion Torrent^{®}). The binding affinity of ADAs to the AbTx, the modified (mutated) AbTx, or the AbTx-AgTx complex are determined based on quantity/numbers of unique molecular identifiers (UMIs) associated with each of the antigen binding molecules bound to the target antigen.

A count matrix of cells expressing the AbTx or the modified (mutated) AbTx and reporter barcodes associated with the ADAs is generated. In some embodiments, the count matrix is a count matrix described in FIG. 30, wherein each row corresponds to a cell expressing an antigen binding molecule (i.e., the known AbTx (unmodified), one or more modified (mutated) AbTx with at least one point mutation, or the AbTx-AgTx complex), and each column corresponds to an antigen (i.e., an ADA). Each element of the count matrix described in FIG. 30 corresponds to a count of quantity of times the antigen bound to a cell expressing a particular antigen binding molecule (i.e., a particular AbTx). This data is used to identify the epitope (the antigen binding site) of the unmodified AbTx, the modified (mutated) AbTx, or the AbTx-AgTx bound by the ADA. This data is also used to identify a patient-specific AbTx, engineered to not elicit an immune response in the patient when treated with the identified patient-specific AbTx.

### Example 9: Characterization of anti-drug antibody (ADA) binding to antibody-therapeutic (AbTx)

The methods provided herein can be implemented to identify a patient specific engineered AbTx that does not elicit an immune response in patient. In some cases, the methods identify a patient-specific engineered AbTx that retains its binding profile to its AgTx and does not elicit an immune response in the patient. Exemplary workflows of identifying the patient specific AbTx are described in FIGS. 24C-24D.

Referring to FIG. 27C, the AbTx is conjugated to reporter oligonucleotides as described herein. Optionally, the AbTx is modified (mutated) to comprise at least one mutation, wherein each modified AbTx is conjugated with a unique reporter oligonucleotide. For example, a panel of AbTx's, e.g., including an unmodified AbTx and one or more modified AbTx's, can be conjugated with reporter oligonucleotides that identify the AbTx. The AbTx and/or modified AbTx is also labeled with a reporter fluorophore or any other detectable labels that can enable identification of the AbTx by, for example flow cytometry or any other known similar methods. In some embodiments, the AbTx and/or modified AbTx is labeled with an enrichment molecule, which can, for example, without limitation, activate FRET-based fluorescence upon binding its target ligand. In some embodiments, the target ligand is labeled with a reporter oligonucleotide or a fluorescent molecule that can be used in downstream enrichment steps.

Continuing with FIG. 27C, cells are engineered to express the ADAs identified in Example 7. Optionally, cells engineered to express a control antibody are provided. The control antibody is a non-ADA that binds an irrelevant target that is not the AbTx (e.g., a cell surface protein, a murine version of a human protein). The control antibodies are a commercially available clone against, for example, without limitation, CD3 or CD8. In some embodiments, the ADAs are tethered to the cell surface when expressed from constructs comprising the 5th exon, but not the 6th exon, of an IgG1 constant region. In some embodiments, the ADAs are secreted when expressed from constructs comprising the 6th exon, but not the 5th exon, of an IgG1 constant region.

The engineered cells are incubated with the AbTx and/or the modified (mutated) AbTx conjugated with reporter oligonucleotides. In some embodiments, the engineered cells are contacted with a panel of the AbTx and/or the modified (mutated) AbTx conjugated with reporter oligonucleotides. In some embodiments, the engineered cells expressing the ADAs are pre-incubated with its labeled antigenic target, which can optionally comprise a reporter oligonucleotide that can identify the antigenic target. In some embodiments, the engineered cells are enriched in order to identify a modified (mutated) AbTx that poorly or strongly binds ADAs. This enrichment process can optionally be repeated in a panning-like process.

The engineered cells bound to the AbTx and/or modified (mutated) AbTx conjugated with reporter oligonucleotides are partitioned and subjected to, for example, 10x 5'V2 Single Cell Immune Profiling kit per manufacturer's instructions. Additional information in this regard can be found at "support. 10xgenomics.com/permalink/getting-started-immune-profiling-feature-barcoding." This generates a reporter barcode library, a V(D)J library, and optional a gene expression library. The reporter barcode library can be used to detect the presence of the AbTx, the modified (mutated) AbTx, and optionally the AgTx ligand. The V(D)J library and the gene expression library are used to detect the presence of ADAs and the control antibodies expressed by the cells. Optionally, the libraries are enriched, e.g., according to the methods described in Example 8.

The generated libraries are sequenced on, for example, a NovaSeq 3 using a NovaSeq S4 200 cycles 2020 v1.5 kit per manufacturer's instructions. Sequencing can also be performed by various systems currently available, such as, without limitation, a sequencing system by Illumina^{®}, Pacific Biosciences (PacBio^{®}), Oxford Nanopore^{®}, or Life Technologies (Ion Torrent^{®}). The binding affinity of ADAs to the AbTx, the modified (mutated) AbTx, or the AbTx-AgTx complex are determined based on quantity/numbers of unique molecular identifiers (UMIs) associated with each of the antigen binding molecules bound to the target antigen.

A count matrix of cells expressing the ADAs and the AbTx or the modified (mutated) AbTx conjugated with and reporter barcodes are generated. In some embodiments, the count matrix is a count matrix described in FIG. 31, wherein each row corresponds to a cell expressing an antigen binding molecule (i.e., the known AbTx (unmodified), one or more modified (mutated) AbTx with at least one point mutation, or the AbTx-AgTx complex), and each column corresponds to an antigen (i.e., an ADA). Each element of the count matrix described in FIG. 31 corresponds to a count of quantity of times the antigen bound to a cell expressing a particular antigen binding molecule (i.e., a particular AbTx). This data can be used to identify (1) cases where each AbTx or each modified (mutated) AbTx is bound by one or more ADAs; (2) whether each AbTx or each modified (mutated) AbTx binds the AgTx ligand; (3) whether an ADA binding of each AbTx or each modified (mutated) AbTx can abrogate AgTx ligand binding; and (4) whether a particular AbTx-ADA binding is dependent on a AgTx binding to AbTx.

The ADAs and AbTx can be reversed in this workflow, where a library of ADAs binds the cells expressing the AbTx and/or the modified (mutated) AbTx, the process of which is described in FIG. 27D.

## Claims

1. A method, comprising:
(a) contacting a tissue sample comprising one or more cells expressing an antigen-binding molecule (ABM) with a reporter oligonucleotide conjugated antigen, wherein the reporter oligonucleotide comprises a reporter barcode sequence and the antigen is an antibody therapeutic and/or antibody drug conjugate;
(b) attaching a first analyte of an ABM-expressing cell of the tissue sample to a first capture domain of a first capture probe of an array of capture probes attached to a substrate, the first capture probe comprising (i) a spatial barcode sequence and (ii) the first capture domain comprising a first capture sequence, wherein the first analyte of the ABM-expressing cell comprises a sequence encoding the ABM expressed by the ABM-expressing cell or a reverse complement thereof, wherein the ABM expressed by the ABM-expressing cell binds to the antigen;
(c) attaching the reporter oligonucleotide to a second capture probe of the array, the second capture probe of the array comprising (i) the spatial barcode sequence and (ii) a second capture domain comprising a second capture sequence;
(d) using the first analyte of the ABM-expressing cell and the first capture probe attached thereto to generate a first spatially barcoded polynucleotide comprising (i) all or a portion of the sequence of the first analyte of the ABM-expressing cell or reverse complement thereof; and (ii) the spatial barcode sequence or reverse complement thereof; and
(e) using the reporter oligonucleotide and the second capture probe attached thereto to generate a second spatially barcoded polynucleotide comprising (i) the reporter barcode sequence or reverse complement thereof and (ii) the spatial barcode sequence or reverse complement thereof,
wherein the method further comprises:
(i) using the first spatially barcoded polynucleotide to determine all or a portion of the sequence of the first analyte attached to the first capture probe, wherein the determining comprises sequencing the first spatially barcoded polynucleotide or an amplicon thereof;
(ii) using the second spatially barcoded polynucleotide to determine the reporter barcode sequence of the reporter oligonucleotide attached to the second capture probe, wherein the determining of the reporter barcode sequence comprises sequencing the second spatially barcoded polynucleotide or an amplicon thereof; and
(iii) using the determined sequences to determine that the ABM expressed by the ABM-expressing cell binds to the antigen.

2. The method of claim 1, wherein the ABM expressed by the ABM-expressing cell is an immune receptor and wherein the contacting of (a) labels the ABM-expressing cell with the reporter oligonucleotide conjugated antigen.

3. The method of claim 2, wherein the immune receptor is:
(i) a BCR;
(ii) a TCR; or
(iii) an Fc receptor, optionally wherein the antigen is an antibody drug conjugate.

4. The method of claim 1, wherein the ABM expressed by the ABM-expressing cell is a secreted antibody and wherein the contacting of (a) labels the secreted antibody with the reporter oligonucleotide conjugated antigen, wherein, prior to or during (b), the labeled secreted antibody is in proximity to the ABM-expressing cell.

5. The method of any one of the preceding claims, wherein the reporter oligonucleotide further comprises a capture handle sequence that is complementary to the second capture sequence of the second capture domain.

6. The method of any one of the preceding claims, wherein the first capture sequence of the first capture domain and the second capture sequence of the second capture domain are identical or different.

7. The method of any one of the preceding claims, wherein the first capture sequence of the first capture domain is a homopolymeric sequence, optionally wherein the homopolymeric sequence is a polyT sequence.

8. The method of any one of claims 1-6, wherein the first capture sequence of the first capture domain is a defined non-homopolymeric sequence, wherein the defined non-homopolymeric sequence specifically binds to a nucleic acid sequence in the first analyte encoding a region of the ABM expressed by the ABM-expressing cell, optionally wherein:
(i) the ABM is selected from: a TCR alpha chain, a TCR beta chain, a TCR gamma chain, a TCR delta chain, an immunoglobulin kappa light chain, an immunoglobulin lambda light chain, and an immunoglobulin heavy chain; and/or
(ii) the region of the ABM is a constant region of the ABM or a variable region of the ABM.

9. The method of any one of the preceding claims, wherein the second capture sequence of the second capture domain is:
(i) a homopolymeric sequence, optionally wherein the homopolymeric sequence is a polyT sequence; or
(ii) a defined non-homopolymeric sequence.

10. The method of any one of claims 5-9, wherein the capture handle sequence is blocked prior to (a), optionally wherein the capture handle sequence is blocked by a blocking probe, optionally wherein the blocking probe is removed prior to (c).

11. The method of any one of the preceding claims, further comprising amplifying the first spatially barcoded polynucleotide with a first primer that specifically binds to a functional sequence of the first capture probe or reverse complement thereof and a second primer that binds to a nucleic acid sequence encoding a variable region of the ABM expressed by the ABM-expressing cell or reverse complement thereof, wherein the first primer and the second primer flank the spatial barcode of the first spatially barcoded polynucleotide.

12. The method of any one of the preceding claims, wherein the tissue sample is in contact with the substrate comprising the array of capture probes attached thereto during (a), (b), or (a) and (b), wherein the method comprises, following (a):
releasing the first analyte from the ABM-expressing cell of the tissue sample; and
migrating the first analyte to the substrate comprising the array of capture probes attached thereto.

13. The method of any one of claims 1-11, wherein the substrate comprising the array of capture probes attached thereto is a second substrate, wherein the tissue sample is mounted on a first substrate during (a), and wherein the method comprises, following (a):
mounting the first substrate on a first member of a support device, the first member configured to retain the first substrate;
mounting the second substrate on a second member of the support device, the second member configured to retain the second substrate,
applying a reagent medium to the first substrate and/or the second substrate, the reagent medium comprising a permeabilization agent,
operating an alignment mechanism of the support device to move the first member and/or the second member such that a portion of the tissue sample comprising the ABM-expressing cell is aligned with a portion of the array of capture probes and within a threshold distance of the array of capture probes, and such that the portion of the tissue sample and the capture probe contact the reagent medium,
wherein the permeabilization agent releases the first analyte from the ABM-expressing cell.

14. The method of any one of the preceding claims, wherein the first analyte comprises a nucleic acid sequence encoding a variable sequence and a constant sequence of the ABM, optionally wherein the variable sequence is a VDJ sequence, optionally wherein the wherein the ABM-expressing cell is a B cell, a natural killer (NK) cell, a T-Reg cell, a CAR-T cell, or a T cell.

15. The method of any one of the preceding claims, wherein the tissue sample is a tissue section, optionally a fresh frozen tissue section, a fixed tissue section, or an FFPE tissue section.

16. The method of any one of the preceding claims, wherein the first spatially barcoded polynucleotide and the second spatially barcoded polynucleotide comprise the same spatial barcode sequence or reverse complement thereof.

## Patentansprüche

1. Verfahren, umfassend:
(a) Inkontaktbringen einer Gewebeprobe, die eine oder mehrere Zellen umfasst, die ein Antigen-bindendes Molekül (ABM) exprimieren, mit einem Antigen, das mit einem Reporter-Oligonukleotid konjugiert ist, wobei das Reporter-Oligonukleotid eine Reporter-Barcodesequenz umfasst und das Antigen ein Antikörper-Therapeutikum und/oder Antikörper-Arzneimittelkonjugat ist;
(b) Anlagern eines ersten Analyten einer ABM-exprimierenden Zelle der Gewebeprobe an eine erste Erfassungsdomäne einer ersten Erfassungssonde einer Anordnung von Erfassungssonden, die an einem Substrat angelagert ist, wobei die erste Erfassungssonde (i) eine räumliche Barcodesequenz und (ii) die erste Erfassungsdomäne umfasst, die eine erste Erfassungssequenz umfasst, wobei der erste Analyt der ABM-exprimierenden Zelle eine Sequenz umfasst, die das ABM, das von der ABM-exprimierenden Zelle exprimiert wird, oder ein umgekehrtes Komplement davon codiert, wobei das ABM, das von der ABM-exprimierenden Zelle exprimiert wird, an das Antigen bindet;
(c) Anlagern des Reporter-Oligonukleotids an eine zweite Erfassungssonde der Anordnung, wobei die zweite Erfassungssonde der Anordnung (i) die räumliche Barcodesequenz und (ii) eine zweite Erfassungsdomäne umfasst, die eine zweite Erfassungssequenz umfasst;
(d) Verwenden des ersten Analyten der ABM-exprimierenden Zelle und der daran angelagerten ersten Erfassungssonde, um ein erstes räumlich barcodiertes Polynukleotid zu erzeugen, das (i) die Gesamtheit oder einen Abschnitt der Sequenz des ersten Analyten der ABM-exprimierenden Zelle oder ein umgekehrtes Komplement davon; und (ii) die räumliche Barcodesequenz oder das umgekehrte Komplement davon umfasst; und
(e) Verwenden des Reporter-Oligonukleotids und der daran angelagerten zweiten Erfassungssonde, um ein zweites räumlich barcodiertes Polynukleotid zu erzeugen, das (i) die Reporter-Barcodesequenz oder ein umgekehrtes Komplement davon und (ii) die räumliche Barcodesequenz oder das umgekehrte Komplement davon umfasst;
wobei das Verfahren ferner umfasst:
(i) Verwenden des ersten räumlich barcodierten Polynukleotids, um die Gesamtheit oder einen Abschnitt der Sequenz des ersten Analyten zu ermitteln, der an die erste Erfassungssonde angelagert ist, wobei das Ermitteln das Sequenzieren des ersten räumlich barcodierten Polynukleotids oder eines Amplikons davon umfasst;
(ii) Verwenden des zweiten räumlich barcodierten Polynukleotids, um die Reporter-Barcodesequenz des Reporter-Oligonukleotids zu ermitteln, das an die zweite Erfassungssonde angelagert ist, wobei das Ermitteln der Reporter-Barcodesequenz das Sequenzieren des zweiten räumlich barcodierten Polynukleotids oder eines Amplikons davon umfasst; und
(iii) Verwenden der ermittelten Sequenzen, um zu ermitteln, dass das ABM, das durch die ABM-exprimierende Zelle exprimiert wird, an das Antigen bindet.

2. Verfahren nach Anspruch 1, wobei das ABM, das durch die ABM-exprimierende Zelle exprimiert wird, ein Immunrezeptor ist und wobei das Inkontaktbringen von (a) die ABM-exprimierende Zelle mit dem Antigen markiert, das mit dem Reporter-Oligonukleotid konjugiert ist.

3. Verfahren nach Anspruch 2, wobei der Immunrezeptor Folgendes ist:
(i) ein BCR;
(ii) ein TCR; oder
(iii) ein Fc-Rezeptor, wobei optional das Antigen ein Antikörper-Arzneimittelkonjugat ist.

4. Verfahren nach Anspruch 1, wobei das ABM, das von der ABM-exprimierenden Zelle exprimiert wird, ein sekretierter Antikörper ist und wobei das Inkontaktbringen von (a) den sekretierten Antikörper mit dem Antigen markiert, das mit dem Reporter-Oligonukleotid konjugiert ist, wobei, vor oder während (b), sich der markierte sekretierte Antikörper in der Nähe der ABM-exprimierenden Zelle befindet.

5. Verfahren nach einem der vorhergehenden Ansprüche, wobei das Reporter-Oligonukleotid ferner eine Erfassungs-Handle-Sequenz umfasst, die komplementär zu der zweiten Erfassungssequenz der zweiten Erfassungsdomäne ist.

6. Verfahren nach einem der vorhergehenden Ansprüche, wobei die erste Erfassungssequenz der ersten Erfassungsdomäne und die zweite Erfassungssequenz der zweiten Erfassungsdomäne identisch oder unterschiedlich sind.

7. Verfahren nach einem der vorangehenden Ansprüche, wobei die erste Erfassungssequenz der ersten Erfassungsdomäne eine homopolymere Sequenz ist, wobei optional die homopolymere Sequenz eine PolyT-Sequenz ist.

8. Verfahren nach einem der Ansprüche 1-6, wobei die erste Erfassungssequenz der ersten Erfassungsdomäne eine definierte nicht-homopolymere Sequenz ist,wobei die definierte nicht-homopolymere Sequenz spezifisch an eine Nukleinsäuresequenz in dem ersten Analyten bindet, die einen Bereich des ABM codiert, das durch die ABM-exprimierende Zelle exprimiert wird, wobei optional:
(i) das ABM ausgewählt ist aus: einer alpha-Kette des TCR, einer beta-Kette des TCR, einer gamma-Kette des TCR, einer delta-Kette des TCR, einer leichten Immunglobulin-Kappa-Kette, einer leichten Immunglobulin-Lambda-Kette und einer schweren Kette des Immunglobulins; und/oder
(ii) der Bereich des ABM ein konstanter Bereich des ABM oder ein variabler Bereich des ABM ist.

9. Verfahren nach einem beliebigen der vorhergehenden Ansprüche, wobei die zweite Erfassungssequenz der zweiten Erfassungsdomäne Folgendes ist:
(i) eine homopolymere Sequenz, wobei optional die homopolymere Sequenz eine polyT-Sequenz ist; oder
(ii) eine definierte nicht-homopolymere Sequenz.

10. Verfahren nach einem der Ansprüche 5-9, wobei die Erfassungs-Handle-Sequenz vor (a) blockiert wird, wobei optional die Erfassungs-Handle-Sequenz durch eine Blockierungssonde blockiert wird, wobei optional die Blockierungssonde vor (c) entfernt wird.

11. Verfahren nach einem der vorhergehenden Ansprüche, das ferner das Amplifizieren des ersten räumlich barcodierten Polynukleotids mit einem ersten Primer, der spezifisch an eine funktionelle Sequenz der ersten Erfassungssonde oder ein reverses Komplement davon bindet, und einem zweiten Primer umfasst, der an eine Nukleinsäuresequenz bindet, die eine variable Region des ABM codiert, das durch die ABM-exprimierende Zelle oder ein reverses Komplement davon exprimiert wird, wobei der erste Primer und der zweite Primer den räumlichen Barcode des ersten räumlich barcodierten Polynukleotids flankieren.

12. Verfahren nach einem der vorhergehenden Ansprüche, wobei die Gewebeprobe mit dem Substrat in Kontakt ist, das die Anordnung von Erfassungssonden umfasst, die daran während (a), (b) oder (a) und (b) angelagert werden, wobei das Verfahren, nach (a), Folgendes umfasst:
Freisetzen des ersten Analyten aus der ABM-exprimierenden Zelle der Gewebeprobe; und Migrieren des ersten Analyten zu dem Substrat, das die Anordnung von daran angelagerten Erfassungssonden umfasst.

13. Verfahren nach einem der Ansprüche 1-11, wobei das Substrat, das die Anordnung von daran angelagerten Erfassungssonden umfasst, ein zweites Substrat ist, wobei die Gewebeprobe auf während (a) auf ein erstes Substrat aufgebracht wird und wobei das Verfahren, nach (a), Folgendes umfasst:
Aufbringen des ersten Substrats auf ein erstes Element einer Trägervorrichtung, wobei das erste Element so eingerichtet ist, dass es das erste Substrat hält;
Aufbringen des zweiten Substrats auf ein zweites Element einer Trägervorrichtung, wobei das Zweite Element so eingerichtet ist, dass es das zweite Substrat hält,
Anwenden eines Reagenzmediums auf das erste Substrat und/oder das zweite Substrat, wobei das Reagenzmedium ein
Permeabilisierungsmittel umfasst,
Betreiben eines Ausrichtungsmechanismus der Trägervorrichtung, um das erste Element und/oder das zweite Element so zu bewegen, dass ein Teil der Gewebeprobe, welche die ABM-exprimierende Zelle umfasst, an einem Abschnitt der Anordnung von Erfassungssonden und innerhalb eines Schwellenwertabstands der Anordnung von Erfassungssonden ausgerichtet ist, und so, dass der Abschnitt der Gewebeprobe und die Erfassungssonde das Reagenzmedium berühren, wobei das Permeabilisierungsmittel den ersten Analyten aus der ABM-exprimierenden Zelle freisetzt.

14. Verfahren nach einem der vorhergehenden Ansprüche, wobei der erste Analyt eine Nukleinsäuresequenz umfasst, die eine variable Sequenz und eine konstante Sequenz des ABM codiert, wobei optional die variable Sequenz eine VDJ-Sequenz ist, wobei optional die ABM-exprimierende Zelle eine B-Zelle, eine natürliche Killer(NK)-Zelle, eine T-Reg-Zelle, eine CAR-T-Zelle oder eine T-Zelle ist.

15. Verfahren nach einem der vorhergehenden Ansprüche, wobei die Gewebeprobe ein Gewebeschnitt, optional ein frisch eingefrorener Gewebeschnitt, ein fixierter Gewebeschnitt oder ein FFPE-Gewebeschnitt ist.

16. Verfahren nach einem der vorhergehenden Ansprüche, wobei das erste räumlich barcodierte Polynukleotid und das zweite räumlich barcodierte Polynukleotid die gleiche räumliche Barcodesequenz oder ein umgekehrtes Komplement davon umfassen.

## Revendications

1. Procédé, comprenant :
(a) la mise en contact d'un échantillon de tissu comprenant une ou plusieurs cellules exprimant une molécule de liaison à l'antigène (ABM) avec un antigène conjugué d'oligonucléotide rapporteur, dans lequel l'oligonucléotide rapporteur comprend une séquence de code à barres de rapporteur et l'antigène est un anticorps thérapeutique et/ou un conjugué anticorps-médicament ;
(b) la fixation d'un premier analyte d'une cellule exprimant ABM de l'échantillon de tissu à un premier domaine de capture d'une première sonde de capture d'un réseau de sondes de capture fixées à un substrat, la première sonde de capture comprenant (i) une séquence de code à barres spatial et (ii) le premier domaine de capture comprenant une première séquence de capture, dans lequel le premier analyte de la cellule exprimant ABM comprend une séquence codant pour l'ABM exprimée par la cellule exprimant ABM ou un complément inverse de celle-ci, dans lequel l'ABM exprimée par la cellule exprimant ABM se lie à l'antigène ;
(c) la fixation de l'oligonucléotide rapporteur à une deuxième sonde de capture du réseau, la deuxième sonde de capture du réseau comprenant (i) la séquence de code à barres spatial et (ii) un deuxième domaine de capture comprenant une deuxième séquence de capture ;
(d) l'utilisation du premier analyte de la cellule exprimant ABM et de la première sonde de capture fixée à celui-ci pour générer un premier polynucléotide codé spatialement par code à barres comprenant (i) la totalité ou une portion de la séquence du premier analyte de la cellule exprimant ABM ou un complément inverse de celle-ci ; et (ii) la séquence de code à barres spatial ou un complément inverse de celle-ci ; et
(e) l'utilisation de l'oligonucléotide rapporteur et de la deuxième sonde de capture fixée à celui-ci pour générer un deuxième polynucléotide codé spatialement par code à barres comprenant (i) la séquence de code à barres rapporteur ou un complément inverse de celle-ci et (ii) la séquence de code à barres spatial ou un complément inverse de celle-ci ;
dans lequel le procédé comprend en outre :
(i) l'utilisation du premier polynucléotide codé spatialement par code à barres pour déterminer la totalité ou une portion de la séquence du premier analyte fixé à la première sonde de capture, dans lequel la détermination comprend le séquençage du premier polynucléotide codé spatialement par code à barres ou d'un amplicon de celui-ci ;
(ii) l'utilisation du deuxième polynucléotide codé spatialement par code à barres pour déterminer la séquence de code à barres de rapporteur de l'oligonucléotide rapporteur fixé à la deuxième sonde de capture, dans lequel la détermination de la séquence de code à barres de rapporteur comprend le séquençage du deuxième polynucléotide codé spatialement par code à barres ou d'un amplicon de celui-ci ; et
(iii) l'utilisation des séquences déterminées pour déterminer que l'ABM exprimée par la cellule exprimant ABM se lie à l'antigène.

2. Procédé selon la revendication 1, dans lequel l'ABM exprimé par la cellule exprimant ABM est un récepteur immunitaire et dans lequel la mise en contact de (a) marque la cellule exprimant ABM avec l'antigène conjugué d'oligonucléotide rapporteur.

3. Procédé selon la revendication 2, dans lequel le récepteur immunitaire est :
(i) un BCR ;
(ii) un TCR ; ou
(iii) un récepteur Fc, facultativement dans lequel l'antigène est un conjugué anticorps-médicament.

4. Procédé selon la revendication 1, dans lequel l'ABM exprimée par la cellule exprimant ABM est un anticorps sécrété et dans lequel la mise en contact de (a) marque l'anticorps sécrété avec l'antigène conjugué de l'oligonucléotide rapporteur, dans lequel, avant ou pendant (b), l'anticorps sécrété marqué est à proximité de la cellule exprimant ABM.

5. Procédé selon l'une quelconque des revendications précédentes, dans lequel l'oligonucléotide rapporteur comprend en outre une séquence point de capture qui est complémentaire de la deuxième séquence de capture du deuxième domaine de capture.

6. Procédé selon l'une quelconque des revendications précédentes, dans lequel la première séquence de capture du premier domaine de capture et la deuxième séquence de capture du deuxième domaine de capture sont identiques ou différentes.

7. Procédé selon l'une quelconque des revendications précédentes, dans lequel la première séquence de capture du premier domaine de capture est une séquence homopolymère, facultativement dans lequel la séquence homopolymère est une séquence polyT.

8. Procédé selon l'une quelconque des revendications 1 à 6, dans lequel la première séquence de capture du premier domaine de capture est une séquence non homopolymère définie, dans lequel la séquence non homopolymère définie se lie spécifiquement à une séquence d'acides nucléiques dans le premier analyte codant pour une région de l'ABM exprimée par la cellule exprimant ABM, facultativement dans lequel :
(i) l'ABM est choisie parmi : une chaîne alpha de TCR, une chaîne bêta de TCR, une chaîne gamma de TCR, une chaîne delta de TCR, une chaîne légère kappa d'immunoglobuline, une chaîne légère lambda d'immunoglobuline et une chaîne lourde d'immunoglobuline ; et/ou
(ii) la région de l'ABM est une région constante de l'ABM ou une région variable de l'ABM.

9. Procédé selon l'une quelconque des revendications précédentes, dans lequel la deuxième séquence de capture du deuxième domaine de capture est :
(i) une séquence homopolymère, facultativement dans lequel la séquence homopolymère est une séquence polyT ; ou
(ii) une séquence non homopolymère définie.

10. Procédé selon l'une quelconque des revendications 5 à 9, dans lequel la séquence de point de capture est bloquée avant (a), facultativement dans lequel la séquence de point de capture est bloquée par une sonde de blocage, facultativement dans lequel la sonde de blocage est retirée avant (c).

11. Procédé selon l'une quelconque des revendications précédentes, comprenant en outre l'amplification du premier polynucléotide codé spatialement par code à barres avec une première amorce qui se lie spécifiquement à une séquence fonctionnelle de la première sonde de capture ou d'un complément inverse de celle-ci et une deuxième amorce qui se lie à une séquence d'acides nucléiques codant pour une région variable de l'ABM exprimée par la cellule exprimant ABM ou un complément inverse de celle-ci, dans lequel la première amorce et la deuxième amorce flanquent le code à barres spatial du premier polynucléotide codé spatialement par code à barres.

12. Procédé selon l'une quelconque des revendications précédentes, dans lequel l'échantillon de tissu est en contact avec le substrat comprenant le réseau de sondes de capture fixées à celui-ci pendant (a), (b), ou (a) et (b), dans lequel le procédé comprend, à la suite de (a) :
la libération du premier analyte de la cellule exprimant ABM de l'échantillon de tissu ; et la migration du premier analyte vers le substrat comprenant le réseau de sondes de capture fixées à celui-ci.

13. Procédé selon l'une quelconque des revendications 1 à 11, dans lequel le substrat comprenant le réseau de sondes de capture fixées à celui-ci est un deuxième substrat, dans lequel l'échantillon de tissu est monté sur un premier substrat pendant (a), et dans lequel le procédé comprend, à la suite de (a) :
le montage du premier substrat sur un premier élément d'un dispositif support, le premier élément étant configuré pour retenir le premier substrat ;
le montage du deuxième substrat sur un deuxième élément du dispositif support, le deuxième élément étant configuré pour retenir le deuxième substrat,
l'application d'un milieu réactif au premier substrat et/ou au deuxième substrat, le milieu réactif comprenant un agent de perméabilisation,
la mise en fonctionnement d'un mécanisme d'alignement du dispositif support pour déplacer le premier élément et/ou le deuxième élément de telle sorte qu'une portion de l'échantillon de tissu comprenant la cellule exprimant ABM est alignée avec une portion du réseau de sondes de capture et à moins d'une distance seuil du réseau de sondes de capture, et de telle sorte que la portion de l'échantillon de tissu et la sonde de capture viennent en contact avec le milieu réactif,
dans lequel l'agent de perméabilisation libère le premier analyte de la cellule exprimant ABM.

14. Procédé selon l'une quelconque des revendications précédentes, dans lequel le premier analyte comprend une séquence d'acides nucléiques codant pour une séquence variable et une séquence constante de l'ABM, facultativement dans lequel la séquence variable est une séquence VDJ, facultativement dans lequel la cellule exprimant ABM est une cellule B, une cellule tueuse naturelle (NK), une cellule T régulatrice, une cellule CAR-T ou une cellule T.

15. Procédé selon l'une quelconque des revendications précédentes, dans lequel l'échantillon de tissu est une section de tissu, facultativement une section de tissu frais et congelé ou une section de tissu fixé ou une section de tissu FFPE.

16. Procédé selon l'une quelconque des revendications précédentes, dans lequel le premier polynucléotide codé spatialement par code à barres et le deuxième polynucléotide codé spatialement par code à barres comprennent la même séquence de code à barres spatial ou un complément inverse de celle-ci.
